# EUROPEAN PATENT SPECIFICATION

(11) **EP 4 501 933 B1**
(45) Date of publication and mention of the grant of the patent: **12.11.2025**
(21) Application number: 23189890.9
(22) Date of filing: 04.08.2023
(51) Int. Cl.: C07F 5/02, C09K 11/06, C07F 5/00

(54) **DIVALENT EUROPIUM-ORGANIC COORDINATION COMPOUND AND METHOD FOR PRODUCING THE SAME**
ZWEIWERTIGE EUROPIUM-ORGANISCHE KOORDINATIONSVERBINDUNG UND VERFAHREN ZU DEREN HERSTELLUNG
COMPOSÉ DE COORDINATION ORGANIQUE-EUROPIUM DIVALENT ET SON PROCÉDÉ DE PRODUCTION

(43) Date of publication of application: 05.02.2025
(73) Proprietor: beeOLED GmbH, 01217 Dresden (DE)
(72) Inventor: Kaden, Felix, 01127 Dresden (DE); Tkachov, Roman, 01159 Dresden (DE); Mewes, Jan-Michael, 53129 Bonn (DE)
(74) Representative: Altmann, Andreas

(56) References cited:
- WO-A1-2021/244801
- WO-A1-2022/218562

## Description

### Technical Field

The invention concerns a metal-organic coordination compound, wherein the coordination compound comprises a mixture and a divalent Europium cation coordinated by a macrocyclic organic ligand, an organic electronic device containing same, and methods for forming the same in particular using thermal evaporation or sublimation or casting from solution.

### Background

Electroluminescent devices that make use of organic light emitting diodes (OLEDs) are state-of-the-art for flat panel display applications used in everyday consumer electronics. For OLEDs, special organic materials, so-called emitters, are employed for the purpose of converting electrical excitation into light emission. For consumer display applications red, green, and blue emitters are needed. However, the development is most exclusively focused on blue emitter materials. The reason is twofold: Firstly, existing red and green emitter materials do combine high efficiency with saturated colors and good chemical stability, whereas only blue emitter materials with poor light-generation efficiency offer reasonable chemical stability. Secondly, advanced consumer display layouts use color conversion to generate the red and green spectral portions from the short wavelength blue emission. Therefore, only blue emitting electroluminescent OLED devices are needed in future displays. Thus, there is an urgent need for a saturated deep blue emitter with high efficiency and good chemical stability.

Here, coordination compounds based on the intrametallic transition of divalent Europium offer very promising properties: The ground state configuration features a half-filled 4d-shell, resulting in a single narrow Gaussian peak in the emission spectrum. At the same time, the emitting state is the lowest excited state of the materials; no undesired longer-lived states are possible. Moreover, as a heavy metal, Europium readily interconverts singlet and triplet excitons that form upon charge-recombination on organic host materials, such that both can be harvested for light generation with Eu-based emitters, leading to high electroluminescence efficiency. Finally, the transition energy of the first electronically excited divalent Europium coordinated with a wide variety of organic ligands appears in the desired deep blue spectral region between 430 and 500nm. Unlike most trivalent lanthanides, the transition responsible for the emission from divalent Europium is Laporte allowed and thus has a lifetime of only 1.25 µs, which is sufficiently short for consumer display applications.

Further, the transition responsible for light emission from Eu(ll) complexes is intrametallic and thus strongly confined on the Eu atom. Thus, electronically exciting the complex does not weaken any chemical bonds, offering the potential of exceptional chemical stability. Yet, during electroluminescent device operation, charge-trapping on Eu frequently occurs, which corresponds to an oxidation of Eu(II) to Eu(III). Consequently, another mandatory requirement for stable OLED operation is a reversible oxidation/reduction, requiring a stabilization of Eu(II) over Eu(III). This creates a certain challenge, since the most stable oxidation state of Eu is typically the trivalent one. In other words, a stabilization of oxidized trivalent europium after it has captured a positive charge must be avoided, which can be accomplished by using rigid coordination environments and hard anions. Thus, prior to application of divalent Europium in electroluminescent device applications, one needs a reasonably oxidation stable metal-organic compound. Two major strategies to stabilize divalent Europium are discussed in the literature. First, using soft and polarizable ligands, electronic stabilization is achieved, which, however, stabilizes the excited d-electron so much that the emission is not blue any longer. The second strategy uses size discrimination macrocyclic ligands. Here, stabilization is achieved geometrically by tuning the cavity size so to match divalent, but not trivalent Europium.

Sufficient electron stabilization may be achieved by using soft cyclopentadienyl type ligands as for example disclosed in Kelly, Rory P., et al. Organometallics 34.23 (2015): 5624-5636. Given their symmetrical, low dipole structure those materials are even sufficiently volatile for sublimation. However, the original deep blue emission of divalent Europium is shifted into the far-red spectral region, non-suitable for display applications.

US 6,492,526 discloses a metal-organic complex comprising divalent Europium and charged pyrazolyl borate ligands. In this case, the desired stabilization of the divalent oxidation state is achieved by application of the strong electron-donating chelating ligand. Yet, such a strong electron-donating ligand leads to a red shifted emission compared to the desirable deep blue emission from the free Eu(II) cation. Using the same approach, green electroluminescent devices were exemplified by Guo, Ruoyao, et al. Molecules 27.22 (2022): 8053.

Another related art to combine the desired properties of divalent Europium with the benefits of organic processing are metal-organic coordination compounds, in which the reactive metal cation is stabilized by cyclic polyether or bicyclic macrocyclic ligands, such as cryptands, see the review by Jiang, Jianzhuang, et al. in Coordination chemistry reviews 170.1 (1998): 1-29. However, in all those cases, the central metal ion has mainly been coordinated to electron rich hard donor atoms, such as oxygen and nitrogen. Consequently, there is little electronic stabilization achieved here. The coordination compounds, albeit still blue emitting, remain very air sensitive. Additionally, in all those literature examples, charge neutrality of the coordination compounds is achieved by incorporation of halogen anions into the coordination compound. However, since those anions are typically outside the first coordination sphere of the divalent Europium, the respective compounds are very polar reflected in a high dipole moment. Consequently, all those macrocyclic and polymacrocyclic coordination compounds are only soluble in very polar solvents, such as methanol, and they show low volatility, which eludes gas transfer processing that is preferable to make high quality electroluminescent devices. Hence, to date, no coordination compounds are known that combine all the desired photophysical properties of the divalent lanthanide with good oxidative stability and sufficient volatility in order to enable fabrication under ambient (non-oxygen free) conditions and/or via gas transfer processes.

In another related art, a specific strategy to prevent oxidation is used by incorporating soft coordinating ligands, such as sulphur or aromatic aryl or heteroaryl rings into the coordination sphere of the central metal. Thereby, oxidation stability of the central metal may be observed, see Gamage, Nipuni-Dhanesha H., et al. Angewandte Chemie 122.47 (2010): 9107-9109. However, such soft coordinating atoms in an asymmetrical environment typically cause the originally deep blue and spectrally pure emission to red-shift substantially to the green and red spectral region and to broaden, which renders those metal-organic coordination compounds undesirable for use in opto-electronic devices.

In additional prior art, crown ethers and cryptands with Eu (II) in OLEDs are described in general terms in WO 2004/058912. Nitrogen-containing (macro)circular molecules with Eu(II) in OLEDs, similar to crown ethers, are described in WO 2011/090977. WO 2014/164712 describes nitrogen-containing (macro)circular molecules with Eu(ll) for MRI applications. There is no mention of OLED applications, which, again, would be difficult as those particular coordination compounds are not sufficiently volatile. Chem. Commun., 2018, 54, pages 4545-4548, discloses nitrogen-containing (macro)circular molecules with Eu(II). In this respect, (macro)circular molecules are understood to accommodate the central atom, for example Eu(II) in the centre of the circular structure.

The favourable deep blue emission characteristics of divalent Europium have not yet successfully been demonstrated in highly efficient electroluminescent devices. By coordinating divalent europium using rigid symmetrical polymacrocyclic ligands, only yellow-green emission was achieved in Li, J., Wang, L., Zhao, Z. et al. Nat Commun 11, 5218 (2020). A substituted deep blue emitting symmetrical polymacrocyclic aza cryptand is disclosed in WO 2021/244801 A1, yet not all of those materials offer sufficient volatility. Corresponding electroluminescent devices have been demonstrated using solution processing and efficiencies were rather poor.

Although OLEDs have been demonstrated with Eu(II) emitters based on such symmetric polycyclic ligands, those particular emitters can hardly be employed for the mass production of consumer displays. Here, the vast majority of all consumer OLED displays are produced using thermal evaporation or sublimation, which was typically not possible using the deep blue emitting materials based on symmetrical ligand design disclosed in WO 2021/244801 A1. In fact, the green-emitting devices disclosed in Li, J., Wang, L., Zhao, Z. et al. Nat Commun 11, 5218 (2020) were processed using thermal sublimation. However, the sublimation yield was only about 10%. Additionally, significant thermal decomposition has been observed during the sublimation process, which renders this material not suitable for mass production using sublimation. In WO 2022/218562, twofold negatively charged polymacrocyclic ligands for Eu(II) are disclosed for improved thermal vacuum sublimation based on the same basic symmetrical cryptand design as Li, J., Wang, L., Zhao, Z. et al. Nat Commun 11, 5218 (2020) and WO 2021/244801 A1. However, again only green emitting Eu(II) compounds are practiced and, once more, the sublimation yield is well below 100%.

Thus, as of today, no deep blue emitting Eu(II) organic coordination compound that can be processed by thermal evaporation or sublimation with high yield and without significant decomposition, is known. The problem is fundamental if a bis-cyclic ligand design is used, that is, cryptands with three equal macrocyclic rings: To achieve charge neutrality, the central twofold positively Europium cation dictates the presence of two negative counter charges (anions) in the complex. For cryptands, this leads to an intrinsically unfavorable chemical structure of the metal-organic complex, typically featuring high dipole moments resulting in polar compounds with low volatility. Alternatively, and resulting in relatively lower dipole moments, the negatively charged groups may form a direct bond with the central metal, which, however, red shifts the desired deep blue color, compare WO 2022/218562.

An object underlying the present invention is to provide an electroluminescent coordination compound which shows deep blue, highly saturated emission combined with high operational stability in an OLED device. In particular, neither the molecule itself nor parts of it shall drift within the OLED device upon application of an electrical field which is known to seriously compromise the stability of such an OLED device and would just prevent application of such OLED devices in consumer electronics.

Furthermore, it is an objective to provide an electroluminescent coordination compound that can be reversibly electronically oxidized and reduced again. In particular, the compound shall be able to trap a hole and subsequently trap an electron with high chemical endurance as those are the primary microscopic steps needed for stable operation of an electroluminescent device.

According to a further object, the electroluminescent coordination compounds shall show an increased sublimation yield such that large area OLED displays can be processed using well-established low-cost sublimation or evaporation technology.

A further objective is to provide anions for metal-organic coordination compounds containing macrocyclic organic ligands and one Eu(ll) cation.

The objectives are achieved according to the present invention, as defined in claim 1, by a metal-organic coordination compound, wherein the coordination compound comprises one divalent Europium ion and a macrocyclic organic ligand in which at least 9 macrocyclic atoms are part of a single cyclic ring, the macrocycle, wherein only those carbon or heteroatoms are counted as macrocyclic atoms that do form the cyclic closed ring system around the Eu(II), comprising 12 to 48, preferably 15 to 24, and most preferably 18 to 20 macrocyclic atoms, which are present in the macrocycle, further comprising in the coordination compound at least one borane cluster anion selected from (3-1) to (3-3), or at least one borate cluster anion formed from two borate cluster fragments selected from (3-4) to (3-8), which are covalently linked with each other at the dashed line, preferably via a linear C₁₋₁₅⁻ alkylene spacer: 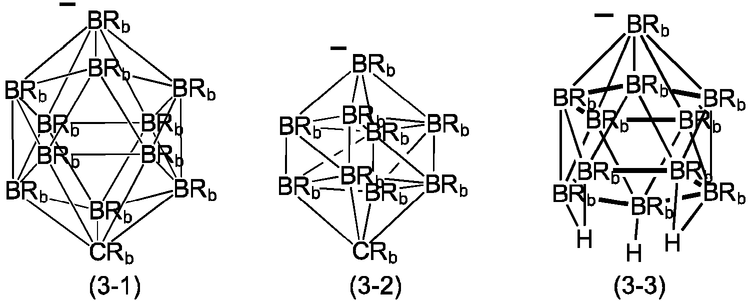 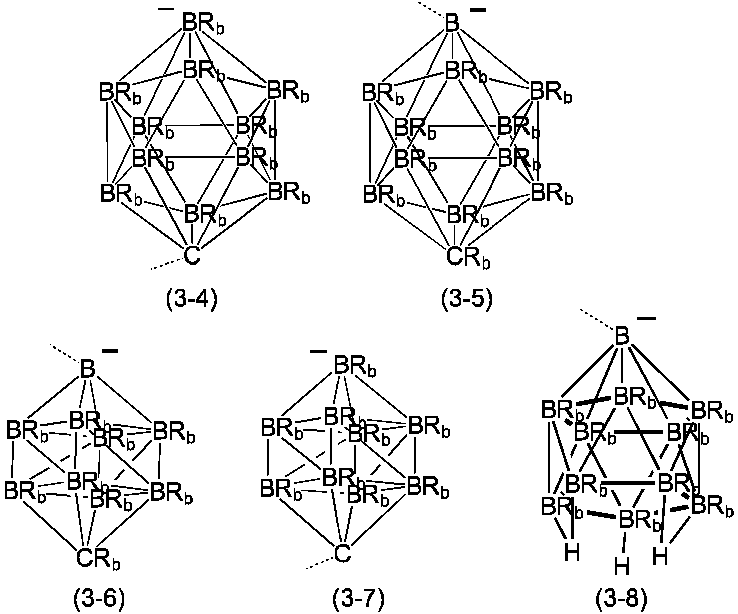
or with the macrocyclic organic ligand being singly or twofold negatively charged by means of covalent attachment of one or two carborate cluster fragments selected from (3-4) to (3-8) with
   - the dashed lines indicating the covalent linkage with each other or to the remainder of the macrocyclic ligand, and
   - **R_{b}** present in (3-1) to (3-8) independently in each occurrence representing either hydrogen, deuterium, fluorene, chlorine, bromine, or alkyl, preferably C₁₋₆⁻ alkyl, most preferably methyl,
and wherein the macrocyclic organic ligand can be covalently attached to the polymer backbone of a polymer with a molecular weight Mₙ above 1000 g/mol;
   and by a mixture comprising
   (a) the coordination compound according to the invention, and
   (b) at least one second electrically neutral organic compound different therefrom, wherein the coordination compound (a) is imbedded into the at least one second electrically neutral organic compound (b), the mixture having preferably one or more of the following features:
      - The second compound (b) is a charge-neutral host material;
      - The second organic compound (b) has a triplet energy higher than 2.5 eV, preferably higher than 2.6 eV and more preferably higher than 2.7 eV;
      - Compounds (a) and (b) are physically mixed or the second charge-neutral compound (b) has a molecular weight Mₙ above 1000 g/mol and the coordination compound (a) and the second charge-neutral compound (b) are chemically linked with each other, for example by a covalent or coordinative chemical bond;
      - The coordination compound (a) is electrically neutral or singly positively charged and is imbedded into the at least one second electrically neutral organic compound (b) in a ratio of 0.1 to 99 vol%, based on the mixture which is 100 vol%;
      - The second organic compound (b) has a lower hole affinity compared to the electrically neutral coordination compound (a);
      - The second organic compound (b) has a lower electron affinity compared to the singly positively charged coordination compound (a);
      - The second material is a charge transport organic material capable of transporting positive or negative charges;

      and by an organic electronic device, comprising: a first electrode; a second electrode; and an organic layer arranged such that it is electrically interposed between the first and second electrodes, wherein the organic layer comprises the coordination compound according to the invention, wherein preferably the organic electronic device is an optoelectronic device, the optoelectronic device preferably being at least one of an organic light emitting diode, an organic photodetector, or a photovoltaic cell;
      and by a method of forming an organic device according to the invention, the method comprising: forming a layer of the coordination compound according to the invention wherein the layer is deposited from a gas phase, in particular using an evaporation and/or sublimation and/or carrier gas process, or wherein the layer is deposited from solution, whereby preferably the solution is comprised substantially or organic solvents with a polarity index of less than 5.0, preferably of less than 4.0, more preferably of less than 3.0;
      and by the use of a coordination compound according to the invention as active or passive emitting material in electroluminescent electronics applications or as a dye or contrast enhancement medium for magnetic resonance tomography;
      and by the use of borane clusters in which the borane cluster anion is selected from (3-1) to (3-3), for forming coordination compounds comprising one divalent Europium ion, a macrocyclic organic ligand, and further comprising at least one borane cluster anion selected from (3-1) to (3-3), 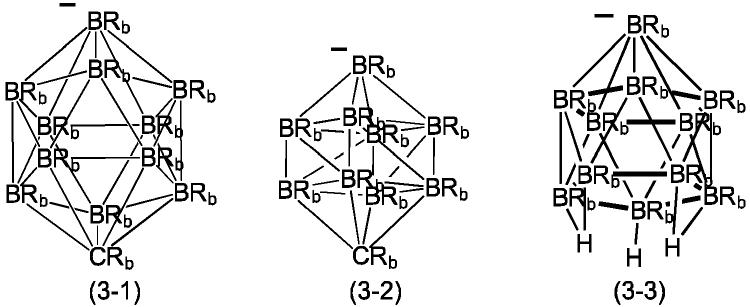
or at least one borate cluster anion formed from two borate cluster fragments selected from (3-4) to (3-8), which are covalently linked with each other at the dashed line, preferably via a linear C₁₋₁₅⁻ alkylene spacer, or with the macrocyclic organic ligand being singly or twofold negatively charged by means of covalent attachment of one or two carborate cluster fragments selected from (3-4) to (3-8) 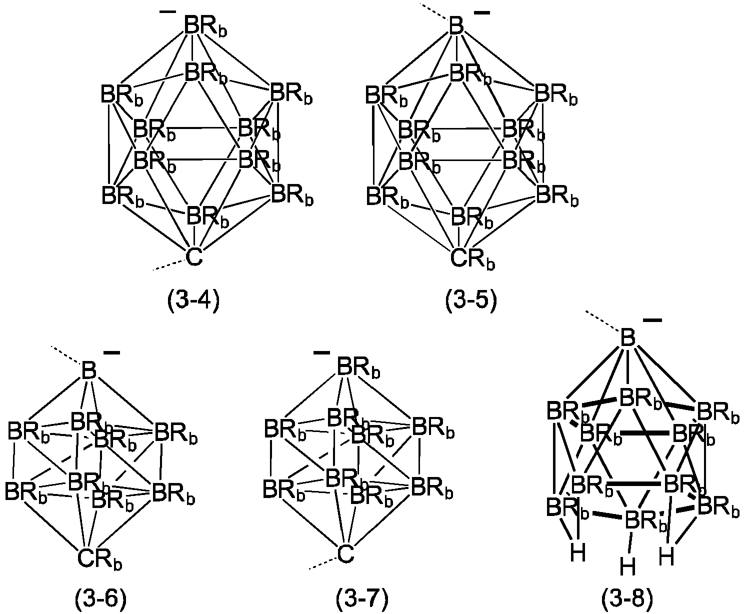 with
   - the dashed lines indicating the covalent linkage to the remainder of the macrocyclic ligand, and
   - **R_{b}** present in (3-1) - (3-8) independently in each occurrence representing either hydrogen, deuterium, fluorene, chlorine, bromine, or alkyl, preferably C₁₋₆⁻ alkyl, most preferably methyl,
and wherein the macrocyclic organic ligand can be covalently attached to the polymer backbone of a polymer with a molecular weight Mₙ above 1000 g/mol.

In a preferred embodiment, the present invention relates to the use of a symmetrical single macrocyclic crown ether ligand with one Eu(II) placed in the middle, such that two anions can be placed symmetrically atop and below the central Eu(II) cation. Such macrocyclic ligands will still be size discriminating to enable the necessary reduction of trivalent Europium into an excited (divalent) Europium which must happen during electroluminescent operation. By employing such ligands, it is possible to create symmetric complexes with blue emission and very small dipole moments, resulting in low polarity and sufficient volatility to enable gas transfer processing. Up to now, no such volatile blue emitting complex has been demonstrated.

According to the invention, the divalent Europium is centred inside the macrocyclic ligand. Many anions such as halogens, cyclopentadienyls, or borates have been investigated in the prior art. All of them are either chemically very instable, i.e. they cause Eu(II) to easily oxidize, or they lead to a red-shift of the emission of the complex in an OLED. It was found that the ambient and general chemical stability of the complexes comprising borate cluster type anions is significantly improved vs complexes comprising other anions. At the same time, the transition energy, i.e., the emission of the coordination compound is still deep blue as desired for consumer display applications. Thus, the coordination compounds according to invention are deep-blue-emitting yet highly stable against (irreversible) oxidation, a combination of features that makes the coordination compound of the invention superior to those described in the prior art. Finally, another clear advantage of these anions employed according to the invention is the large number of possible residues R_{b}, which enables synthetically easy modification and fine-tuning of the anion structure and of the photophysical properties resulting therefrom.

Furthermore, a feature of the present invention is to provide distinct symmetrical coordination compounds with divalent Europium in the centre. Here, the single macrocyclic coordinating ligand may form an equatorial plane together with the Europium, while the two closocarborate type anions are arranged on an axis perpendicular through this plane, with the Europium in the centre. Thus, the anions are integrated in a way that they simultaneously stabilize the central divalent Europium and, because of symmetry, lead to a small dipole moment for the overall complex, thereby significantly improving its volatility. Thus, in a unique combination, highly chemically stable, volatile, and deep blue emitting divalent Europium coordination compounds, perfectly suited for display mass production, are obtained.

The divalent Europium compounds comprising a macrocyclic ligand and two closocarborate anions are not just more stable under ambient conditions, but also in the lowest electronically excited state that is relevant for emission, the electron excited to the 4d-orbital is strongly bound to the complex. This is critical for OLED devices, as this makes it easy to confine the charges of the excited emitter within various host materials. OLED design with an emitter according to invention thus becomes very easy because charge transport matrixes with their relatively low LUMO energy's can be successfully employed as well. The combination of the properties mentioned enables the design of very efficient deep blue emitting OLED devices with high operational stability. With this combination of ideal emission characteristics, good processing characteristics, and freedom of OLED design, the coordination compounds according to the invention are ideally suited as active emitting materials in electroluminescent consumer electronics applications.

### Summary of the invention

In various aspects of this invention, metal-organic coordination compounds comprising divalent Europium coordinated by a macrocyclic ligand and closocarborate anions, and methods for synthesizing those divalent Europium compounds are provided.

The objective is achieved by a Europium-organic coordination compound comprising anions or anionic groups as defined above and in claim 1. To make use of the potentially excellent emission properties of divalent Europium, the luminescent active cation must be sufficiently chemically stabilized such as to avoid undesired irreversible oxidation of divalent into trivalent Europium. The key ingredient is the use of closocarborate or borate anions or anionic groups according to formula (3-1) to (3-8) in combination with a macrocyclic organic ligand. Here, we surprisingly found that those anions offer a much superior stabilization of the divalent Europium against oxidation than any other organic or inorganic anion or anionic group, such as halogens, alcoholates, or other weakly coordinating anions like BF₄, PF₆, or BArF. At the same time, those closocarborate anions - whether being covalently attached to the macrocyclic organic ligand or not - arrange on opposing sides of the plane defined by the central cation and the macrocyclic organic ligand, resulting in a low overall dipole moment. Consequently, very high volatility is achieved such that sublimation yields close to 100% are typically observed. Finally, the direct metal-anion ionic bond is still sufficiently weak, such that Eu(II) retains its saturated deep blue emission spectrum.

To ensure the preferred symmetrical coordination compounds with low dipole moment, the central divalent Europium must well geometrically fit into the macrocyclic organic ligand that coordinates to it. Thus, the ionic diameter of the divalent Europium must fit the cavity formed by the macrocyclic ligand. The latter may depend somewhat on what atoms (carbon atoms and heteroatoms) exactly are present as part of the macrocycle forming "the care" of the macrocyclic organic ligand. For example, replacing nitrogen by sulfur in a heterocyclic structure slightly increases the ring size due to the longer carbon-sulfur bond. However, a good coordination of the divalent Europium ion by the macrocyclic organic ligand is generally achieved if the shortest sequence of atoms forming the macrocycle in the macrocyclic organic ligand being made of 12 to 48, preferably 15 to 24, most preferably 18 to 20 covalently linked atoms, wherein preferably the macrocycle contains 2 to 14, more preferably 3 to 10, most preferably 5 to 8 non-neighboring heteroatoms, preferably the metal organic coordination compound is charge neutral and contains two, preferably identical, borane cluster anions selected from (3-1) to (3-3) in the coordination compound, or contains two, preferably identical, covalently attached borate cluster fragments selected from (3-4) to (3-8).

The macrocyclic ligand itself may preferably be chosen according to the generic formula (1-1): 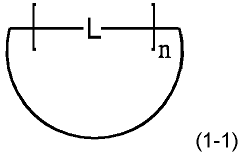 with
- n being 4-8, preferably 4-6, most preferably 6; and
- L being independently in each occurrence a divalent organic group formed by removing two hydrogen atoms from an organic molecule containing at least two hydrogen atoms, with
   ∘ the shortest sequence of atoms linking each L with the remainder of the macrocycle in the macrocyclic ligand being 2 to 4, preferably 3 atoms, and with
   ∘ one of those 2 to 4, preferably 3 atoms independently for each L being selected from the heteroatoms B, N, P, S, Se, Si or O, preferably N, S, or O, the other atoms of those atoms being carbon,
wherein preferably the heteroatoms in the macrocycle are non-neighboring.

Here, the macrocyclic organic ligand is constituted of n subunits, each containing at least one heteroatom. The heteroatoms ensure good coordination of Eu(II) by the macrocyclic organic ligand and are preferably selected from N, P, O, S, and Se. Yet, Group 6 and hard Group 5 donor atoms cause less undesirable red-shift of the emission energy, so preference is given to the heteroatoms N, S, and O. In addition to the coordinating heteroatom(s), one to three carbon atoms are present in the shortest sequence of atoms linking each subunit L to the remainder of the macrocycle, which helps to form chemically stable configurations. Preference is given to structures where the heteroatoms are non adjacent, more preferably where they are separated from each other by two or three, more preferably by three carbon atoms, i.e., for subunits each containing two carbon atoms and one heteroatom. Further, the number of coordination heteroatoms as part of the macrocycle in the organic macrocyclic ligand is 4-8, preferably 4-6, most preferably 6. Together with the preferred two additional carbon atoms for every heteroatom and arranged symmetrically around each heteroatom, one arrives at the chemically very robust and widely known 18-crown-6 motive, where 18 describes the number of macrocyclic atoms in the cyclic ligand and 6 denotes the number of heteroatoms thereof, typically O, known as crown ether. Such macrocyclic organic ligands perfectly stabilize the central divalent Europium over trivalent Europium if combined with the anions according to formula (3-1) to (3-8) above.

Starting from the preferred 18-crown-6 motive, with formula (1-1) being narrowed down to n = 6 and L containing 3 atoms and with the mandatory coordinating heteroatom in the middle of the three atoms, the subunits L are even more specifically independently in each occurrence selected from formula (2-1) to (2-9), preferably from (2-2), (2-4), or (2-5): 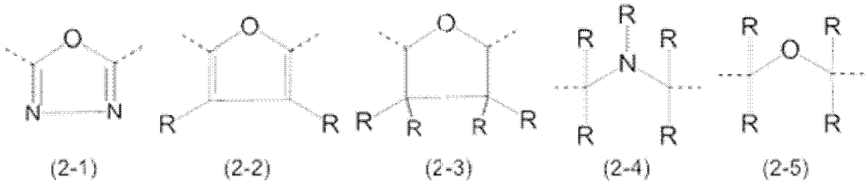 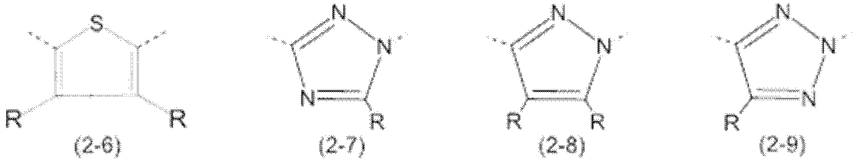 with R being H, D, or any covalently linked substituent being identical or different in each occurrence, and the dashed lines indicating the covalent linkage to the remainder of the macrocyclic ligand according to formula (1-1).

Here, the preferred macrocycle in the macrocyclic ring with its 18 macrocyclic atoms is constituted of subunits that are chemically robust, well-coordinating, and each individually having a high triplet energy. Again, this selection shall ensure that the energy as well as the electron in the excited state of Eu(II) in the 4d orbital is mostly confined on the central metal, thereby avoiding undesired charge transfer states or Dexter energy transfer. The most desirable properties for OLED operation, i.e., deep blue color and high electron binding energy in the ground and excited state are achieved by the furanyl fragment (2-2) and the purely aliphatic segments (2-4) and (2-5) as well as the analogon of (2-5) with O substituted by S, are thus preferred.

Further preferred are rotation- or mirror symmetric organic ligands such as derived from the generic structures G1 to G9: 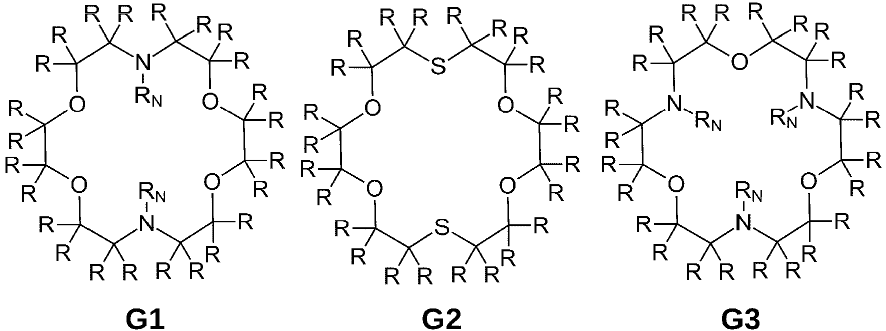 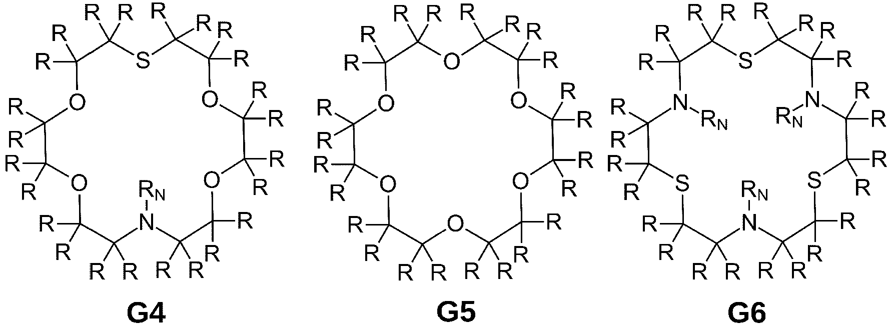 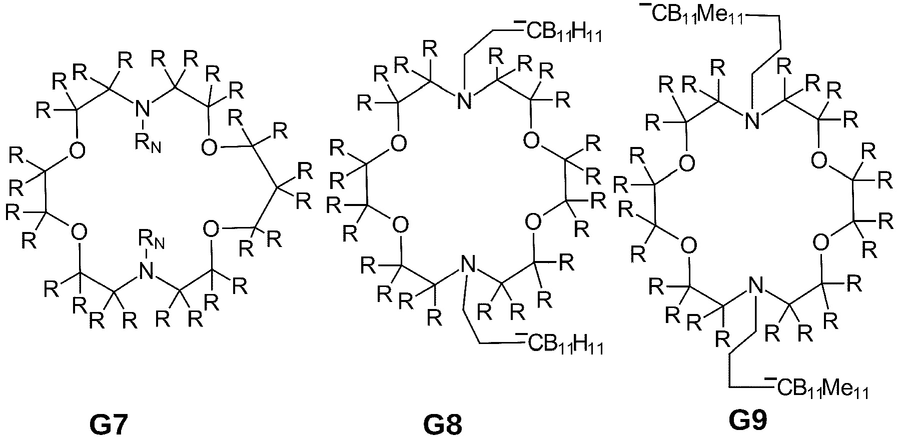 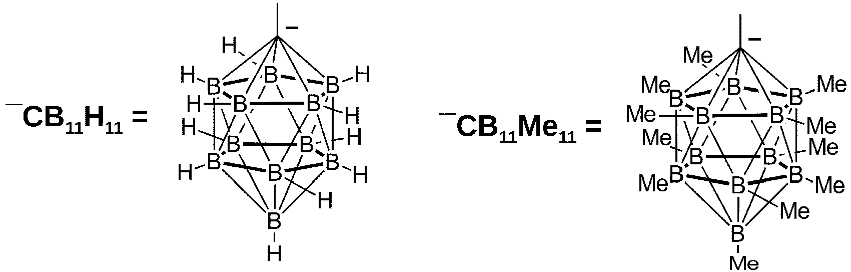 **R** being identical or different in each occurrence and being H, D, or any covalently linked monovalent organic group formed by removing a hydrogen atom from an organic molecule containing at least one hydrogen atom whereby two, three, or four instances of R being present on the same or neighboring carbon atoms can be fused, and **R_{N}** representing hydrogen, deuterium, or any covalently linked monovalent organic group formed by removing a hydrogen atom from an organic molecule containing at least one hydrogen atom.

This molecular design ensures favourable size of the macrocycle in the macrocyclic ring with 18 to 19 atoms. G1 to G7 are charge neutral, therefore two anions selected from the generic formulae (3-1) to (3-3) are present in the final coordination compounds according to the invention. G8 and G9 are twofold negatively charged by means of two identical carborate fragments according to formula (3-4), which are covalently attached using alkyl, in this case propyl or ethyl, spacer groups. The substituents R might be any charge neutral, organyl group that is formed by removing one hydrogen from a chemically stable organic molecule. However, substituents R, present on neighbouring carbon atoms in G1 to G9, may fuse to form aromatic or aliphatic cyclic groups. For example, 4 substituents R present on two neighbouring carbon atoms separated by an oxygen may fuse into a furanyl fragment, i.e., into formula (2-2) from above. The two R on each carbon atom together with the respective (macrocycle) ring carbon atom form a carbon-carbon double bond as in 1,3-butadien. See also E9 below. Similarly, 4 Rs present on two neighbouring macrocyclic carbon atoms may form together with them 1,2-phenylene group. See also E10 below. By adding further fused rings in the 3,4- or 4,5-position, a naphtylene or phenanthrene group can be formed.

In further preferred embodiments, the metal organic coordination compound according to the invention is selected from E1 to E17: 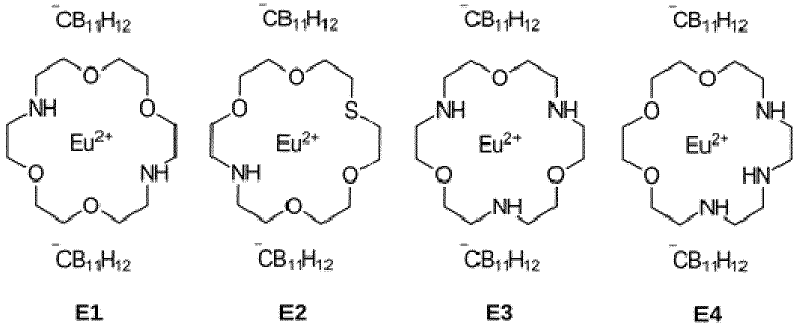 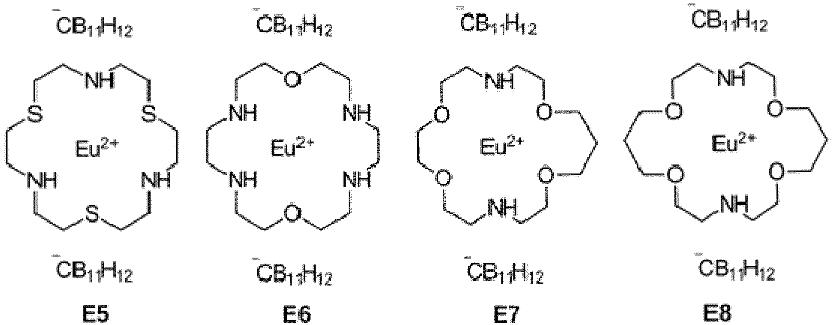 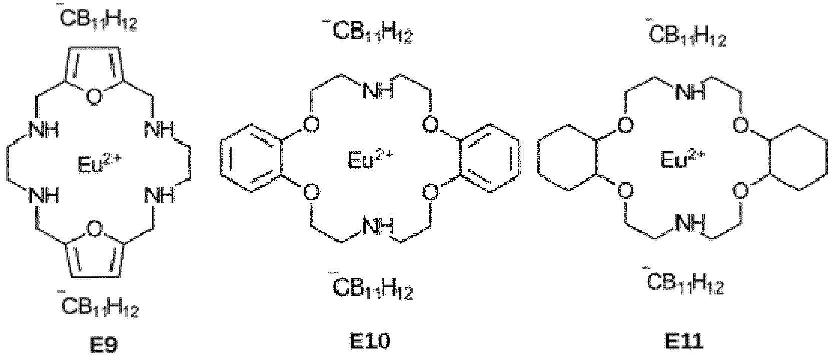 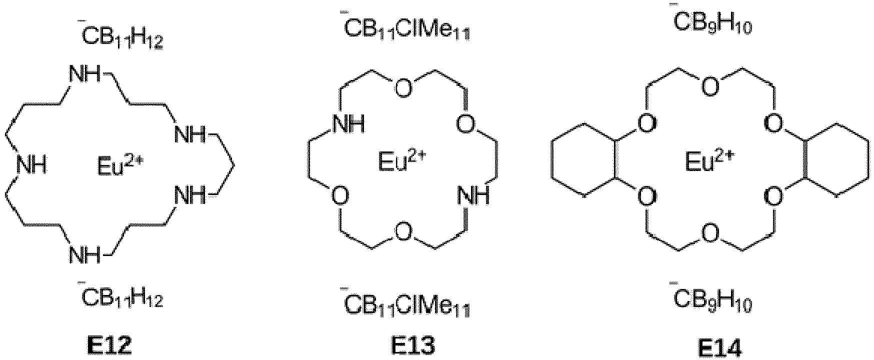 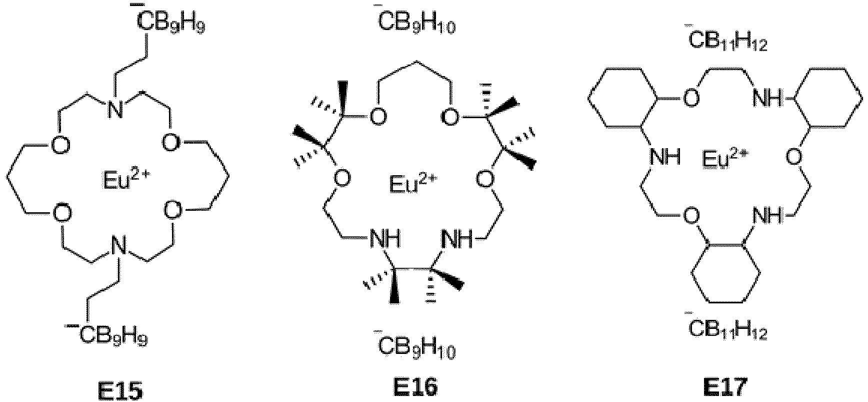 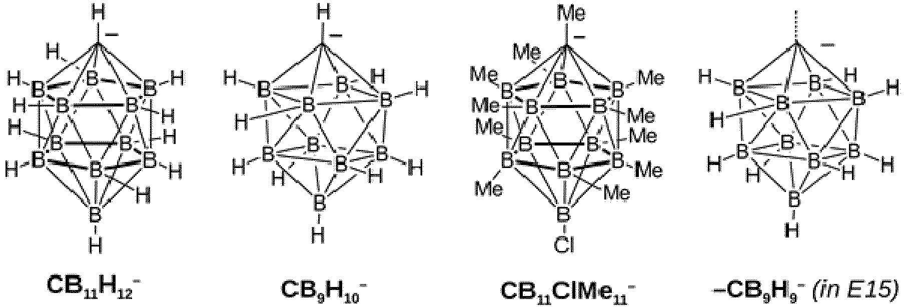 with **R** being identical or different in each occurrence and being H, D, or any covalently linked monovalent organic group formed by removing a hydrogen atom from an organic molecule containing at least one hydrogen atom whereby two, three, or four instances of R being present on the same or neighboring carbon atoms can be fused, and **R_{N}** representing hydrogen, deuterium, or any covalently linked monovalent organic group formed by removing a hydrogen atom from an organic molecule containing at least one hydrogen atom.

All those embodiments combine a perfect ring size to accommodate divalent Europium to achieve sufficient geometric stabilization with the right amount of electron stabilization provided by the various hetero atoms. Here, E1 to E6, E9-11, E13, and E17 comprise of 18 atoms as part of the macrocyclic ligand. Slightly larger cavities are used in E7 featuring 19 atoms, while E8, E12, and E15 consist of 20 atoms as part of the macrocyclic ring. With the exception of E12 having only 5 heteroatoms, always 6 heteroatoms coordinate to the central metal cation. The heteroatoms are selected from O, N, and S here, and their relative abundance as part of the macrocycle of the macrocyclic ring is fine-tuned to arrive at perfect deep blue color point and electron-binding energies suitable for OLED devices. All those 14 coordination compounds comprise of closo-carborate anions. In E1 to E12 they are non-substituted, while E13 comprises two free anions with a single chlorine and eleven methyl substituents. In compounds E14 and E16, a smaller 10-vertex closocarborane is exemplified. In E15, the 10-vertex carborate fragment is covalently attached to the nitrogen atoms of the macrocyclic ligand via ethyl spacers. Thus, the organic ligand excluding the metal cation is twofold negatively charged. Such modifications of the basic anions help to fine tune emission color and energy levels for optimal function in OLED device. Most of the embodiments E1 to E17 are based on non-substituted macrocyclic organic ligands, and there for E1 to E8, E12 and E13 above show the macrocycle surrounding the Eu(II). In E15 two nitrogen atoms are substituted to covalently attach two anionic groups. In E9 to E11, E14, and E17, neighbouring carbon atoms are substituted and fused into cyclohexane, furan, and benzene rings. In E16, every other ethyl bridge connecting the nitrogen donor atoms is fourfold substituted by methyl groups. E11 illustrates the difference between "macrocycle" and "macrocyclic organic ligand" as used herewith. Whereas E11 shows the macrocyclic organic ligand (as part of the metal-organic coordination compound) which includes the two cyclohexylene rings and all hydrogens, the macrocycle describes and encompasses "only" the shortest sequence of 18 atoms forming the inner ring surrounding the Eu(ll) and thus ignores the possible substituents.

In various embodiments, the coordination compound is covalently linked to the backbone of a polymer with a molecular weight Mₙ above 1000 g/mol. Here the mixture is a polymeric compound, which might have benefits in terms of very high glass transition temperatures or may ease the processing of the mixtures using wet solution techniques.

In various preferred embodiments, the metal-organic complex according to the invention is imbedded into at least one second charge-neutral organic compound at 0.1-99.0 vol%. Such a second material may, for example, be a charge transport organic material capable of transporting positive or negative charges.

In various preferred embodiments, the metal-organic coordination compound according to the invention is used in an organic electronic device, comprising a first electrode, a second electrode, and an organic layer arranged such that it is electrically interposed between the first and second electrodes, wherein the organic layer comprises a pure layer of the metal-organic coordination compound or the compound comprised in a mixtures or the polymeric compound described above in various embodiments. Further preferred are embodiments with the organic electronic device being an organic light emitting diode, an organic photodetector, or a photovoltaic cell.

In preferred embodiments, the layer comprising the metal-organic coordination compound according to any of the embodiments described above is deposited from gas phase, in particular using evaporation and/or sublimation and/or carrier gas processes. Thermal sublimation and/or evaporation are the standard processes used in industrial consumer display production processes and given the high sublimation yield of the materials according to the invention, fabricating organic electronic devices, in particular OLED consumer displays, is readily possible using those processes. Alternatively, the coordination compound applied in the organic electronic device is deposited from solution, which may offer advantages in terms of throughput or production cost.

### Brief Description of the Drawings

In the drawings, like reference characters generally refer to the same parts throughout the different views. The drawings are not necessarily to scale, emphasis is instead generally being placed upon illustrating the principles of the invention. In the following description, various aspects of the invention are described with reference to the following drawings, in which:
**FIG. 1** exemplifies macrocyclic organic ligands that might be present in metal-organic complexes according to the invention.
**FIG. 2** exemplifies *closo* and *nido* borate type anions that might be present in metal-organic complexes according to invention.
**FIG. 3**, and **FIG. 4** illustrate schematic cross sections of organic electronic devices according to various aspects.
**FIG. 5, FIG. 6, and FIG. 7** depict emission spectra of various coordination compounds.

### Detailed Description

The following detailed description refers to the accompanying drawings that show, by way of illustration, specific details and aspects in which the invention may be practiced.

The word "exemplary" is used herein to mean "serving as an example, instance, or illustration". Any aspect or design described herein as "exemplary" is not necessarily to be construed as preferred or advantageous over other aspects or designs.

Various embodiments relate to metal-organic compounds, including a divalent Europium cation coordinated by a macrocyclic organic ligand and various *closo-* and nido-borate type anions and the applications of those compounds.

In this description, a coordination compound is taken to mean a compound where the central active metal, divalent Europium, is coordinated without a direct metal carbon bond. The terms complex, metal-organic compound, and coordination compound will be used interchangeably.

The term "macrocyclic" ring, molecule, or ligand refers to organic molecules comprising at least 9 atoms independently in each occurrence selected from O, S, Si, C, B, N, P as part of cyclic chain of atoms with at least two of the bonds between the ring forming atoms being aliphatic. A crown ether, comprising of at least 3 -C-C-O- fragments is an example of a macrocyclic molecule.

The "aza" designation in the fragments described herein, i.e., aza-crown ether, etc. means that one or more carbon atom or (other) heteroatom of a parent compound is replaced by a nitrogen atom, without any limitation. For example, in a crown ether, -O- is replaced by -NH- to give the respective aza crown ether. One of ordinary skill in the art can readily envision other nitrogen analogs of the aza-derivatives, and all such analogs are intended to be encompassed by the terms as set forth herein.

As used herein, "deuterium" refers to an isotope of hydrogen. Deuterated compounds can be readily prepared using methods known in the art.

It is to be understood that when a molecular fragment is described as being a substituent or otherwise attached to another moiety, its name may be written as if it were a fragment (e.g., phenyl, phenylene, naphthyl, dibenzofuryl) or as if it were the whole molecule (e.g., benzene, naphthalene, dibenzofuran). As used herein, these different ways of designating a substituent or attached fragment are considered to be equivalent.

The terms "halo," "halogen," and "halide" are used interchangeably and refer to fluorine, chlorine, bromine, and iodine.

In this description, the arylene is a divalent organic fragment that is derived from an aromatic or heteroaromatic hydrocarbon (arene or heteroarene) by removing two hydrogen atoms from the aromatic or heteroaromatic hydrocarbon, preferably from different carbon and/or hetero atoms. One example is a (hetero) aromatic hydrocarbon that has had hydrogen atoms removed from two, preferably adjacent, hydrogen-bearing atoms (in case of aromatic hydrocarbon two carbon atoms, in case of heteroaromatic hydrocarbons two atoms selected from carbon and heteroatoms). An aromatic hydrocarbon or arene (or sometimes aryl hydrocarbon) is a cyclic hydrocarbon comprising only sp²-hybridized carbon atoms, leading to a delocalized π-system. Such (hetero) aromatic units may as well improve the charge transport or charge capture properties of the metal organic coordination compounds. Yet, preference is given to (hetero) aromatic groups with sufficiently high triplet energy to confine the excitation to the central Europium atom and, again, preference is given to relatively strong electron-donating aromatic groups without heteroatoms or with only oxygen or nitrogen as heteroatoms. Each (hetero) aromatic group(s) preferably contain(s) 1 to 5, more preferably 1 to 4, most preferably 1, 2 or 3, preferably fused or annelated, ring structures, preferably (hetero) aromatic) ring structures that contain 0 or 1 non (hetero)aromatic ring, like in a benzofuran group. An alkylene is a divalent organic fragment that is derived from an alkyl group by removing one additional substituent.

The term "alkyl" refers to and includes both straight and branched alkyl chain radicals and can furthermore also include cycloalkyl radicals. Preferred alkyl groups are those containing from one to fifteen, preferably one to ten, more preferably one to five carbon atoms and includes methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, and the like. Additionally, the alkyl group is optionally substituted, e.g., by halogen, preferably fluorine, or cycloalkyls.

The term "cycloalkyl radical" refers to and includes monocyclic, polycyclic, and spiro alkyl radicals. Preferred cycloalkyl groups are those containing 3 to 12, preferably 3 to 8 ring carbon atoms and includes cyclopropyl, cyclopentyl, cyclohexyl, bicyclo[3.1.1]heptyl, spiro[4.5]decyl, spiro[5.5]undecyl, adamantyl, and the like. Additionally, the cycloalkyl group is optionally substituted, e.g., by halogen, deuterium, alkyl or heteroalkyl.

The terms "heteroalkyl" or "heterocycloalkyl" refer to an alkyl or a cycloalkyl radical, respectively, having at least one carbon atom replaced by a heteroatom. Preferably the at least one heteroatom is selected from O, S, N, P, B, and Si, more preferably O, or N. Preferably 1 to 5, more preferably 1 to 3, most preferably 1 or 2 heteroatoms are present in the radical. The radical can be covalently linked with the remainder of the molecule via a carbon or heteroatom (e.g., N). Additionally, the heteroalkyl or heterocycloalkyl group is optionally substituted as indicated for alkyl and cycloalkyl.

The term "alkenyl" refers to and includes both straight and branched chain alkene radicals. Alkenyl groups are essentially alkyl groups with more than one carbon atom that include at least one carbon-carbon double bond in the alkyl chain. Cycloalkenyl groups are essentially cycloalkyl groups that include at least one carbon-carbon double bond in the cycloalkyl ring. The term "heteroalkenyl" as used herein refers to an alkenyl radical having at least one, preferably 1 to 5, more preferably 1 to 3, most preferably 1 or 2 carbon atoms replaced by a heteroatom. Preferably, the at least one heteroatom is selected from O, S, N, P, B, and Si, more preferably O, S, or N. Preferred alkenyl/cycloalkenyl/heteroalkenyl groups are those containing two/three/one to fifteen carbon atoms. Additionally, the alkenyl, cycloalkenyl, or heteroalkenyl group is optionally substituted, as indicated above.

The terms "aralkyl" or "arylalkyl" are used interchangeably and refer to an alkyl group that is substituted with an aryl group. Additionally, the aralkyl group is optionally substituted, as indicated for alkyl and aryl.

The term "heterocyclic group" refers to and includes aromatic and non-aromatic cyclic radicals containing at least one, preferably 1 to 5, more preferably 1 to 3 heteroatoms. Preferably the at least one heteroatom is selected from O, S, N, P, B, and Si, more preferably O, S, or N. Hetero-aromatic cyclic radicals may be used interchangeably with heteroaryl. Preferred hetero-non-aromatic cyclic groups are those containing 3 to 7 ring atoms which includes at least one hetero atom, and includes cyclic amines such as morpholino, piperidino, pyrrolidino, and the like, and cyclic ethers/thio-ethers, such as tetrahydrofuran, tetrahydropyran, tetrahydrothiophene, and the like. Additionally, the heterocyclic group may be optionally substituted, e.g., by halogen, deuterium, alkyl or aryl. The heterocyclic group can be covalently linked with the remainder of the molecule via carbon and/or heteroatoms, preferably one carbon or nitrogen atom. The heterocyclic group can as well be linked with two carbon and/or heteroatoms with the remainder of the molecule.

The term "aryl" refers to and includes both single-ring aromatic hydrocarbon groups and polycyclic aromatic ring systems. The polycyclic rings may have two or more rings in which two carbons are common to two adjoining rings (the rings are "fused") or wherein one carbon is common to two adjoining rings (e.g., biphenyl) wherein at least one of the rings is an aromatic hydrocarbon group, e.g., the other rings can be cycloalkyls, cycloalkenyls, aryl, heterocycles, and/or heteroaryls. Preferred aryl groups are those containing three to eight aromatic carbon atoms. Especially preferred is an aryl group having six carbons. Suitable aryl groups include phenyl, and radialene. Most preferred is phenyl that can be substituted by non-aromatic groups. Additionally, the aryl group is optionally substituted, e.g., by halogen, alkyl, heteroalkyl, or deuterium. The aryl group is connected to the remainder of the ligand via one or two carbon atoms.

The term "heteroaryl" refers to and includes both single-ring aromatic groups and polycyclic aromatic ring systems that include at least one heteroatom. The heteroatoms include, but are not limited to and are preferably selected from O, S, N, P, B, and Si. In many instances, O, S, or N are the preferred heteroatoms. Hetero-single ring aromatic systems are preferably single rings with 5 or 6 ring atoms, and the ring can have from one to five/six, preferably 1 to 3 heteroatoms. The hetero-polycyclic ring systems can have two or more rings in which two atoms are common to two adjoining rings (the rings are "fused") or wherein one carbon is common to two adjoining rings (e.g., bipyridine) wherein at least one of the rings is a heteroaryl, e.g., the other rings can be cycloalkyls, cycloalkenyls. The hetero-polycyclic aromatic ring systems can have from 1 to 5, preferably 1 to 3 heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyrrole, pyrazole, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably 1,3,4-Oxadiazole, furan, thiophene, 1,2,4-triazole, 1,2,3-triazole, and pyrazole groups, from which one hydrogen atom has been removed from a hydrogen-bearing carbon or heteroatom to form the covalent link to the remainder of the molecule. The heteroaryl group with two hydrogens removed can as well be linked with two carbon and/or heteroatoms with the remainder of the molecules, in which case the heteroaryl group can be part of a larger cyclic group. Additionally, the heteroaryl group is optionally substituted, e.g., by halogen, deuterium, alkyl or aryl.

Generally, aryl and heteroaryl groups with triplet level > 2.5 eV or more preferably >2.6 eV or most preferably > 2.7 eV such as the groups derived from benzene, furan, dibenzofuran, dibenzoselenophene, carbazole, imidazole, pyridine, pyrazine, pyrimidine, triazine, and the respective aza-analogs of each thereof are of particular interest. There are many possible ways to probe the triplet energy level of an organic molecule. A simple way is to use suitable quantum mechanical calculations. A more experimental way takes the onset of the phosphorescence spectrum, which is usually detected using gated spectroscopy, as pioneered by Romanovskii et al. "Phosphorescence of π-conjugated oligomers and polymers." Physical Review Letters 84.5 (2000): 1027. In combination with high triplet energy, generally preference is given to benzene or low polarizable, hard heteroaryl groups, such as 1,3,4-Oxadiazole, furan, thiophene, 1,2,4-triazole, 1,2,3-triazole, and pyrazole carbazole, dibenzofuran, and benzofuran groups.

The alkyl, cycloalkyl, heteroalkyl, heterocycloalkyl, alkenyl, cycloalkenyl, heteroalkenyl, alkynyl, aralkyl, heterocyclic group, aryl, and heteroaryl groups or residues, as used herein, are independently unsubstituted, or independently substituted, with one or more (general) substituents, preferably the substituents mentioned above.

Preferably, the (general) substituents are selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof with the number of carbon atoms and heteroatoms as defined above for the respective term. Furthermore, one or two substituents can be selected from polymer chains which can be covalently linked with the remainder of the molecule by a suitable organic spacer. Therefore, the cyclic organic ligand can be covalently linked with a polymer chain or a polymer backbone.

In some instances, the preferred general substituents are selected from the group consisting of deuterium, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term.

In yet other instances, the more preferred general substituents are selected from the group consisting of deuterium, alkyl, cycloalkyl, aryl, heteroaryl, and combinations thereof with the number of carbon atoms and heteroatoms as defined above for the respective term.

The terms "substituted" and "substitution" refer to a substituent other than H that is bonded to the relevant position, e.g., a carbon or nitrogen. For example, when R represents mono-substitution, then one R must be other than H (i.e., a substitution). Similarly, when R represents di-substitution, then two of R must be other than H. Similarly, when R represents no substitution, R, for example, can be a hydrogen for available valencies of straight or branched chain or ring atoms, as in carbon atoms for benzene and the nitrogen atom in pyrrole, or simply represents nothing for ring atoms with fully filled valencies, e.g., the nitrogen atom in pyridine. The maximum number of substitutions possible in a straight or branched chain or ring structure will depend on the total number of available valencies in the ring atoms or number of hydrogen atoms that can be replaced. All residues and substituents are selected in a way that a chemically stable and accessible chemical group results.

Any type of substituent can replace a hydrogen atom in an organic or heterorganic group of compound (a) or (b) or the second organic layer above, as long as it results in a chemical compound which is stable under conditions that occur in an OLED display device or organic electronic device and which does not chemically react with other compounds and components of the OLED display device or organic electronic device.

As used herein, "combinations thereof" indicates that one or more members of the applicable list are combined to form a known or chemically stable arrangement that one of ordinary skill in the art can envision from the applicable list. For example, an alkyl and deuterium can be combined to form a partial or fully deuterated alkyl group; a halogen and alkyl can be combined to form a halogenated alkyl substituent; and a halogen, alkyl, and aryl can be combined to form a halogenated arylalkyl. In one instance, the term substitution includes a combination of two to four of the listed groups. In another instance, the term substitution includes a combination of two to three groups. In yet another instance, the term substitution includes a combination of two groups. Preferred combinations of substituent groups are those that contain up to fifty atoms that are not hydrogen or deuterium, or those which include thirty to forty atoms that are not hydrogen or deuterium, or those that include up to 30 atoms that are not hydrogen or deuterium counted for all substituents of a given molecule, or for the respective molecule in total. In various embodiments, a combination of substituent groups will include up to twenty atoms that are not hydrogen or deuterium, counted for all substituents of a given molecule.

In some instances, a pair of adjacent residues can be optionally joined (i.e., covalently linked with each other) or fused into a ring. The preferred ring formed therewith is a five-, six-, or seven-membered carbocyclic or heterocyclic ring, including both instances where the portion of the ring formed by the pair of substituents is saturated and where the portion of the ring formed by the pair of substituents is unsaturated. As used herein, "adjacent" means that the two substituents involved can be on the same ring next to each other, or on two neighboring rings having the two closest available substitutable positions, such as 2, 2' positions in a biphenyl, or 1, 8 position in a naphthalene, or 2,3-positions in a phenyl, or 1,2-positions in a piperidine, as long as they can form a stable fused ring system. In some instances, a pair of non-adjacent substituents can be optionally joined usually by an, at least partial, alkane or heteroalkene chain of atoms, thereby forming another macrocyclic ring system as part of the macrocyclic ring present as part of the coordination compound. In some instances, a pair of substituents present on the same carbon atom can be optionally joined, i.e., into an aryl or heteroaryl group, thus, forming a spiro linkage on said particular carbon atom.

In this description, the term organyl refers to any chemically stable organic arrangement of atoms where one or more hydrogen atom has been removed such as to use those free vacancies to covalently link the organic group with another molecular entity. Thus, the term organyl encompasses the majority of the above defined organic groups e.g., fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term. The term organyl shall especially as well encompass sufficiently chemically stable negatively charged species, such as anions, or anionic side groups.

Organic groups are derived or formed by removing one or more hydrogen atoms from organic molecules containing at least a corresponding number of hydrogen atoms. For example, benzene results in phenyl or phenylene groups by removing one or two hydrogen atoms, resulting in a monovalent or divalent organic group.

Suitable organic molecules and groups are as identified above, e.g. alkyl, aryl, heteroaryl groups.

In this description, an electroluminescent coordination compound is any material that is able to emit light upon electrical excitation, resulting from recombination of electrons and holes. It shall be irrelevant in this context whether the recombination of the electrons and holes takes place directly on the electroluminescent compound or first an excitation is formed on a different compound and subsequently transferred to the electroluminescent compound. Further, it shall be irrelevant in this context whether the light emitted is visible for the human eye or not. Especially, compounds emitting infrared and near ultra-violet emission shall be explicitly included. Further, the mixture comprising the electroluminescent coordination compound does not necessarily have to be used in an electronic device but, for example, may be used as a dye or a contrast enhancement medium for magnetic resonance tomography. In particular it may be used for down-converting high energy photons after optical excitation.

In the OLED community, the emission is usually classified as fluorescence if it originates from an optically allowed (spin-conserving) transition and decays within a few nanoseconds, such as the transition from the first excited singlet state to the singlet ground state, or, as phosphorescence if the emission originates from a spin-forbidden transition and decays with a few micro-milliseconds, such as from the first excited triplet state to the singlet ground state. The emission from the first electronically excited divalent Europium results from an optically allowed spin-conserving d-f transition, which, due to small overlap between the 5d and 4f orbitals, has a rather long excited state lifetime of 1.2 µs (compared to typical fluorescence). Thus, it clearly is neither fluorescent, nor phosphorescent in the typical context used in OLED community. It should correctly be referred to as emission from an intrametallic transition.

All embodiments of the invention contain a metal-organic coordination compound, wherein the coordination compound comprises a divalent Europium and a macrocyclic organic ligand, with the macrocyclic organic ligand being either charge neutral and two identical borate or carborate anions selected from (3-1) to (3-3) are present in the coordination compound: 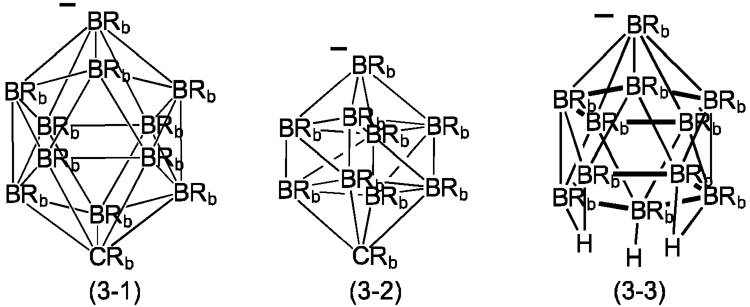 or being twofold negatively charged by means of covalent attachment of two borate cluster fragments selected from (3-4) or (3-8) 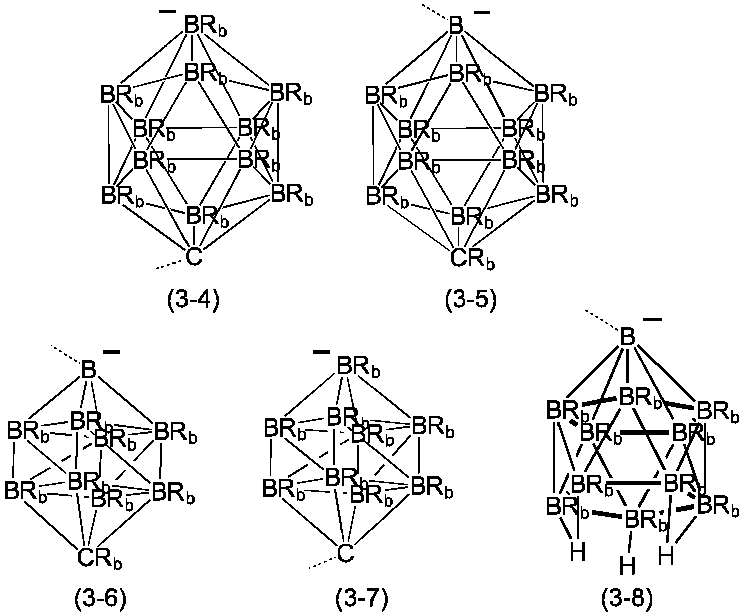 with the dashed lines indicating the covalent linkage to the remainder of the macrocyclic ligand, and **R_{b}** present in (3-1) - (3-8) independently in each occurrence represents either hydrogen, deuterium, fluorene, chlorine, bromine, or an alkyl chain - preferably methyl.

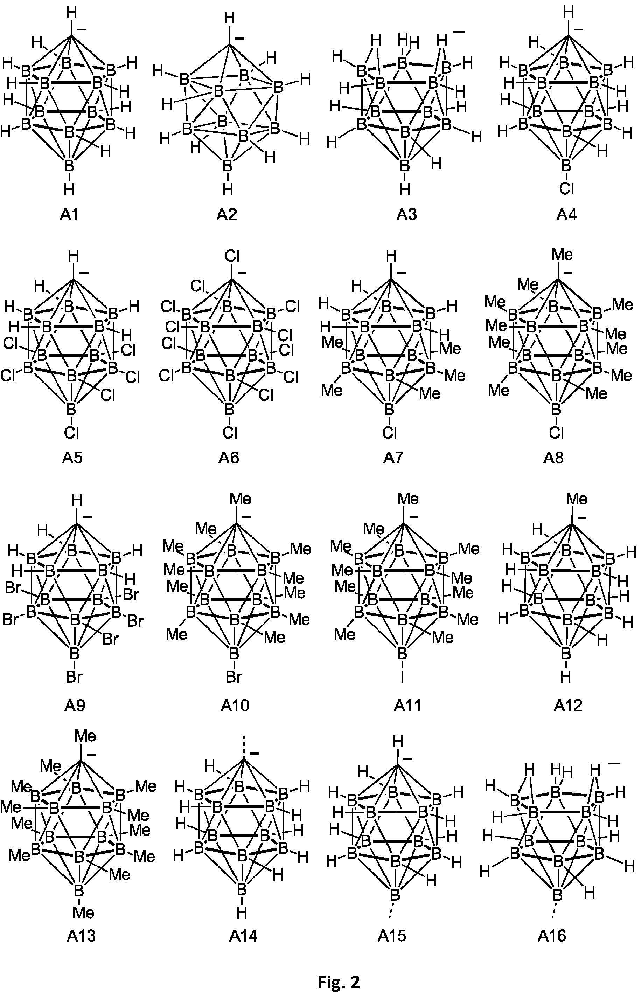

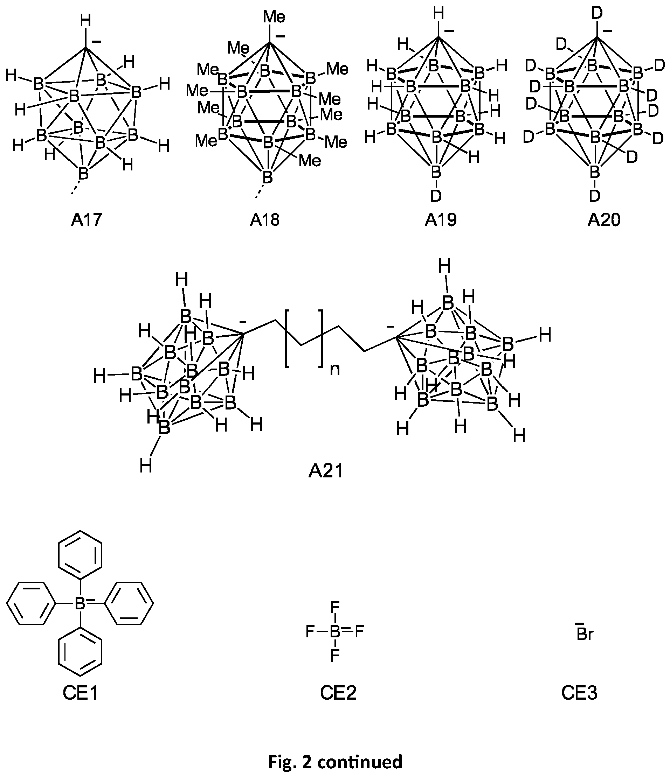

All metal-organic coordination compounds according to the invention comprise two identical substituted or non-substituted borates of the closo- and/or nido-type or closo-carborate anions. Without limiting the scope of the invention, A1 to A21 depicted in Fig 2 illustrate examples of anions that might be present in the metal-organic coordination compounds according to the invention. In various embodiments, those anions are fully non-substituted, i.e. Rb is equal to H in all instances such as in A1 to A3 in Fig. 2. In various other embodiments alkyl substitution may be present. For example, each instance of Rb present in (3-1) might be a methyl group, such as shown in example A13 in Fig. 2. Alternatively, it might be that only the carbon atom present for example in (3-1) or (3-2) is substituted with an alkyl chain, such as in example A12 in Fig. 2. In other instances, 6 instances of Rb present for example in (3-1) or (3-4) might be alkylated, for example using methyl substitutions. In yet other embodiments, some or all instances of Rb are halogenated using fluorene, chlorine, bromine, compare A4 to A11 in Fig. 2. For halogenation, preference is given for chlorine and bromine over fluorene. While any number of instances Rb might or might not be halogenated, preference is generally given to half substituted anions, i.e., where 6 instances of are halogenated in case of (3-1), (3-4), and (3-5) and where 5 instances of Rb are halogenated in case of (3-2), (3-6), (3-7) and (3-8). A5 and A9 shown in Fig. 2 represent examples of half-halogenated anions. Further preference is given to singly halogenated anions, where just one instance of Rb is halogenated, for example chlorinated. Here, preference is given for substituting the single Rb opposite the carbon atom present in (3-1), (3-2), (3-4), and (3-5) by chlorine, compare A4 in Fig. 2. In specific examples, substitution of different types may be present. For example, it may be that the carbon atom present in (3-1) is methylated while 6 further instances of Rb are halogenated, for example using bromine. A7 to A12 in Fig. 2 exemplify the situation of mixed substitutions present in anions according to the invention.

In all metal-organic coordination compounds according to the invention, there is a macrocyclic organic ligand present that coordinates the divalent Europium metal. The macrocyclic organic ligand excluding the metal may be charge neutral. In this case, the two positive charges of the Europium cation are charge compensated in the final coordination compound by two identical instances of *nido-*type or closo-carborate-type cluster anions depicted in (3-1) to (3-3) above. No other organic or inorganic anions, such as halogenides are present as part of the complex. Alternatively, the macrocyclic organic ligand excluding the metal cation is already twofold negatively charged. In this case, the two negative charges are substantially localized on two preferably identical anionic side groups being selected from the *closo* carborate or *nido*-type anions depicted in (3-4) to (3-8). Those anionic side groups are covalently attached to the remainder of the macrocyclic ligand using any organyl group, which is indicated by a dashed line in structures (3-4) to (3-8). Without limiting the scope of the invention, those linkers may be any branched, cyclic or linear alkane or heteroalkane chain. Yet, it may well be an alkyl, or heteroalky chain. Similar aryl or heteroaryl or any other cyclic group may form the linker or can be part of the linker. The linker itself may optionally be substituted, for example, using deuterium or halogens. Yet, without limiting the general scope of the invention, the preferred linker is a relatively short alkane chain of up to 10 carbon atoms, preferably linear C₁₋₁₂⁻ alkylene spacers. Particular for (3-4) and (3-7), even more preferred are alkane chains of 2 to 3 atoms, i.e. ethylene or propylene linkers. Particular for (3-5), (3-6), and (3-8), even more preferred are alkane chains of 2 to 3 atoms being linked via an ether group to the anions. Whilst any substitution of those particular linkers is clearly in the scope of the invention, preference is given to unsubstituted linkers.

To qualify as a macrocyclic organic ligand according to the invention and the generally accepted definition, preferably at least 9 macrocyclic atoms should be part of a single cyclic ring, the macrocycle. Clearly, the overall number of atoms being part of the macrocyclic organic ligand will be substantially higher; note that only those carbon or heteroatoms are counted as macrocyclic atoms that do form the cyclic closed ring system around the Eu(II), i.e. the "macrocycle" according to the invention. For example, the classical Dibenzo-18-crown-6 contains 18 macrocyclic atoms (12 carbon and 6 oxygen atoms, respectively, in the macrocycle), while overall 50 atoms are present. Within the scope of the invention are divalent Europium coordination compounds comprising organic macrocyclic ligands with preferably 9 or a higher number of macrocyclic atoms as part of the cyclic ring, the macrocycle. Yet, for good coordination of the divalent Europium, the cavity formed by the macrocyclic ring should neither be too small nor too large. This cavity, however, depends somewhat on the exact nature of the heteroatoms present in the macrocycle. For example, if nitrogen is substituted by sulfur in a particular aza-crown ether, the number of macrocyclic atoms does obviously not change, yet the cavity size will tendentially increase. Therefore, it is not possible to give an exact number of macrocyclic atoms that would always perform best. However, according to the invention, macrocycles comprising 12 to 48, preferably 15 to 24, and most preferably 18 to 20 macrocyclic atoms, i.e. in the macrocycle.

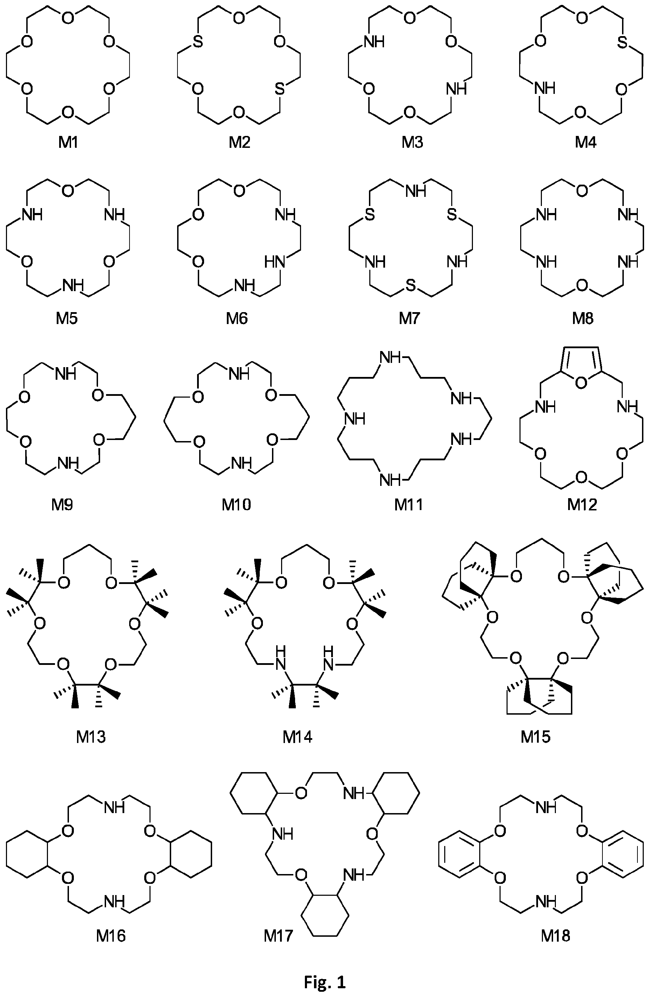

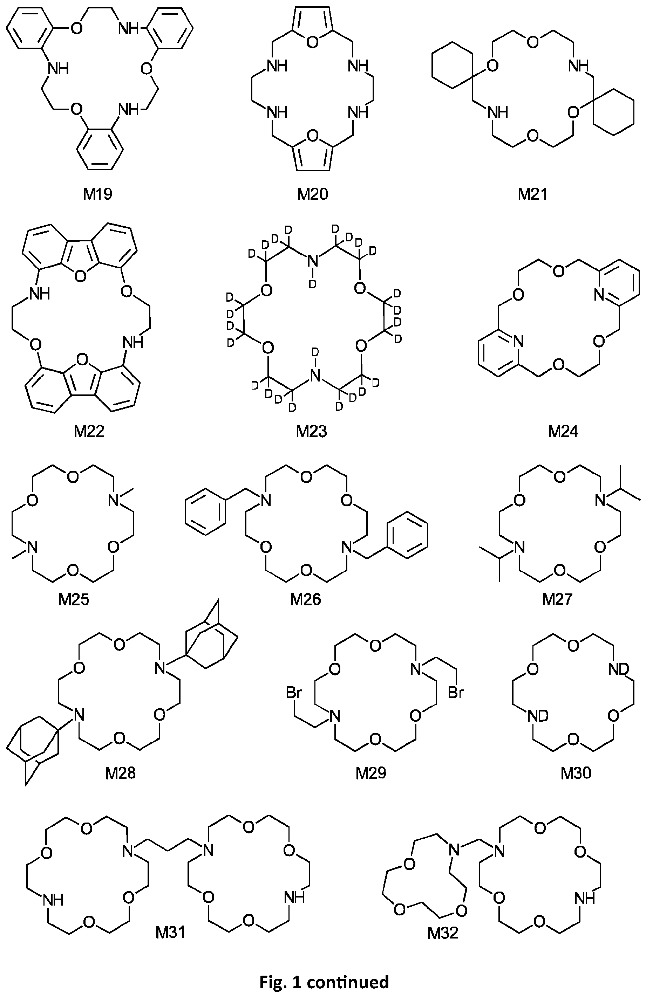

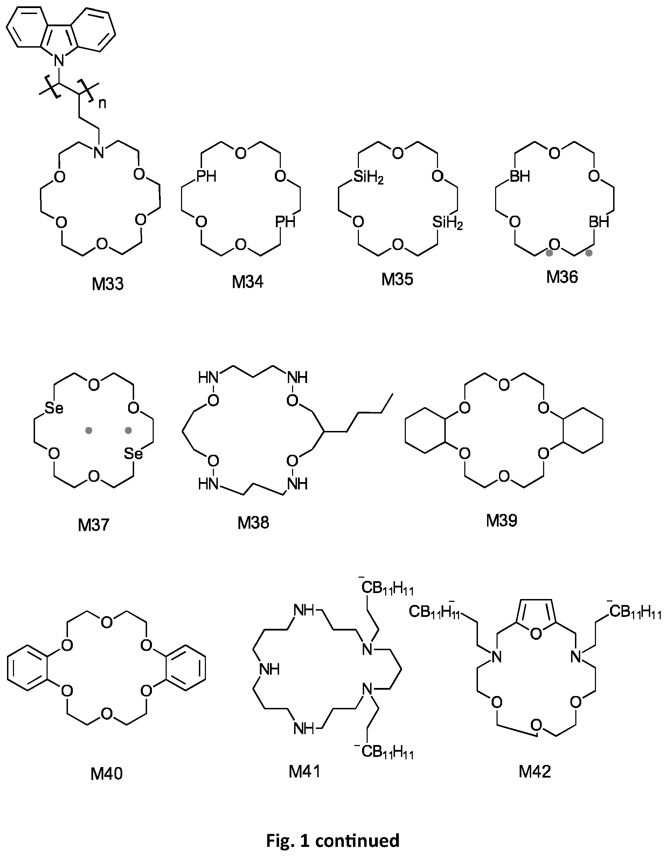

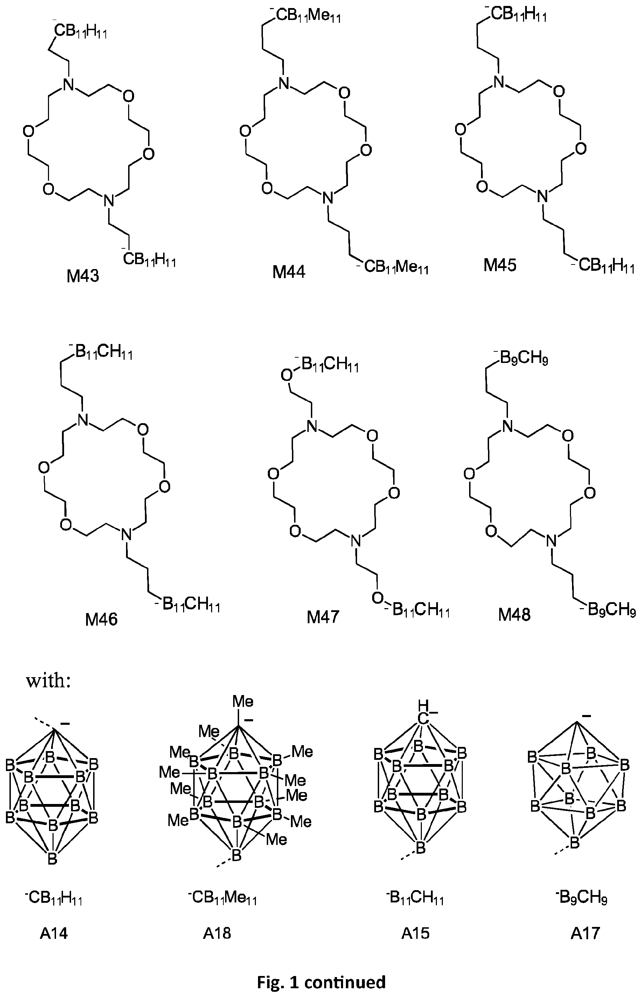

The macrocyclic organic ligand may comprise any element with at least two valencies that is able to form chemically stable cyclic rings. Indeed, an overwhelmingly large number of macrocyclic rings is described in the literature, for example, crown ethers, calixarenes, porphyrins, and cyclodextrins, which are all within the scope of the invention as long as they are charge neutral, or twofold negatively charged in the sense of the invention. Within the scope of the invention, according to one embodiment the macrocycle only comprises non-coordinating atoms, such as Si, or C. However, preference is given to macrocycles with a plurality of heteroatoms being present that are able to coordinate divalent Europium. In such a situation, two macrocyclic heteroatoms might be direct neighbors within the macrocyclic organic ligand and this might give very chemically robust macrocycles, which are in the scope of the invention. However, preference is given to macrocycles with the coordinating heteroatoms being separated by at least one carbon atom, and more preference is given to arrangements, where each two macrocyclic coordinating heteroatoms are separated from each other by two or three carbon atoms.
Any further ring structures in addition to the macrocycle, forming part of the macrocyclic organic ligand in addition to the macrocycle are preferably covalently linked with one macrocycle atom, or form part of the macrocycle by 1,2-, 1,3- or 1,4-linkage of the further ring structure, with the remainder of the macrocycle, and are not additionally covalently linked with other atoms of the macrocycle. Examples can be furanyl inserted in the ring (see M12, M20, M22 and M42 in Fig. 1), 1,2-cyclohexyl inserted in the ring (see M16, M17, M39, twofold in M15), 1,2-phenylene inserted in the ring (see M18, M19, M22, M40), spiro compounds like 1,1-cyclohexane (see M21), pyridine inserts in the ring (M24), other cyclic structure covalently linked with one macrocycle atom (M31, M32). Also fused groups are possible, like dibenzofuran (see M22, there replacing 5 macrocycle atoms by the sequence C-C-O-C-C).

Therefore, the preferred macrocyclic organic ligand might be described by formula (1-1): 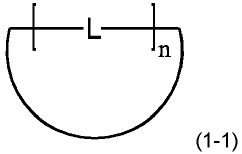 with n being 4-8, preferably 4-6, most preferably 6; and L being independently in each occurrence a divalent organic group formed by removing two hydrogen atoms from an organic molecule containing at least two hydrogen atoms with the shortest sequence of atoms linking each L with the remainder of the macrocycle in the macrocyclic ligand being 2 to 4, preferably 3 atoms, and with one of those 2 to 4, preferably 3 atoms independently for each L being selected from the heteroatoms B, N, P, S, Se, Si or O, preferably N, or O, the other atoms of those atoms being carbon, wherein preferably the heteroatoms in the macrocycle are non-neighboring.

Formula (1-1) describes macrocyclic organic ligands with a minimum of 8 and a maximum of 32 macrocyclic atoms in the macrocycle (n = 4, shortest sequence in L = 2 atoms; n = 8, shortest sequence in L = 4 atoms). In each instance of **L,** there is at least one heteroatom present. Thus, there are 4 to 32 heteroatoms preferably selected from N, P, O, S, or Se present that are able to coordinate to the divalent Europium. The linker L represents any (divalent) organyl group, such as, but not limited to, a linear (divalent) heteroalkane, a (divalent) heteroaryl, or any other (divalent) heterocyclic group. All those groups may be further substituted, for example, instead of a simple linear (divalent) heteroalkene, a branched (divalent) heteroalkene chain might be present. For (divalent) cyclic organyl groups, always the shortest sequence of atoms linking the (divalent) organyl group to the remainder of the ligand according to formula (1-1) shall constitute the macrocyclic atoms, i.e., should be 2-4, preferably 3 atoms. For example, if L represents a (divalent) furanyl group covalently connected to the remainder of the molecule in the 2,5 positions, then the shortest sequence of atoms is 3 and one oxygen is present. Similarly, if **L** represents a (divalent) pyrazinyl group covalently connected to the remainder of the molecule in the 2,6 positions, then the shortest sequence of atoms is 3 and one nitrogen is present. There is also a longer sequence of 5 atoms, which also has one nitrogen present, which, however, is not the shortest sequence of atoms according to the definition. Finally, an (divalent) aniline group connected via the 2,6 positions is considered. Again, the shortest sequence of atoms is 3, yet none of those 3 atoms is a heteroatom such as N, P, S, or O. The nitrogen present in aniline is not part of the shortest sequence of atoms in this situation, and as such this group does not fulfill the requirements of (1-1) and thus is not falling under the generic formula (1-1).

With respect to the generic formula (1-1) preference is generally given to **n** = 6 and for 3 atoms being in the shortest sequence of **L**. Further preference is given to heteroatoms O, N, S being present as part of L. Without limiting the general scope of the invention, L might independently in each occurrence be selected from (2-1) to (2-9): 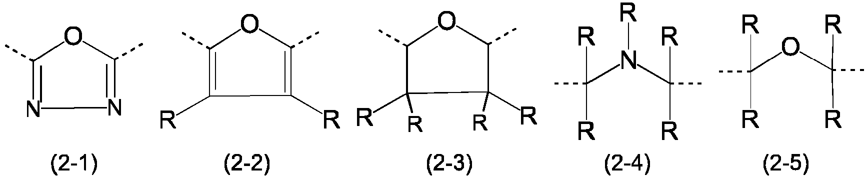 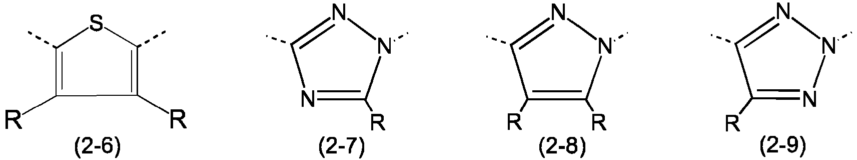 with R being H, D, or any covalently linked substituent being identical or different in each occurrence, and the dashed lines indicating the covalent linkage to the remainder of the macrocyclic ligand according to formula (1-1). In each linker (2-1) to (2-9) the shortest sequence of atoms is exactly three and there is a heteroatom in the center of this sequence that is able to coordinate the central Europium. In (2-7) to (2-9) there is a second non-coordinating macrocyclic heteroatom present, those linkers are thus asymmetric with respect to the sequence of atoms (-C-N-N-). Generally, symmetrical linkers are preferred, i.e., situations where one macrocyclic coordinating heteroatom is sandwiched by two carbon atoms, represented by the linkers depicted in (2-1) to (2-6). Further preference is given to weakly polarizable and chemically robust units, in particular preference is given to (2-2), (2-4), and (2-5). In (2-2) to (2-9) positions for optional substitutions are indicated by R. R may be any suitable organyl group formed by removing one hydrogen from an organic molecule containing at least one hydrogen atom, including, but not limited to, aryl, heteroaryl, or branched, cyclic or linear alkane or heteroalkene, halogens or deuterium. Additionally, two instances of R present in the same or two different instances of **L** may link to each other to form a cyclic group. For example, two Rs present in (2-3) in the same instance of **L** on neighboring carbon atoms may link to each other to form a cyclohexane group. Similarly, two Rs present in (2-3) in the same instance of L on the same carbon atom may link to each other to form a spiro group. In this context (2-1) to (2-3) represent particular embodiments of the more general linker (2-5); the former ones can be constituted by linking 2 or 4 instances of R. In various embodiments, instances of R have 1 to 80 atoms. Yet, in other instances, it will be 1 to 30 atoms. R is generally charge-neutral. However, in up to two instances, R might be a closocarborate anionic group according to formula (3-4) to (3-8).

Without limiting the general scope of the invention, further preferred examples of macrocyclic ligands according to formula (1-1) are illustrated by the generic formulae G1 to G9: 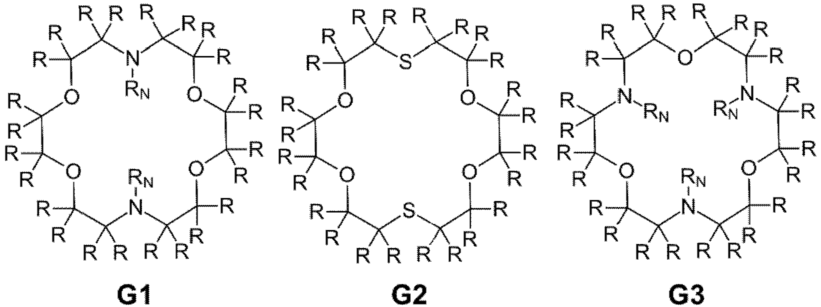 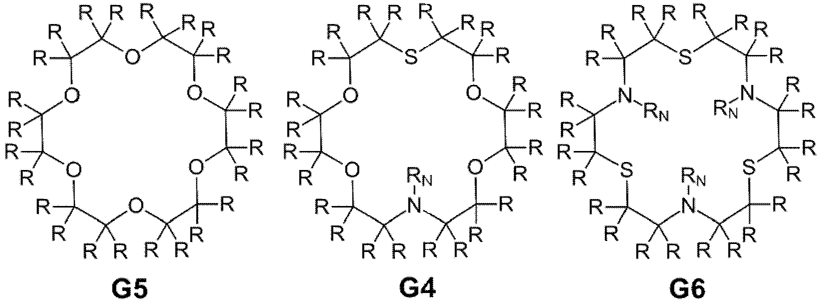 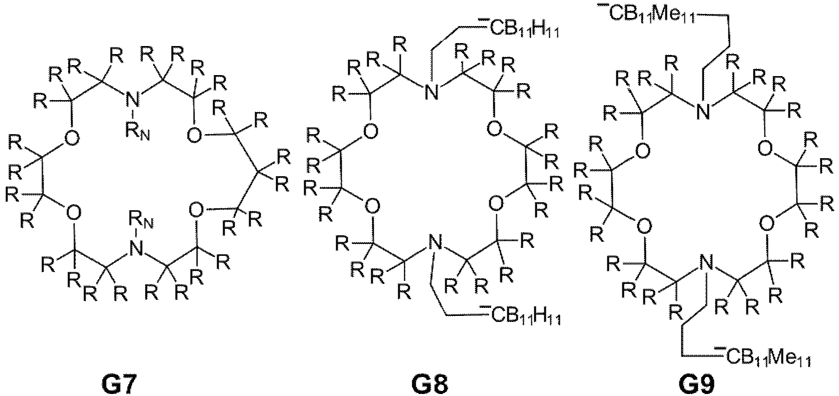 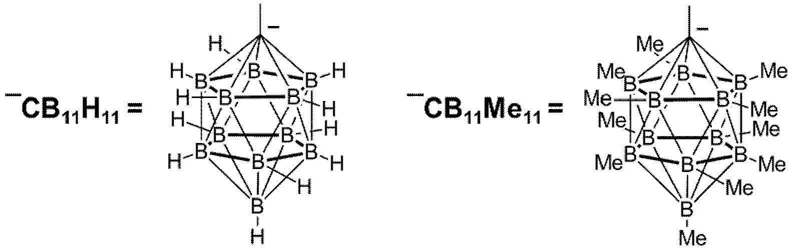 With **R** being identical or different in each occurrence and being H, D, or any covalently linked monovalent organic group formed by removing a hydrogen atom from an organic molecule containing at least one hydrogen atom whereby two, three, or four instances of R being present on the same or neighboring carbon atoms can be fused, and **R_{N}** representing hydrogen, deuterium, or any covalently linked monovalent organic group formed by removing a hydrogen atom from an organic molecule containing at least one hydrogen atom.

The preferred macrocyclic ligands G1 to G9 are in most cases substituted or non-substituted aza or thio crown ethers. Only in G5 all heteroatoms are oxygen, it is thus a conventional crown ether, with the nominal non-substituted macrocyclic ring being of the 18 crown 6 type. All generic structures G1 to G9 feature 6 macrocyclic heteroatoms able to coordinate to divalent Europium. Those 6 atoms are selected from O, S, and N. No two heteroatoms are direct neighbours, i.e., they are not linked directly by a chemical bond. Instead, all heteroatoms are separated from each other by at least two or three carbon atoms, i.e., by a linear ethylene or propylene chain segment. Therefore, the overall number of macrocyclic atoms present in most structures G1 to G9 is 18, i.e., 12 carbon and 6 hetero atoms. Only in G7 a different number of 19 macrocyclic atoms is exemplified by using a single propyl spacer between two oxygen heteroatoms. The Rs depicted in G1 to G9 again represent any organyl group formed by abstracting a hydrogen atom from a chemical stable group. However, like above, the Rs should not be electrically charged. Therefore, metal-organic coordination compounds according to the invention that are based on G1 to G7 linear ethylene or propylene comprise two anions selected from formula (3-1) to (3-3). G8 and G9 already contain covalently linked anions according to formula (3-4) and are thus two-fold negatively charged and will form a charge neutral coordination compound together with the divalent Europium.

The substituents R may be linear or branched alkane side chains if a good solubility in non-polar solvents is needed. For other applications, coordination compounds with high thermal stability, for example, suitable for evaporation or sublimation, may be needed. In this case, small and light weight substituents are preferably employed, i.e., organyl fragments with individually less than 20 atoms and or individually having a molecular weight of less than 100 g/mol. Similarly, halogenated side groups may improve volatility and/or stability of the coordination compound according to the invention. In case the intended application of the coordination compound of the invention is light generation, for example by electroluminescence, the ligand preferably may be chosen such that the excitation of the coordination compound is confined to the central Europium cation. In this way, an intrametallic transition with highest efficiency is obtained. With respect to the choice of the substituents, side groups that are not easily polarized and that have a sufficiently high triplet level above the transition of the first electronically excited state of the central divalent Europium are preferred. Low polarizability is ensured for aliphatic or heteroaliphatic substituents or using small aryl- or heteroaryl systems with less than 8 conjugated atoms or heteroatoms. In case heteroatoms are part of conjugated side groups, preference is generally given to the heteroatoms N and O, and even more preference is given to O. In various embodiments, side groups with a triplet energy > 2.5 eV, or preferably > 2.7 eV are used. Sufficiently high triplet energy of > 2.7 eV is generally observed for organyl groups comprising of aliphatic or heteroaliphatic fragments or for fragments comprising only small aryl- or heteroaryl systems with less than 8 conjugated atoms or heteroatoms, which thus should preferably be employed if a deep blue electroluminescent emitter is the intended use of the coordination compounds according to the invention. Today, triplet energies of organic molecules can easily and sufficiently accurately be calculated using a wide range of open source or commercial density functional theory (DFT) software packages. Alternatively, triplet energies can be experimentally probed for example as described in van Dijken, A., et al. "Carbazole compounds as host materials for triplet emitters in organic light-emitting diodes: polymer hosts for high-efficiency light-emitting diodes." Journal of the American Chemical Society 126.24 (2004): 7718-7727. As a simple rule, suitable high triplet energy building blocks of the macrocyclic organic ligand are preferably either non-aromatic (aliphatic) or comprise aromatic building blocks containing at most 8 aromatic carbon or hetero atoms, whereby various aromatic building blocks are being separated from each other by at least one nonaromatic carbon or heteroatom. In this context, the nitrogen and oxygen present in carbazole and dibenzofuran are considered a nonaromatic heteroatom.

Within the scope of the invention are two or more substituents R that link with each other which are present on neighboring carbon or nitrogen atoms of the macrocycle or on carbon and/or nitrogen atoms of the macrocycle that are separated by one (further) atom of the macrocycle. Two Rs on neighboring macrocycle carbon and/or nitrogen atoms can also be replaced by a double bond, as well as two Rs on a macrocycle carbon atom can form a double bond to a non-macrocycle atom. Preferred are such linkages for Rs situated on neighbouring macrocyclic carbon atoms. Two neighbouring carbon atoms will share a direct carbon-carbon bond. However, two neighbouring macrocyclic carbon atoms may as well be separated from each other by a single macrocyclic heteroatom. In this case, no direct carbon-carbon bond is present, yet those two macrocyclic carbon atoms are still considered neighbours along the macrocyclic ring within this description. There is a wide range of possibilities on how two or more Rs might be linked with each other, many of those are obvious to those skilled in the art. Without limiting the general scope of the invention, just some examples are described. For example, based on G5, in only one instance two Rs being situated on neighbouring carbon atoms that are directly chemically connected may link into a cyclohexane group resulting in a cycle hexyl 18 crown 6 macrocyclic ring. Along the same lines, 4 Rs being situated on two neighbouring carbon atoms might fuse into a benzene ring, resulting in a benzo-18 crown 6 macrocyclic ring, which is described by the generic structure G5. Such linkage can happen several times, for example, if 8 Rs on two separate ethyl linkers are fused into two benzene rings, one might arrive at dibenzo-18 crown 6, which is again based on the generic structure G5. In any case, additional non-fused substitutions R, for example a methyl group, may be present. As said, also substituents on two carbon atoms that are separated by a single heteroatom may fuse. By example of G5, the two substituents on two carbon atoms separated by one of the 6 oxygens may fuse, giving a tetrahydrofuran fragment as part of the macrocyclic ring. Similarly, 4 substituents on those two carbon atoms may fuse into a furanyl group which is shown in M22 in Fig. 1 as part of a dibenzofuran moiety. In any case, those fused organyl groups may be further substituted. As an example, without limiting the general scope of the invention, a furanyl group may be further substituted with a non-macrocyclic benzene ring thus forming 2-benzofurane, being incorporated at the 1,3 positions into the macrocyclic ring. Similarly, the furanyl might be doubly substituted into a dibenzofuran fragment, being incorporated into the macrocyclic system at the 4,6 positions, such that 5 macrocyclic atoms are part of the dibenzofuran fragment, see M22 in Fig. 1. The substitutions on the nitrogen are labeled Rn in G1 to G9. Within the scope the invention, an instance of Rn may be fused with an instance of another substitution R from a carbon atom. However, outside the scope of the invention is a linkage of two instances of R with each other that are not covalently linked to two neighboring macrocycle atoms (thus in a 1,2-substitution scheme) or to two macrocycle atoms separated by one further macrocycle atom (thus in a 1,3-substitution scheme). Such linkages would often form highly three-dimensional cages, typical of the cryptand-type macrocyclic ligands, compare WO 2021/244801. Those are often not able to accommodate the closo-carborate or nido-borate anions in such a way that results in a low total dipole moment, and, as such, are not well-suited for the design of high volatile emitter molecules suitable for gas-transfer processing.

Without limiting the scope of the invention, M1 to M48 shown in **FIG. 1** further illustrate examples of macrocyclic ligands according to the invention. The concrete compounds depicted serve for illustration purposes and shall help to show the scope of the invention. In this context, most of the possible substituents that may be present on certain carbon or heteroatoms have been omitted for clarity. They may nevertheless be present. Similarly, the compounds M41 to M48 shown use specific anions selected from (3-4) to (3-8) above, but it is understood that a certain substituent group R_{b} that is exemplified might be exchanged for another one that is in the scope of the invention and in detail described above. For example, an anion exemplified with chlorine in various instances of R_{b} might equally be considered to have bromine, deuterium or a methyl group instead.

The majority of examples depicted in **FIG. 1** exemplifies macrocyclic organic ligands with 18 macrocyclic carbon and heteroatoms. Exceptions are compounds M9, M13, M14 and M15 with 19 macrocyclic atoms and M10, M11, M38, M41, and M42 with 20 macrocyclic atoms. 6 Heteroatoms are present in the majority of examples depicted in **FIG. 1**. Exceptions are examples M11 and M41 exemplifying 5 heteroatoms as well as M38 having 8 macrocyclic heteroatoms. With the exception of M38, the macrocyclic heteroatoms are separated from each other by two or three carbon atoms. The heteroatoms are mostly selected from N, O, and S. Only M34, M35, M36, and M37 are based on a 18crown6 motive with two macrocyclic oxygens being replaced by P, Si, B, or Se respectively.

As explained above, any suitable macrocyclic carbon or heteroatom may be substituted using a suitable substituent. Here M1 to M11 are depicted without any substituents. M13 and M14 illustrate the situation where 6 macrocyclic carbon atoms are each twice substituted by a methyl group. M25 to M29 depict various situations where two macrocyclic nitrogen's present in the organic ligand are substituted with linear (M25, M29) or branched (M27) alkyl groups, benzyl (M26), or adamantyl (M28). In M30, the hydrogens present on the two nitrogen's are substituted by deuterium. M31, M32, M33, and M38 illustrate the case where just a single instance of R is substituted. For M31, and M32 one nitrogen is substituted with a linear chain attached to a cyclic aza crown ether group, i.e., to another macrocyclic organic ligand. M38 depicts the situation of a single substituent on a carbon that breaks the original high symmetry of the macrocyclic ligand, which may be beneficial to achieve high glass transition temperatures or good solubility. Finally, M33 shows the attachment of the metal-organic coordination compound according to the invention to a polymer by means of a linear alkane chain being substituted on a macrocyclic nitrogen, with n indicating the number of repeat units. The depicted polymer, a hole transporting high triplet energy poly vinyl carbazole, serves as an example only, many more situations are easily envisioned for those skilled in the art. In particular, not every repeat unit of the polymer needs to carry an instance of the coordination compound of the invention. Similarly, the polymer may be a random, or block copolymer, or it may have suitable additional side groups that allow to crosslink the polymer in the presence of the emitter, resulting in a high-glass transition temperature for the polymeric network.

For applications requiring high chemical stability, it may be beneficial to substitute hydrogen partly or completely by deuterium. For example, all possible hydrogens present in the macrocyclic ligand are replaced by deuterium in M23. On the other hand, in example M30 only the two most chemical reactive hydrogens present on the macrocyclic nitrogen atoms, are substituted by deuterium.

As explained above, two or more substituents present on the macrocyclic organic atoms may be linked to each other, and various examples for this situation are given in Fig. 1. For example, in M16 and M17 and M39, two instances of R present on neighboring macrocyclic carbon atoms with a direct bond are linked into cyclohexane fragments, twice in case of M16 and M39, and three times in case of M17. M21 depicts the situation where two instances of R present on the same macrocyclic carbon atom fuse into the cyclohexane groups. Thus, the macrocyclic carbon atom is spiro-linked to its two substituents. In the case of M15, two times two instances of R present on neighboring macrocyclic carbon atoms with a direct bond are linked into bis-cyclic aliphatic groups; overall this substitution pattern appears 3 times in M15, thus a total of 12 hydrogens are substituted. Similarly, in M18 and M19 two (M18) or three (M19) times four instances of R fuse into a benzene fragment, with two macrocyclic carbon atoms being now aromatic. In M12, M20, and M24, four substituents on two neighboring macrocyclic carbon atoms without a direct bond, i.e. separated by a heteroatom, fuse into a heteroaryl fragment. Here furanyl is formed in case of M12, and M20 with oxygen and the two substituted carbon being macrocyclic ring atoms, while in case of M24 pyridine is formed, and nitrogen and the two substituted carbon atoms are part of the macrocyclic ring. In case of M22, benzene and furan forming substituents are combined thus forming di benzofuran with 5 atoms of the di benzo furan being macrocyclic ring atoms. In this particular example, 16 substituents are not equal to hydrogen.

Finally, a substitution may as well be used to attach the anionic groups according to the generic formulas (3-4) and (3-8) to the macrocyclic ring, which is exemplified in M41 to M48.The borate cluster anion is linked to the remainder of the macrocyclic ligand using an ethylene (M41, M42, M43) or propylene (M44, M45, M46, M48) or ethylene-ether (M47) spacer linked to two heteroatoms of the diaza macrocycle, in particular being a substitution on nitrogen.

"The macrocycle" refers to only the sequence of atoms forming the ring core around the Eu(II). "The macrocyclic organic ligand" includes the macrocycle and adds hydrogen or substituents to the remaining free valencies of the macrocycle atoms.

The macrocyclic organic ligands M1 to M40 depicted in Fig. 1 need to be combined with two, preferably identical, anions selected from formulae (3-1) to (3-3) to arrive at various metal organic coordination compounds according to the invention.. In M41 to M48, anionic groups selected from the generic formulae (3-4) to (3-8) are covalently attached.

Exemplary anions that might be present in metal coordination compounds according to the invention are shown as A1 to A21 in **Fig.** 2. The anions may be non-substituted, as exemplified for A1 to A3 and A14 to A17 and A21. Yet, substitutions at positions R_{b} of formulae (3-1) to (3-8) are readily within the scope of the invention and easily chemically accessible. For example, A20 demonstrates a fully deuterated anion, i.e., where all hydrogens have been replaced by deuterium. A13 and A18 show fully methylated versions of the anions, which can be covalently attached (A18) or separate (A13). Similarly, fully halogenated anions are possible, see for example A6, while A5 and A9 illustrate the situation of a half-halogenated anions, each featuring 6 bromines or chlorines per anion with another 6 hydrogens. Beyond doubt, mixed anion substitution patterns are within the scope of the invention and easily chemically accessible. Examples A8, A10, and A11 illustrate the situation where one R_{b} is substituted by halogen, while the remaining 11 hydrogens are all substituted by methyl groups, similar in A7 one position R_{b} is halogenated and some but not all other positions R_{b} are methylated. One or more positions R_{b} may as well be used to covalently link the anion to a polymer. Similar One or more positions R_{b} may be substituted by a charge transport molecule, as one bearing a carbazole or triazine group capable to transport holes or electrons, respectively.

Finally, A21 depicts a situation where two anions based on the generic formula (3-1) are linked with each other, which is both chemically feasible and indeed forms very stable coordination compounds. As such, the situation wherein two identical anions linked with each other via a linear C₁₋₁₅⁻ alkylene chain, preferably linear C₁₋₁₃⁻ alkylene chain, more preferably C₇₋₁₁⁻ alkylene chain, but not to the macrocyclic organic ligand, is in the scope of the invention.

Generally, all anions based on the generic formulas (3-1) to (3-8) are comparably bulky. As such, they will substantially withstand drift if placed in a strong electrical field, such as present in an electroluminescent device configuration. Therefore, anion drift, such as observed in so-called light emitting chemical cells, is elegantly circumvented. In electroluminescent applications, the emitter according to the invention will depict desirable, substantially stable, and time-independent current voltage characteristics.

Part of these anions are known per se and described in WO 2021/244801 and WO 2022/218562 in combination with cryptand-type polycyclic ligands. Their synthesis and derivatives are described for example in Chemical Reviews, 113(10), August 2013 by C. Douvris and J. Michl. "Chemistry of the Carba-closo-dodecaborate anion CB11M12-". It is stated that "Polyhedral boranes and carborane clusters are characterized by delocalized electron deficient bonding. [...] Suitably substituted, anions of this class are among the least nucleophilic and the most weakly coordinating of all known anions. Most are very stable even to the most aggressive reagents. They also can be extremely difficult to oxidize and highly lipophilic. This combination of properties makes them useful in applications as diverse as counterions for reactive cationic organometallic catalysts, electrolytes for nonaqueous solvents and electrical batteries, solubilizers of cations in organic solvents for solvent extraction of radionuclides, and catalysis of radical polymerization of alkenes, to name just a few examples. They have been proposed for use in materials science, medicine, and nanoscience".

The broad class of anions (3-1) to (3-8) is derived from three-dimensional polyhedral boron hydrides in which (except for (3-3) and (3-8)) at one vertex, a boron atom has been replaced with a carbon atom.

In various embodiments of the invention, the coordination compound according to the invention is mixed with a second charge-neutral (electrically neutral) organic compound. The second organic material may be chosen from a wide range of generic classes of substantially organic materials, including, but not limited to, small organic molecules, preferably with a molecular weight less than 1500 g/mol, large molecules or polymers with organic weight of more than 1500 g/mol, dendrimers, metal-organic complexes, or organic materials not comprising any metals. Generally, the second organic material may also be processed in a low molecular weight form and later be combined into a higher molecular weight form. Examples here are metal-organic frameworks, or crosslinkable polymers. Generally preferred are second organic materials that add a desired functionality to the mixture comprising the metal-organic coordination compound according to the invention. Such a functionality can be, without limitation, improved air or moisture stability, improved thermal stability, improved charge transport ability, improved dispersion of the coordination compound according to the invention, improved optical characteristics - such as lowering the refractive index of the mixture, improved processing, to name just a few.

In various embodiments, the coordination compound is imbedded into the at least one second charge-neutral organic compound at 0.1 - 99.0 vol%, preferably at 1 - 90 vol%, and most preferably at 3 - 30 vol%, based on the mixture which is 100 vol%. Thus, in various embodiments, the coordination compound might take a majority or a minority of the overall volume of the mixture. Yet, in a more preferred embodiment, the coordination compound takes a minority part of the volume and has the function of an organic dopant, whereas the one or more second organic materials take a majority part of the volume, for example to funnel energy or charges towards the coordination compound.

In various embodiments, the second organic material being part of the mixture according to various embodiments may be a matrix having suitable optical properties, such as, for example, a high transparency in certain desired parts of the visible spectrum. If this matrix is brought in optical contact to a light source emitting at a sufficiently short wavelength, this light may be absorbed by the coordination compound and re-emitted at a substantially longer wavelength. A suitable light source may for example be a light emitting diode, emitting light at a wavelength substantially shorter than 430 nm, which may be re-emitted by the coordination compound according to the invention at a wavelength longer than 430 nm. The matrix may have any physical dimensions. It may for example be a thin layer of 10 nm to 10,000 nm. The matrix may as well be of granular form or in form of small particles of average diameter between 10 nm and 100,000 nm. For use in optical devices, in the latter cases, the matrix may be applied inside another host material for support.

In various embodiments, the second organic material (b) may serve as charge transporting host to enable electrical excitation of the coordination compound (a) present in the mixture according to the invention. To achieve this desired functionality without severely compromising the triplet energy level, the second charge-neutral compound in preferred embodiments comprises (hetero)arene fragments derived from benzene, pyridine, pyrrole, furan, carbazole, dibenzofuran, triazine, triazole, or benzofuran by removing one or two hydrogen atoms.

In various alternative embodiments the second charge-neutral compound (b) has a molecular weight Mₙ above 1000 g/mol, and the coordination compound (a) and the second charge-neutral compound (b) are chemically linked with each other, for example by a covalent or coordinative chemical bond. Here the mixture is a polymeric compound, which might have benefits in terms of very high glass transition temperatures, or it may ease the processing of the mixtures using wet solution techniques.

The combination of unique emission properties with the ease of processing makes the coordination compounds according to the invention, i.e, based on divalent Europium in combination with closo-carborate or *nido* type cluster anions according to formula (3-1) to (3-8), highly attractive for application in organic electronic devices. In this description, an organic electronic device may be any device or structure including substantially organic layers or subunits, where an electro-magnetic stimulus may be converted into an electrical field or current or vice versa, or where an input electrical current or electrical field may be used to modulate an output electrical current or electrical field.

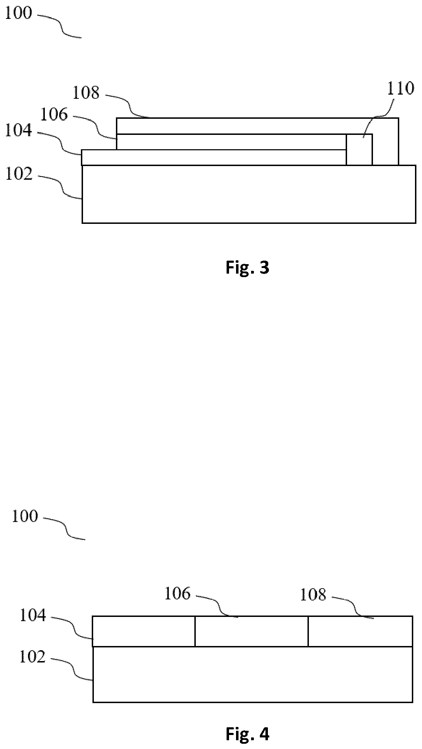

**FIG. 3** and **FIG. 4** illustrate embodiments of an organic electronic device 100 configured as optically active device. The organic electronic device may include a first electrode 104, e.g. on a substrate 102 or as the substrate; a second electrode 108; and an organic layer 106 arranged such that it is electrically interposed between the first and second electrodes 104, 108. The first and second electrodes 104, 108 may be electrically insulated from each other by an insulating structure 110, e.g. a resin or polyimide. The first and second electrodes 104, 108 may be stacked over each other (FIG. 3) or may be arranged in a common plane (FIG. 4).

The organic layer 106 may include the coordination compound according to any of the described or illustrated embodiments, e.g., the layer may contain just the coordination compound, or a mixture with a further organic or non-organic material.

In various embodiments, the organic electronic device 100 may be an optoelectronic device, the optoelectronic device being at least one of an organic light emitting diode (OLED), a light emitting cell (LEC), an organic photodetector, or a photovoltaic cell. That is, photons from an external electromagnetic field may be absorbed in the organic layer 106 and converted into current by means of an electrical field between the first and second electrodes 104 and 108. Such a device would be a photodiode (OPD), and its primarily use may be to sense external light. It would be an organic photovoltaic (OPV) device if the primarily use is to convert light into current.

The organic layer 106 is arranged electrically between the first and second electrodes 104 and 108, such that an electronic current may flow from the first electrode 104 through the organic layer 106 to the second electrode 108 and vice versa during operation, e.g., in light emission applications. Alternatively, in photoelectric applications, a charge carrier pair may be formed in the organic layer 106 and charge carriers of the charge carrier pair may be transported to the first and second electrodes 104 and 108, respectively. In other words, in light emission applications, upon application of sufficient voltage, holes and electrons are injected from the anode and the cathode, respectively. From there, they drift towards the organic layer 106, where charges of opposite sign recombine to form a short-lived localized excited state. The short-lived excited state may relax to the ground state thereby giving rise to light emission.
The first and second electrodes 104, 108 may be substantially unstructured layers, e.g., for general lighting applications, or may be structured, e.g., for light emitting diodes or transistors for pixels in a display application.

The organic electronic device 100 may be configured to emit substantially monochromatic light, such as red, green, blue, or polychromatic light such as white. The light may be emitted through the first electrode 104 (bottom emitter), through the second electrode 108 (top emitter), or through first and second electrodes 104 and 108 (bidirectional emitter). The light may as well be emitted substantially in a direction parallel to the organic layer 106 using suitable opaque electrodes 104 and 108. In such a layout, lasing may be achieved, and the device may be an organic laser, which, in this description, may be considered as a specific type of electroluminescent devices.
The coordination compounds according to various embodiments may have excellent emission properties, including a narrow, deep blue emission spectrum with short excited state lifetime. Given their high atomic weight and open shell septet multiplicities, the optical transition of Eu2+ may be as well widely indifferent to excitation with either spin 1 or spin 0 electron-hole pairs. In other words, singlet and triplet excitations are harvested, resulting in high light emission efficiency. As such, the coordination compounds according to various embodiments may be ideally suited for application in organic electroluminescent devices, such as organic light emitting diodes (OLED). In this description, an electroluminescent device may be any device including an organic layer disposed between and electrically connected to an anode 104/108 and a cathode 108/104. Upon application of sufficient voltage, holes and electrons may be injected from the anode 104/108 and the cathode 108/104, respectively, and drift towards the organic layer 106, where charges of opposite sign recombine to form a short-lived localized excited state. The short-lived excited state may relax to the ground state thereby giving rise to light emission. Relaxation pathways without light emission, such as thermal relaxation, may be possible too, but may be considered undesirable, as they lower the conversion efficiency of current into light of the device. Organic light emitting cells (LECs) may be considered as a subclass of OLED devices comprising of mobile charged species inside the active organic layer 106, which are able to drift inside an external applied electrical field. As such OLEDs encompass LEC type devices.

Further layers may be formed and in electrical connection between the first and second electrodes 104, 108, e.g., configured for charge carrier (electron or hole) injection, configured for charge carrier transport, configured for charge carrier blockage or configured for charge generation. Further optically functional layers, e.g., a further electroluminescent material and/or a wavelength conversion material may be formed electrically between the first and second electrodes 104, 108 and in the optical path of the organic layer 106, e.g., on top of the second electrode 108 and/or on the opposite side of the substrate 102. In addition, encapsulation structures may be formed encapsulating the electrically active area, e.g., the area in which electrical current flows and may be configured to reduce or avoid intrusion of oxygen and/or water into the electrically active area. Further optically functional layers, e.g., an antireflection coating, a waveguide structure and/or an optical decoupling layer may be formed within the optical light path of the organic layer 106.

As example, hole or electron blocking layers may be used to optimize the individual hole and electron currents through the organic electronic device 100. This may be known to those skilled in the art as charge balance in order to optimize efficiency and operational stability. In various embodiments, dedicated hole or electron charge transport layers may be present in the organic electronic device 100 to space the emission region from the first and second electrodes 104, 108.

Examples of hole transport materials include known materials such as fluorene and derivatives thereof, aromatic amine and derivatives thereof, carbazole derivatives, dibenzofuran, dibenzothiophene, and polyparaphenylene derivatives. Examples of electron transport materials include oxadiazole derivatives, triazine derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and its derivative, diphenoquinone derivatives, and metal complexes of 8-hydroxyquinoline and its derivatives and various derivatives of silanes.

In various embodiments, those charge transport layers may include electrical dopant molecules or metals or may be in contact to charge injection layers.
Any of those auxiliary layers may be fully organic or may include inorganic functional moieties. For example, charge transport layers may be made of the class of Perovskite materials.

The coordination compound according to the invention as illustrated or described in any one of the embodiments may be used as a thin substantially organic emission layer 106 of any thickness in the range of 0.1 to 100 nm, preferably in the range of 3 to 20 nm. The concentration of the coordination compound throughout the cross section of the organic layer may be constant or varied. Typical variable concentrations are in the range of 0 to 100 vol%, i.e., the layer may be partially containing either no coordination compound or just coordination compound. Such situations are indeed frequently observed if mobile evaporation sources are employed to co-sublime the organic material with the coordination compound. The emission layer 106 may as well be configured as a plurality of mixtures according to the invention having different concentrations of coordination compound. The emission layer 106 may as well be configured such that the metal-organic coordination compound is present in two adjacent layers, with a different second organic material being present in both layers. It may for example be a substantially hole transporting material in layer 112, yet a substantially electron transporting organic material in layer 113. Alternatively, the concentration of the coordination compound according to the invention imbedded into a second material may be substantially different thereby again forming two adjacent layers 112 and 113, with preferably different charge transport qualities.

A second organic material being present in the layer containing the coordination compound according to the invention such as the organic layer 106 may in various embodiments be a charge transport material to improve charge transport into and through the organic layer 106 or to facilitate charge recombination. Charge transport materials may be any material that are able to transport either holes or electrons or both types of charges upon applying a suitable electrical field. In particular a charge transport material may be any aryl, or heteroaryl organic compound or any metal complex or any mixture thereof. Without limiting the scope of the invention, examples of materials present in the light emitting layer 106 include carbazole derivatives, including carbazole-phosphine oxide materials, oxadiazole derivatives, triazine derivatives, silane derivatives and any other material or combination of materials with sufficiently high triplet level that may transport charges or otherwise benefit the device performance.

The organic layer 106 that contains the coordination compound according to the invention may contain any further organic or inorganic material in a range of 0.1 to 99.0 vol% that is not intended to transport charges. For example, the organic layer 106 may include polymers (in a mixture or as a compound) which may be added to improve film quality and prevent crystallization. Other materials may be added to evenly space the coordination compound inside the organic layer 106.

In various embodiments, the organic electronic device 100 includes two or more subunits each including at least one light emitting layer. The subunits may be stacked over each other physically separated and electrically connected by a charge generation layer or, alternatively, may be arranged side by side. The subunits may be subpixels of a pixel in a display or general lighting application. The light emitted by the subunits may be mixed to generate a light of a predetermined color. Each subunit may emit light of the same or a different color. The overall light emitted by such organic electronic device 100 may contain a narrow spectral region, such as blue, or may contain a wide spectral region such as white, or a combination thereof. The coordination compound according to the invention comprising the divalent Europium illustrated or described in any one of the embodiments may or may not be present in each individual subunit of the organic electronic device 100.

In various embodiments, the light emitted by the organic electronic device 100 may be in optical contact to at least one optically active layer, including any optically active materials such as organic molecules or quantum dots. The optically active layer may be a spectral filter element, which may absorb part of the light emitted by the organic electronic device 100. In another embodiment, the optically active layer may absorb at least part of the light emitted by the organic electronic device 100 and may reemit it at longer wavelength (wavelength conversion), e.g., by quantum dots. As an example, the organic layer 106 may be configured to emit light substantially at wavelengths shorter than 500 nm and the optically active layer may be configured to substantially reemit light at wavelengths longer than 500 nm. The optically active layer may be placed in between the anode and cathode of the organic electronic device 100 or outside of it. The optically active layer may as well be part of the organic layer 106.

The term "substantially" may refer to (relative) amounts of a compound and typically means at least 80 wt% preferably at least 90 wt%, more preferably at least 95 wt%, specifically 100 wt%, not considering impurities or additives. It may define ranges of 80 to 100 wt% etc. for the content or purity of a compound. This definition refers e.g. to amounts like weight or volume or other parameters like wavelength. For single values, "substantially" allows for a variation or deviation from a given number by 20% or less, preferably 10% or less, more preferably 5% or less, specifically 1% or less. An emission substantially in the deep blue spectral region below 500 nm means that at least 80% of the emission are below 50 nm etc., e.g. of a measured integral of the emission intensity over wavelength. A substantially organic substrate contains at least 80% of organic substances. When not indicated otherwise, % means wt%.

The organic electronic device 100 may be configured as a large area OLED device used for illumination, signage, or as a backlight. Alternatively, the organic electronic device 100 may include a plurality of OLEDs arranged in a pixelated layout (plurality of OLED pixels) that are individually connected electrically, e.g., for flat panel display applications. Here, individual pixels may have the capability of emitting light of substantially narrow spectral portions; especially of red, green, and blue. The mixture may or may not be present in any of the individual pixels.

In another embodiment, the individual pixels may be configured to emit white light. Red, green, and blue spectral portions are generated by using suitable filter elements in optical contact with the respective pixelated OLEDs.

In another embodiment, the OLED pixels emit blue light, and the red and green spectral portions may be generated by using a suitable color conversion element in optical contact with the OLED pixels.

In another embodiment, the OLED pixels emit substantially in the near-UV spectral region with wavelength < 450 nm and the red and green and blue spectral portions may be generated by using a suitable color conversion element in optical contact with the OLED pixels.

An organic electronic device 100 according to various embodiments may be fabricated using a wide range of commonly used techniques, including, but not limited to, deposition of all or some layers, from gas phase vacuum deposition, solution phase, or gas phase using a carrier gas method.

Due to the arrangement of the anions with respect to the cationic metal centuries, metal-organic coordination compounds according to the invention show a relatively low dipole moment allowing for thermal deposition from gas phase with high yields. Thus, in various embodiments, deposition via the gas phase in vacuum may be used, whereby the coordination compound may either undergo sublimation or evaporation and may be co-deposited with a second organic materials thereby forming mixed layers. The coordination compounds according to the invention show an improved sublimation/evaporation yield. The transfer into the gas phase may be further improved by using a carrier gas technology, whereby an inert gas that may not be deposited into the organic layer comprising the coordination compound assist the sublimation or evaporation of the coordination compound. During the deposition process from gas phase, the coordination compound may be co-deposited with a second organic material using two separate thermal sources, in other words, a mixture is formed in-situ during deposition. During such co deposition two or more additional materials may be deposited using a triple or quadruple evaporation, whereby the coordination compound according to the invention is further diluted. Alternatively, the metal-organic complex according to the invention may as well be thermally processed from one crucible. In other words, a mixture or a precursor thereof is already present before sublimation or evaporation. A precursor may for example be an additional auxiliary ligand present as part of the coordination compound that dissociates during thermal evaporation and that is substantially not built into the organic layer comprising the coordination compound according to the invention. Preferred in this situation are similar volatilities for the second organic material and the coordination compound. Alternatively, the coordination compound and the second organic material may form a frozen glass, thereby forcing the individual components to sublime at similar temperatures.

Another preferred technique to fabricate layers including the coordination compound according to various embodiments may be deposition from a liquid phase using a mixture of or a single organic solvent, whereby the coordination compound according to various embodiments may be dissolved or forms a suspension within the organic solvent; in this description may be referred to as the ink. The ink using this deposition process may include a wide variety of other materials apart from the coordination compound according to various embodiments to allow fabrication of triple or higher-order mixed layers from solution. Additives within the ink may for example, but may not be limited to, be organic or inorganic materials capable of transporting charges, materials that improve the film formation, materials that improve the distribution of the coordination compound according to the invention within a third material, organic or inorganic materials that improve the efficiency of the device, e.g., by reducing the refractive index. The deposition from solution may not be limited to any specific technique. Examples of the deposition from solution include spin coating, casting, dip coating, gravure coating, bar coating, roll coating, spray coating, screen printing, flexographic printing, offset printing, inkjet printing. Owning to their low dipole moments, the improved solubility of the coordination compounds according to the present invention in non-polar solvents eases the deposition from solution and makes it advantageous. In particular, solvents with relatively low polarity index such as tetrahydrofuran or toluene may be used as part of the ink formulation.

Various post processing techniques may be applied to improve the performance or stability of the organic electronic device. In one embodiment, some or all layers of the organic electronic device include functional groups capable of chemically crosslinking upon thermal or electromagnetic exposure thereby forming larger covalently bound molecules with improved physical properties. In another embodiment functional groups are present in a second organic material and after crosslinking the second organic material becomes a polymer with the coordination compound according to the invention being embedded into. In yet another embodiment functional groups capable of crosslinking are present on both, the coordination compound and a second organic material, and after crosslinking, a single polymeric material covalently linking a second organic material with the metal-organic coordination compound according to the invention is formed.

In various embodiments, the coordination compound may be formed in-situ using deposition from solution. Here, the organic macrocyclic ligand of the present invention, excluding the divalent Europium, may first be deposited onto a suitable substrate or other organic layer thereby forming a seed layer. Any suitable technique may be used to fabricate this seed layer. This seed layer, including the organic ligand, may include any further material. Preferred may be further additional inert organic or inorganic materials that aid the layer formation or improve its thermal stability or improve the distribution of the organic ligand or second organic material within this seed layer. In a next deposition step and sequential to forming the seed layer including the organic macrocyclic ligand excluding the metal, a layer including a molecular salt comprising the divalent Europium may be fabricated using a solution process. The Europium compound may be any charge neutral compound including Eu(II) and one or two suitable anions. The two suitable anions may or may not be selected from the generic anions described by formulae (3-1) to (3-3). The ink including the Europium compound may as well include one or more additives to fabricate mixed layers. These additives may be organic or inorganic materials to aid charge transport, may be organic or inorganic materials that improve the efficiency of the device, or may be any material that improves the film formation. At the interface of the seed layer including the organic ligand according to the invention, the solubilized divalent Europium compound will interact with the macrocyclic organic ligand to form the coordination compound according to various embodiments in-situ. Alternatively, the process of first forming the layer containing the ligand and secondly processing the lanthanide compound may be inverted.

For one or more aspects, at least one of the components set forth in one or more of the preceding figures may be configured to perform one or more operations, techniques, processes, and/or methods as set forth in the example section below.

### EXAMPLES

The examples set forth herein are illustrative and not exhaustive.

Any of the examples may be combined with any other example (or combination of examples), unless explicitly stated otherwise. The foregoing description of one or more implementations provides illustration and description but is not intended to be exhaustive or to limit the scope of aspects to the precise form disclosed. Modifications and variations are possible in light of the above teachings or may be acquired from practice of various aspects. Any of the above-described embodiments may be combined with any (other) example as mentioned below (or combination of examples), unless explicitly stated otherwise.

### I. Details on how to calculate relevant parameters of Europium coordination compounds using quantum-chemical methods

The computational protocol to determine structures, emission energies (**EE**), and the excited-state electron binding energies of Eu(II) complexes in their electronic ground (4*f ⁷*5*d ⁰*) (**EBE**) and first (energetically lowest) excited (4*f*⁶5*d* ¹) (**ES-EBE**) state consists of 3 main steps, all of which can be accomplished with freely available open-source software (xTB: https://github.com/grimme-lab/xtb; CREST https://github.com/crest-lab/crest; CENSO https://github.com/grimme-lab/CENSO) and the commercially available quantum-chemical program package ORCA (https://www.faccts.de/orca/). These three steps are firstly, the generation of a large comprehensive ensemble of all possible structures of given complex in a large energy window with fast semi-empirical methods, secondly a multi-level energetical screening, optimization, and reranking of the structures in the large ensemble with increasingly accurate density-functional theory (DFT) to determine the relevant (energetically lowest) conformer(s), and thirdly the calculation of the desired properties by combining time-dependent (TD)-DFT for the excited states with DFT for the ground states.

In principle, any quantum-chemistry software can be used for these calculations and would provide near-identical results if the methods described in detail below (functionals, dispersion corrections, basis sets, geometric Counter Poise corrections, effective core potentials, solvent models and parameters) are applied in the same way. Due to the statistical (non-deterministic) nature of the simulations in step one of this approach, the results are not exactly reproducible. However, if the simulation times are sufficiently long, the approach will always yield the same relevant (lowest energy) conformer after step 2, which will have very similar properties in the calculations in step 3. As a very conservative estimate, the precision of the predicted dipole moments (over repeated runs for the same molecule) should be ±1 D and ±0.1 eV for emission energies and ionization potentials.

In detail, the three main steps of the computation approach are:
- **Generation of a comprehensive conformer ensemble** with the CREST program version 2.12 using the implemented semi-empirical tight-binding model GFN2-xTB in gas phase, as well as in combination with the Analytical Linearized Poission-Boltzmann (ALPB) solvation model with parameters for toluene as implemented in the xTB software version 6.4.1.
   This conformer generation step comprises at least two iterations with seven parallel metadynamics simulations, each with at least 30 picoseconds (ps) of simulation time (420 ps total simulation time). This step is done twice: Once with the ALPB solvation model with parameters for toluene solution, and once in gas phase, both starting from the input structure optimized at the same level of theory, yielding an overall simulation time of at least 840 ps (more can be necessary to generate a complete ensemble for particularly flexible complexes). The conformer ensembles from these two independent runs are then combined and sceened in the presence of the ALPB solvation model with parameters for hexane to obtain a final ranked ensemble that is passed on to the next step 2).
   Since CREST, by default, excludes all molecules with topological changes in the bonding pattern compared to the starting structure (as these are isomers not conformers), changes on the central Eu-atom have to be explicitly allowed by excluding Eu from the topology check (-notopo Eu). This is required to ensure that structures with different coordination geometries and numbers of ligand-atoms attached to Eu are also considered (e.g. an iodine coordinated to Eu(II) can move away and coordinate to an NH-proton instead). If isolated (not covalently bound) anions are included in the complex, like iodide or closocarborate, the NCI mode of CREST is invoked (-nci -wscal 1.3). All structures within 8 kcal/mol of the lowest structure are kept in the ensemble (and are thus included in step 2).
   Commandline prompt for the run in toluene with the input-structure input.xyz:
      *crest input.xyz -gfn2 -alpb toluene (-nci -wscal 1.3) -mdlen 30 -nmtd 7 -notopo Eu -ewin 8*
      Commandline prompt for the run in gas phase:
      *crest input.xyz -gfn2 (-nci -wscal 1.3) -mdlen 30 -nmtd 7 -notopo Eu -ewin 8*
      Commandline promt for the screening of the combined ensembles:
      *crest -screen combined.xyz -gfn2 -alpb hexane -notopo Eu -ewin 8*
- **Multi-level screening, optimization, and reranking of the conformer ensemble** from 1. in 4 sub-steps with DFT methods of increasing accuracy using the CENSO program as driver and ORCA 5.0.3 to carry out the DFT calculations.
   **(A) Prescreening** based on PBE-D4/def2-SV(P)+gCP, single-point energies are calculted using the structures from step 1 (notation PBE-D4/def2-SV(P)+gCP// GFN2/ALPB(toluene)). Solvent effects are included by adding the GFN2-xTB/ALPB free energy of solvation (CENSO default) and the 28-electron def2-ECP effective core potential is used for Eu and all atoms heavier than krypton (ORCA default). All conformers whose energy is within 7 kcal/mol of the lowest conformer at this level of theory are included in step 2B. *Commandline prompt: censo -inp crest_conformers.xyz -solvent toluene -u 7 -chrg 0 -prog orca -part0 on -func0 pbe-d4 -part0_threshold 7*
   **(B) Screening** based on r2SCAN-3c, single-point energies are calculated for the structures from step 1 using the SMD model with parameters for toluene to include solvent effects (r²SCAN-3c/SMD(toluene)//GFN2-xTB/ALPB(toluene)). The resulting single-point energies are combined with thermostatistical contributions (zero-point vibrational energy, entropic and enthalpic vibrational, translational and rotational contributions) evaluated at the GFN2-xTB/ALPB(toluene) level of theory using the modified rigid-rotor harmonic-oscillator (mRRHO) approximation (CENSO default) to obtain free energies. All conformers with a free energy that is within 4.5 kcal/mol of the lowest conformer at this level of theory are included in step 2C. *Commandline prompt: censo -inp crest_conformers.xyz -solvent toluene -u 7 -chrg 0 -prog orca -part0 on -func0 pbe-d4 -part0_threshold 7 -part1 on -part1_threshold 4.5*
   **(C) Reoptimization** of the structures (from step 1) with r²SCAN-3c using the SMD solvent model with parameters for toluene and the "lax" convergence criteria setting of CENSO. Entropic and enthalpic contributions are included at the GFN2-xTB/ALPB(toluene) theory level using mRRHO approximation combined with the single-point Hessian approach (CENSO default). All conformers with a free energy that is within 1.5 kcal/mol of the lowest conformer at this level of theory are included in step 2D.
      *Commandline prompt: censo -inp crest_conformers.xyz -solvent toluene -u 7 -chrg 0 -prog orca -part0 on -func0 pbe-d4 -part0_threshold 7 -part1 on -part1_threshold 4.5 -part2 on - optlevel2 lax -part2_threshold 1.5*
   **(D) Final Reranking** of the structures from 2C using single-point energies calculated with the ωB97X-V functional using the def2-TZVPP basis set and def2-ECPs and the SMD(toluene) model for solvent effects. Thermostatistical corrections (enthalpy, entropy) are taken from step 2C.
      If there is more than one structure within 1.0 kcal/mol of the lowest structure at this level of theory and the respective structures are similar, then only the lowest structure is taken to the next step (3). If there are two or more structures within 1 kcal/mol with different coordination geometries (different atoms coordinating to Eu, different coordination geometry), they are all included in the next step.
      *Command-line prompt: censo -inp crest_conformers.xyz -solvent toluene -u 7 -chrg 0 -prog orca -part0 on -func0 pbe-d4 -part1 on -part2 on -part3 on -func3 wb97xv -basis3 def2-tzvpp*
- **Calculating of EE, EBE, and ES-EBE** from 2D with the ωB97X-D3 dispersion-corrected density functional (ORCA keyword !wB97X-D3) using the def2-TZVP basis set and def2-ECPs 28-electron pseudopotential for Eu, the def2-SVP basis set (and if necessary def2-ECPs) for all other atoms, the geometrical Counter Poise correction (ORCA: gCP(DFT/SVP)) for basis set incompleteness error, and the SMD solvation model with (slightly modified, see below) parameters for anisole. Convergence thresholds for geometry optimizations in ORCA are set to "loose" (!LOOSEOPT).
   **EE: Emission energies** are calculated by optimizing the lowest electronically excited state of the lowest conformer from 2D with time-dependent (TD-)DFT/ωB97X-D3 in the Tamm-Dancoff approximation (TDA) using the basis sets, corrections as specified in 3. For the excited-state optimization, the electronic part of the SMD solvation model (C-PCM) is replaced with the linear-response polarizable-continuum model (LR-PCM) using equilibrium solvation for the gradient calculation during the optimization, and non-equilibrium solvation for final emission energies (ORCA default). The emission energy corresponds to the energy of the first excited state after the optimization is converged.
   **EBE: Electron binding energies of the neutral coordination compound** are calculated as the difference between the electronic energy of the fully relaxed complex with ground-state Eu(II) and the energy of the fully relaxed complex with Eu(III) (singly positively charged) according to the following equation: EBE = E(Eu2) - E(Eu3). The optimizations use the ωB97X-D3 level of theory as specified in 3.
   **ES-EBE: Electron binding energy of Eu(II) in the lowest electronically excited state** is calculated as the electronic energy difference between the geometry-optimized complex with Eu(II) in its first excited state and the corresponding geometry-optimized complex with Eu(III) (Eu3, singly positively charged) according to the following equation: ES-EBE = E(Eu2*) - E(Eu3). Optimization of the electronically excited Eu(II) complex uses the same TDA-ωB97X-D3 level of theory as specified in 3B. Optimization of the Eu(lll) complex uses the same ωB97X-D3 level of theory specified in 3 (sample inputs S2 and S3).

### Technical Details and Sample Inputs:

The workflow described in step 1. and 2. of this protocol is based on the one presented in the following article with some modifications to the default energy cutoffs and methods:
*Efficient Quantum Chemical Calculation of Structure Ensembles and Free Energies for Nonrigid Molecules*
S. Grimme, F. Bohle, A. Hansen, P. Pracht, S. Spicher, and M. Stahn
J. Phys. Chem. A 2021, 125, 19, 4039-4054 (https://pubs.acs.org/doi/pdf/10.1021/acs.ipca.1c00971) All ORCA calculations for steps 2 and 3 employ the resolution-of-the-identity (RI) and chain-of-spheres-exchange (COSX) approximation with the implemented fitting basis sets (def2/J) and default integration grids.

The following sample jobs (S#) include all keywords relating to the methods described above. However, additional keywords and settings might be required in some or all cases to avoid technical problems (e.g. SCF convergence issues). As these settings depend on the individual case, they are not provided here.
*S1: Ground-State-Optimization of Eu(II) complex (Dipole Moment 3A and Ionization Potential 3C)* 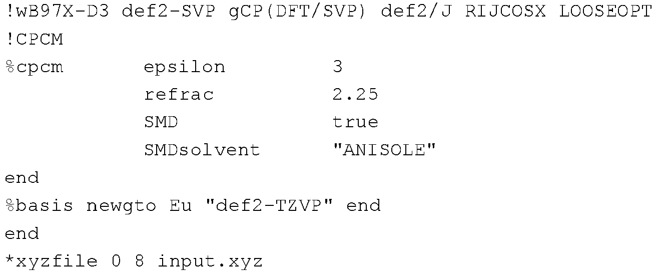
*S2: Excited-State-Optimization of Eu(II) complex (Emission energy 3B and Ionization Potential 3D)* 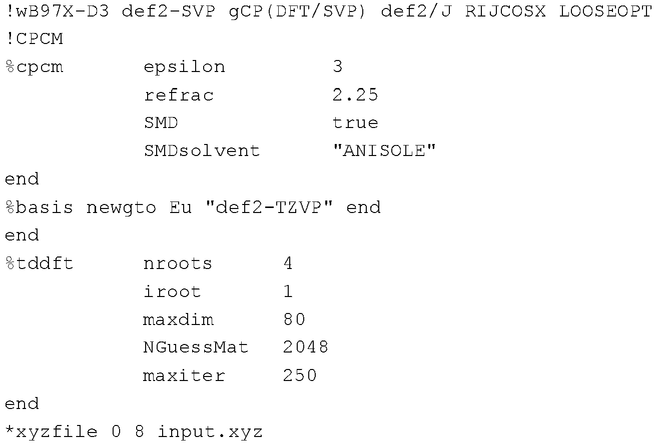
*S3: Ground-State-Optimization of Eu(III) complex (Ionization Potentials 3C and 3D)*

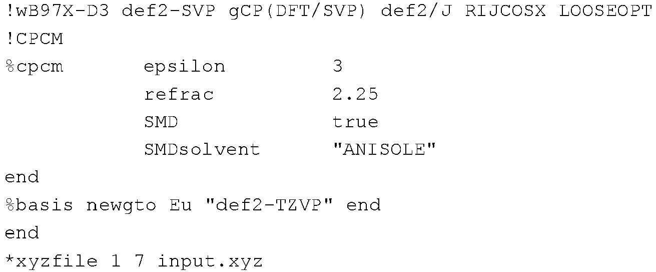

Collection of calculated parameters of metal-organic coordination compounds according to the invention or various comparative examples

**Table 1 Summary of experimental data for various metal organic coordination compounds according to invention with macrocyclic organic ligands (M) according to Fig. 1 and anions (A) according to Fig. 2 as well as one counter example (CE), obtained using quantum mechanical calculations.**

| **Macrocyclic ligand acc**. **to** **Fig. 1** | **Anion acc. to** **Fig. 2** | **Dipole (D)** | **Ems (eV)** | **EA (eV)** |
|---|---|---|---|---|
| M3 | CE1 | 12.3 | 2.70 | 2.88 |
| M1 | A1 | 4.3 | 3.09 | 3.22 |
| M2 | A1 | 2.9 | 2.75 | 3.27 |
| M3 | A1 | 4.1 | 2.83 | 2.96 |
| M4 | A1 | 2.6 | 2.73 | 2.95 |
| M5 | A1 | 3.8 | 2.77 | 2.78 |
| M6 | A1 | 0.7 | 2.76 | 2.77 |
| M7 | A1 | 4.5 | 2.70 | 3.03 |
| M8 | A1 | 4.2 | 2.66 | 2.82 |
| M9 | A1 | 2.9 | 2.73 | 2.73 |
| M10 | A1 | 2.3 | 2.74 | 2.71 |
| M11 | A1 | 2.9 | 2.73 | 2.84 |
| M12 | A1 | 3.0 | 2.97 | 3.10 |
| M13 | A1 | 3.6 | 2.98 | 3.01 |
| M14 | A2 | 4.3 | 2.73 | 2.65 |
| M15 | A1 | 5.1 | 3.02 | 3.37 |
| M16 | A1 | 4.2 | 2.77 | 2.73 |
| M17 | A1 | 2.1 | 2.76 | 2.60 |
| M18 | A1 | 2.7 | 2.76 | 2.86 |
| M19 | A1 | 3.5 | 2.83 | 2.74 |
| M20 | A1 | 1.3 | 2.80 | 2.85 |
| M22 | A1 | 1.5 | 2.94 | 3.22 |
| M25 | A1 | 4.0 | 2.91 | 3.05 |
| M26 | A1 | 6.9 | 2.89 | 3.03 |
| M34 | A1 | 3.5 | 2.28 | 3.27 |
| M38 | A1 | 3.4 | 2.52 | 2.34 |
| M43 | - | 3.2 | 2.72 | 3.30 |
| M44 | - | 6.5 | 2.84 | 3.37 |
| M41 | - | 3.7 | 2.75 | 3.31 |
| M42 | - | 2.2 | 2.80 | 3.28 |
| M3 | A12 | 4.3 | 2.68 | 2.89 |
| M3 | A13 | 1.8 | 2.96 | 3.29 |
| M3 | A5 | 4.2 | 2.86 | 3.21 |
| M3 | A9 | 4.1 | 2.59 | 3.12 |
| M3 | A6 | 2.8 | 2.82 | 3.41 |
| M3 | A2 | 2.0 | 2.60 | 2.70 |
| M3 | A8 | 1.5 | 2.69 | 2.75 |
| M3 | A10 | 2.3 | 2.62 | 2.79 |
| M3 | A11 | 4.0 | 2.72 | 2.98 |
| M39 | A2 | 2.1 | 2.78 | 2.74 |
| M40 | A1 | 0.9 | 2.93 | 3.11 |
| M37 | A1 | 4.6 | 2.74 | 3.26 |

A wide variety of macrocyclic organic ligands and anions from Fig. 1 and 2, respectively are combined into luminescent metal organic coordination compounds according to the invention and their material properties has been thoroughly analyzed using the quantum mechanical protocols as described above. CE-1 denotes a counter example where tetraphenyl borate has been used as anion instead of an anion according to the invention, i.e. according to the generic formulae (3-1) to (3-8). The macrocyclic ligand of CE-1 is the same di aza 18crown6 one as exemplified in M3 in Fig. 1. Further details on synthesis and chemical structure of CE-1 can be found below.

The first column in table 1 quotes the calculated dipole moment of all compounds in Debye. A low dipole moment indicates high volatility and good solubility in non-polar solvents. Indeed, all metal-organic coordination compounds according to the invention feature a low dipole moment of less than 7 Debye, while most of them are even below 3.5 D. Contrary, the counter example CE-1 shows the typical dipole moment of above 10 D that is expected for macrocyclic organic salts.

The next column shows the calculated emission energy, Ems in electron volts. Here, with very few exceptions, the vast majority of examples emits in the desirable deep blue spectral region. Notable exceptions are M34+A1, and M38+A1, where the emission shifted into the green or even red spectral region. In M34+A1, this is due to the presence of the less preferred P donor atoms. In case of M38+A1, the reason for the red-shift lies in the less preferred arrangement with two 4*2 neighboring macrocyclic heteroatoms.

The final column shows the electron-binding energy of the complex in its lowest electronically excited state given in electron volts, also called excited-state electron-binding energy (ES-EBE), which roughly corresponds to the electron affinity (EA) value of typical organic emitter molecules. As such, this value describes how strong the electronically excited emitter binds its electrons, which is a critical parameter for charge-carrier confinement as well as an indication of chemical stability in ambient conditions. A high value above 2.5 eV is critical for OLED function as this simplifies the desired confinement of the excitation energy and charge carriers and allows for a much wider selection of host materials. Indeed, most values are very high keeping in mind that divalent Europium compounds, especially those emitters already known the literature, usually exhibit very poor electron binding energies in the range of 2.0-2.2 eV. Here, nearly all examples using closocarborate anions or anionic groups exhibit values well above 2.5 eV. Only in M38+A1 the value is below this threshold with 2.38 eV, which can be traced back to the less preferred arrangement of with two neighboring macrocyclic donor-atoms.

Moreover, a high ES-EBE also corresponds to good oxidation stability via its connection to the ground-state electron-binding energy (EBE) according to the formular: EBE ≈ ES-EBE+Ems. Here, emitters with an EBE of > 5.5 eV are typically sufficiently stable to allow for handling the metal-organic coordination compounds in ambient conditions, i.e., context with oxygen will not cause an irreversible oxidation of the compounds into Eu(lll) derivatives.

### Variation of the Anion for macrocylic organic ligand M3

**Table 2: The effect of exchanging the anion in Emitter M3 on the calculated dipole moment (in Debye), emission energy (Ems in eV), and the excited-state electron-binding energy (ES-EBE in eV).**

| **Anion acc. to** **Fig. 2** | **Dipole** | **Ems** | **ES-EBE** |
|---|---|---|---|
| CE3 | 0.0 | 2.76 | 1.55 |
| CE2 | 2.1 | 2.68 | 1.87 |
| CE1 | 12.3 | 2.73 | 2.79 |
| A1 | 4.1 | 2.83 | 2.96 |
| A2 | 2.0 | 2.60 | 2.70 |
| A3 | 3.5 | 2.68 | 2.69 |
| A4 | 4.2 | 2.88 | 2.60 |
| A5 | 4.2 | 2.86 | 3.21 |
| A6 | 2.8 | 2.82 | 3.41 |
| A7 | 1.7 | 2.70 | 2.89 |
| A8 | 1.5 | 2.69 | 2.75 |
| A9 | 4.1 | 2.59 | 3.12 |
| A10 | 2.3 | 2.62 | 2.79 |
| A11 | 4.0 | 2.72 | 2.98 |
| A12 | 4.3 | 2.68 | 2.89 |
| A13 | 1.8 | 2.96 | 3.29 |

**Table 2** illustrates how key properties of emitters based on the macrocyclic organic ligand M3 change when different anions are used, whereby A1 to A13 as well as CE1 to CE3 refer to the anions depicted in Fig. 2. Beginning with the comparative examples, i.e. with anions not according to the invention. Using those it is immediately clear that none of them fulfil all three requirements to function in OLED device at the same time. CE3 (bromide) and CE2 (BF4-) exhibit the desirable low dipole and deep-blue emission, but, due to their strong interaction with Eu(II), lead to ES-EBE values far higher than the desired 2.5 eV. Thus, these are clearly not suitable for application in OLED due to issues with charge-carrier confinement and low ambient stability. In contrast, CE1 (BPh4-) shows good emission color and ES-EBE, however, due to its unfavorable asymmetric structure, it attains by far the largest dipole moment of 12.3 D. As a result, the emitter using comparative anion CE1 is highly polar, not volatile, and thus not suitable for gas-transfer processing, as confirmed by the sublimation experiments.

Finally, all closo-carborate and nido-borate anions depicted as A1-A13 fulfil all three requirements: They all have low dipole moments below 4.5 D, their emission color mostly lies in the deep blue region, and their ES-EBE values are all higher than 2.5 eV. The variation of the ES-EBE (over 1.01 eV between A4 and A6) and emission color (over 0.37 eV between A9 and A13) presents another desirable feature of the complexes according to invention: By minor modification of the anion for a given macrocyclic ligand, e.g., through halogenation from A4 (1 Cl) over A5 (6 CI) to A6 (12 CI), these critical parameters can be fine-tuned to the intended purpose, e.g., in an OLED application to match the energy level of the host material for maximum efficiency.

In conclusion, the combination of the anions according to the invention with the macrocyclic organic ligands yields highly robust metal organic complexes with divalent Europium that are deep blue and can be processed using gas transfer processes. The combination of deep blue color, high ambient stability, and possibility to sublime has indeed not been described in any prior art and as such describes a highly beneficial combination of properties, suitable to make deep blue and efficient OLED devices.

### II. Syntheses of various divalent Europium coordination compounds

All experimental manipulations were carried out under an inert atmosphere. Tetrahydrofuran (Acros Organics, extra dry, AcroSeal) was dried and distilled from K/benzophenone or purchased as anhydrous from Acros Organics. Toluene (Acros Organics, Extra Dry, AcroSeal) purchased as anhydrous from Acros Organics, Methanol (Acros Organics, Extra Dry, AcroSeal) purchased as anhydrous from Acros Organics. Trimethylammonium carbadodecaborate (Katchem, >97%) were used as purchased. Europium(II) iodide (Aldrich, AnhydroBeads, 10 mesh, 99.999% trace metals basis) was used as purchased. 1,4,10,13-Tetraoxa-7,16-diazacyclooctadecan (Aldrich, ≥96%) and 1,4,7,14,17,20-hexaoxa[7.7]orthocyclophan (Aldrich, 98%) were sublimated before the use. 6,9,12-trioxa-3,15-diaza-1(2,5)-furanacyclohexadecaphane was prepared according to a published procedure (dx.doi.org/10.1021/jo501966q | J. Org. Chem. 2014, 79, 10334-10341).

### Syntheses of bis[bis(trimethylsilyl)amide]bis(tetrahydrofuran) europium(II) (Eu(HMDS)2)

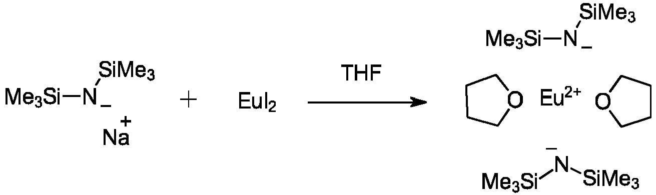

Sodium bis(trimethylsilyl)amide (2 eq, 9.5 g, 49.2 mmol) was added under a nitrogen atmosphere to the solution of europium diiodide (1 eq, 10 g, 24.6 mmol) in 300 ml of anhydrous THF. The mixture was stirred at RT overnight. THF was removed in vacuum and residue was extracted with hexane (250 ml). The extract was filtered and concentrated to ca. 50 ml volume. Cooling of the solution to 0 °C resulted in precipitation of the title product as orange-yellow crystals. Yield: 90% (13.77g)

### Synthesis of trimethylammonium 12-chloro-1-carbadodecaborate

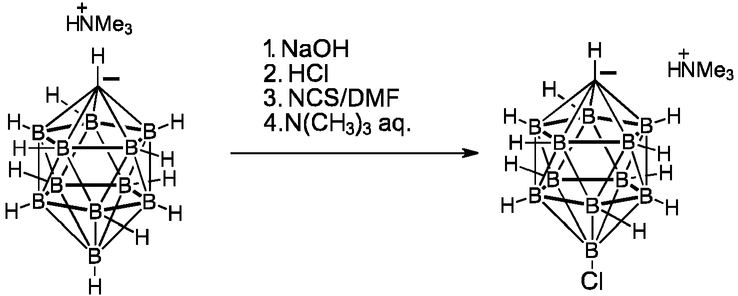

Trimethylammonium carbadodecaborate (1 eq, 4 g, 19.7 mmol) is dissolved in 15 ml of 10% sodium hydroxide. The clear solution is stirred and heated under reduced pressure until released trimethylamine is removed. The solution is cooled with an ice bath, acidified with concentrated hydrochloric acid (pH 1-2) and extracted with diethylether (2x50ml). Combined extracts are evaporated to dryness on rotation evaporator. Residue is dissolved in DMF (20ml), cooled down to -10°C, the solution of N-chlorosuccinimide (0.9 eq, 2.37 g, 17.7 mmol) is added dropwise. The cooling bath is removed, reaction mixture is stirred at room temperature for 1 h. Solvent is evaporated at reduced pressure, residue is dissolved in water and neutralized with an aqueous solution of trimethylamine resulting in precipitation of a product as a white powder. The crude product is separated by filtration, washed with water and dried. Purification of the product is achieved by re-crystallization from water. Yield: 3.4g (72.7%). ESI-MS (-): (M-TMA)- m/z: 177.16 (100.0%), 178.16 (80.5%), 176.17 (78.6%), 179.16 (62.3%)

### Preparation of Trimethylammonium 7,16-Bis(3-(1-carbadodecaboranuidyl)propyl)-1,4,10,13-tetraoxa-7,16-diazacycloocta-decane

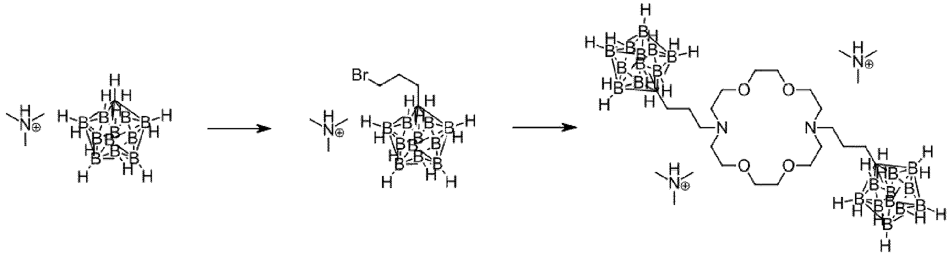

The preparation of the title compound was prepared in a modified synthesis of a previously reported procedure by Michl et al. (J. Am. Chem. Soc. 2004, 126 (48), 15795-15801):
Cesium 1-(3-Bromopropyl)-1-carbadodecaborate: Trimethylammonium (1 eq, 2.03 g, 10 mmol) was dissolved in a aqueous solution of NaOH (20%, 60 ml). The solution was extracted with Et2O (3x 25 ml), the combined extracts washed with a aqueous solution of CsCl (20%, 2x 30 ml) and the aqueous CsCl-layer re-extracted with Et2O (3x 30 ml). All organic layers were combined and concentrated in vacuum. The obtained white solid was dried properly in vacuum at 100 °C before it was redissolved in THF (125 ml). The solution was cooled to -78 °C and n-BuLi (1.6 M in hexane, 2 eq, 12.5 ml, 20 mmol) was added dropwise. The mixture was allowed to slowly warm to room temperature. Stirring at rt was continued for 30 min. This solution was then added to a solution of freshly distilled 1,3-Dibromopropane (3.5 eq, 7.07 g, 35 mmol) in THF (27 ml) via cannula at 0 °C within 10 min. After addition was completed, the mixture was warmed to rt and stirring was continued for 18 h. The reaction was quenched by adding H2O (10 ml). The solution was concentrated in vacuum. The residue was diluted with H2O (15 ml) and extracted with Et2O (4x 70 ml). The combined extracts were washed with a aqueous solution of CsCl. (20 %, 3x 100 ml) and the aqueous CsCl-layers re-extracted with Et2O (3x 120 ml). All organic layers were combined, concentrated and dried in vacuum. The residue was purified by recrystallization from water. The title compound was isolated as colorless crystals.

Yield: 2.8 g (71 %); MS (ESI): m/z = 264.3 [M-Cs]-; Elemental analysis: Calc.: C 12.1 %, H 4.32 %; found: C 11.8 %, H 4.58 %.

**Trimethylammonium 7,16-Bis(3-(1-carbadodecaboranuidyl)propyl)-1,4,10,13-tetraoxa-7,16-diazacyclooctadecane:** A mixture of Cesium 1-(3-Bromopropyl)-1-carbadodecaborate (2 eq, 774 mg, 1.95 mmol) and 1,4,10,13-tetraoxa-7,16-diazacyclooctadecan (1 eq, 253 mg, 0.96 mmol) in MeOH (30 ml) was heated in a pressure tube to 80 °C for 5 d. The mixture was cooled to rt, the precipitate was filtered off, washed with EtOH and Et2O and dried in vacuum. The obtained solid was dissolved in an aqueous solution of Trimethylamine (50 %, 50 ml) at 40 °C. The solution was stirred for 2 h. The excess Trimethylamine was evaporated. The precipitating solid was filtered, washed with ice-cold water and EtOH and dried in vacuum. The title compound was yielded as a brittle foam.

Yield: 240 mg (33 %); MS (ESI): m/z = -314.1 [M-2 Me3NH]2-, 630.0 [M-2 Me3NH+H]-; 1H-NMR (60 MHz, acetone-d6): δ [ppm] =

### Synthesis of europium(II) (tetrahydrofuran)carbadodecaborate

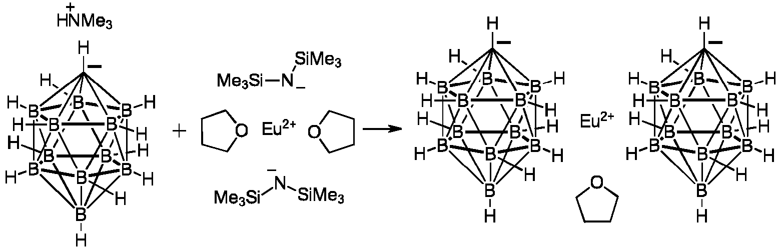

A solution of 5.76 mmol (2.0 eq., 1170mg) trimethylammonium carbadodecaborate in 20 ml THF was dropwise treated with a solution of 2.88 mmol (1.0 eq., 1777mg) europium(II) bis[bis(trimethylsilyl)amido]bis(tetrahydrofuran)europium in 40 ml THF. The resulting suspension was stirred for 1 h at room temperature and then filtrated. The resulting powder was washed with THF and dried under reduced pressure. Yield: 97 %.

Europium (II) tetrahydrofurane 12-chloro-1-carbadodecaborate was prepare analogously from 12-chloro-carbadodecaborate, trimethylammonium salt (1 eq, 2.2 g, 9.26 mmol) and bis[bis(trimethylsilyl)amido]bis(tetrahydrofuran)europium (0.5 eq, 2.86 g, 4.63 mmol) in diethylehter in quantitative yield.

### Synthesis of trimethylammonium tetraphenylborate

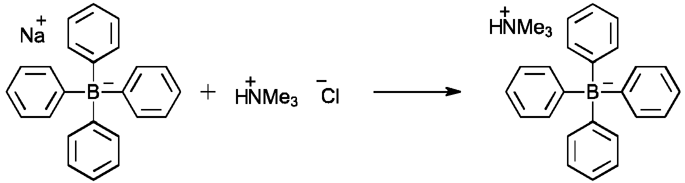

The solution of trimethylamine hydrochloride (1.1 eq, 1.08 g, 11.3 mmol) in 10 ml of deionized water was dropwise added to the solution of sodium tetraphenylborate (1 eq, 3.52 g, 10.3 mmol) in 50 ml of deionized water. The white precipitate was separated by suction filtration, washed with deionized water and dried in vacuum at 75 °C overnight. Yield: 3.2 g (81.9 %)

### Synthesis of europium(II) tetraphenylborate

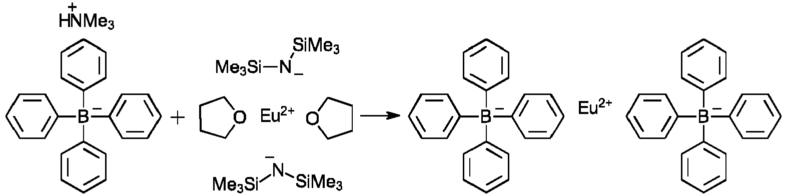

A solution of 2.00 mmol (2.0 eq., 759mg) trimethylammonium tetraphenylborate in 25 ml THF was dropwise by 60 °C treated with a solution of 1.00 mmol (1.0 eq., 617mg) europium(II) bis[bis(trimethylsilyl)amido]bis(tetrahydrofuran)europium in 20 ml THF. The resulting suspension was stirred for 1 h at room temperature and then filtrated. The resulting powder was washed with THF and hexane and dried under reduced pressure. Yield: 54 %.

### Synthesis of europium(II) 1,4,10,13-tetraoxa-7,16-diazacyclooctadecan carbadodecaborate (M3+A1)

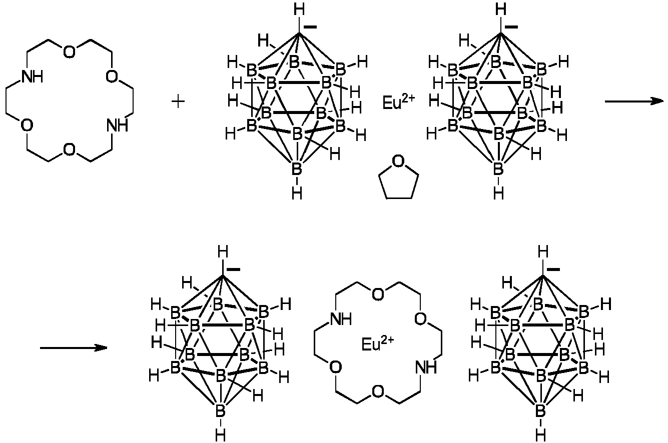

A solution of 1.85 mmol (1.0 eq., 486mg) 1,4,10,13-tetraoxa-7,16-diazacyclooctadecan in 20 ml toluene was dropwise treated with a solution of 1.85 mmol (1.0 eq., 945mg) europium(II) (tetrahydrofuran)carbadodecaborate in 120 ml toluene. The resulting suspension was stirred for 1 h at room temperature. The solvent was removed in vacuum. The resulting solid powder was washed with diethyl ether. Yield: 77 %. Resulted powder was sublimated at 310°C (1.66E-6 mBar), yield 90%. **MS (ESI):** m/z = 557.6 (M - CB11H12-)+; 285.4 (free crown + Na+)+; 263.4 (free crown + H+)+; 238.7 (M - 2 CB11H12- + 2MeOH)2+; 223.7 (M - 2 CB11H12- + MeOH)2+. Elemental Analysis. Calc.: C, 24.01; H, 7.20; N, 4.00. Found.: C, 23.83; H, 7.14; N, 4.06.

**Europium(II) 1,4,10,13-tetraoxa-7,16-diazacyclooctadecan 12-chloro-1-carbadodecaborate (M3+A4)** was synthesized analogously from europium (II) tetrahydrofurane 12-chloro-1-carbadodecaborate (1 eq, 1.16 g, 1.34 mmol) and diaza-18-crown-6 (1 eq, 0.352 g, 1.34 mmol) Yield:88,3%.
**MS (ESI):** m/z = 592.4 (M - CB11H12-)+; 285.4 (free crown + Na+)+; 263.4 (free crown + H+)+; 238.7 (M - 2 CB11H12- + 2MeOH)2+

### Synthesis of europium(II) 1,4,10,13-tetraoxa-7,16-diazacyclooctadecan tetraphenylborate (M3+CE-1)

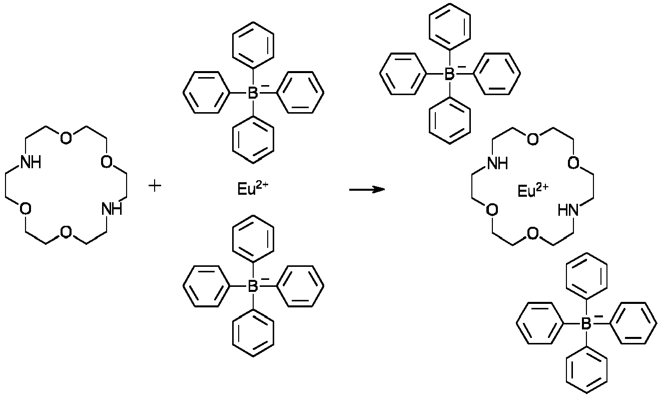

A solution of 0.29 mmol (1.0 eq., 77mg) 1,4,10,13-tetraoxa-7,16-diazacyclooctadecan in 10 ml MeOH was dropwise treated with a solution of 0.29 mmol (1.0 eq., 273mg) europium(II) tetraphenylborate in 10 ml MeOH. The resulting suspension was stirred for 1 h at room temperature and then filtrated. The resulting powder was washed with MeOH and hexane. When attempting to sublimate, the compound decomposed. **ESI (MS)** m/z = 734.3 (M - B(Ph)₄⁻)⁺; 285.4 (free crown + Na⁺)⁺; 263.4 (free crown + H⁺)⁺; 239.6 (M - 2 B(Ph)₄⁻ + 2MeOH)²⁺; 223.6 (M - 2 B(Ph)₄⁻ + MeOH)²⁺ **EA** (of dried material): Calc.: C, 68.45; H, 6.32; N, 2.66. Found.: C, 68.43; H, 6.30; N, 2.61.

### Synthesis of europium(II) 2,3,11,12-dicyclohexano-1,4,7,10,13,16-hexaoxacyclooctadecan carbadodecaborate (M39+A1)

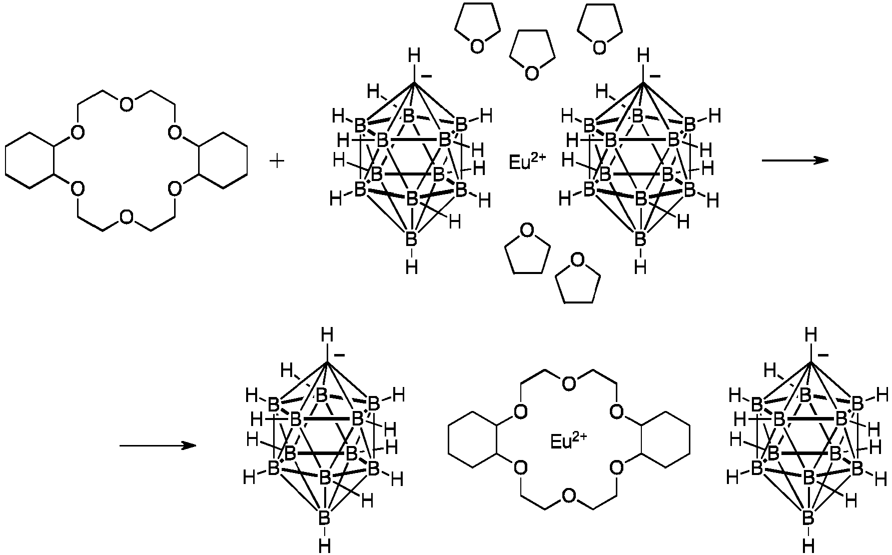

A solution of 0.54 mmol (1.0 eq., 202 mg) 2,3,11,12-dicyclohexano-1,4,7,10,13,16-hexaoxacyclooctadecan in 4.2 ml toluene was added dropwise to a solution of 0.53 mmol (0.97 eq., 420 mg) europium(II) (tetrahydrofuran)carbadodecaborate in 61 ml toluene. The resulting suspension was stirred for 1 h at room temperature. The solvent was removed in vacuum. The resulting solid powder was washed with toluene and hexane. Yield: 95 %. Resulted powder was sublimated at 335°C (1.00E-6 mBar), yield 58%. **MS (ESI):** m/z = 667.8 (M - CB₁₁H₁₂⁻)⁺; 395.5 (free crown + Na⁺)⁺; 373.5 (free crown + H⁺)⁺; 294.6 (M - 2 CB₁₁H₁₂⁻ + 2MeOH)²⁺; 278.9 (M - 2 CB₁₁H₁₂⁻ + MeOH)²⁺; 262.9 (M - 2 CB₁₁H₁₂⁻)²⁺. Elemental Analysis. Calc.: C, 32.60; H, 7.46. Found.: C, 32.62; H, 7.30.

### Synthesis of europium(II) 1,4,7,14,17,20-hexaoxa[7.7]orthocyclophan carbadodecaborate (M40+A1)

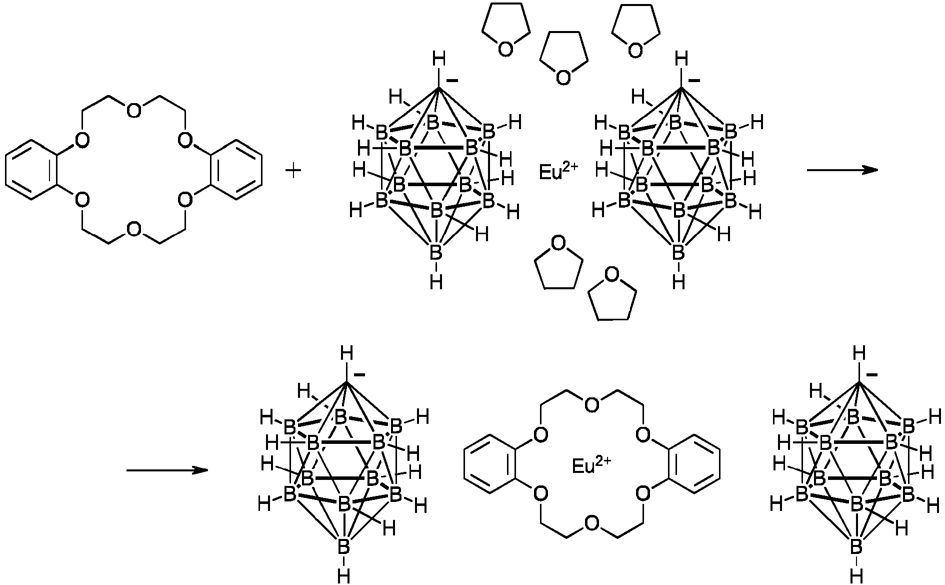

A suspension of 0.54 mmol (1.0 eq., 195 mg) 2,3,11,12-dibenzo-1,4,7,10,13,16-hexaoxacyclooctadecan in 100 ml toluene was heated until it became a clear solution. This solution was dropwise treated with a solution of 0.53 mmol (0.98 eq., 424 mg) europium(II) (tetrahydrofuran)carbadodecaborate in 60 ml toluene. The resulting suspension was stirred for 1 h at the elevated temperature. The solvent volume was reduced to 50 % in vacuum. The resulting suspension filtrated and the solid was washed with toluene and hexane. Yield: 95 %. Resulted powder was sublimated at 235°C (5.40E-7 mBar), yield 12%. **MS (ESI):** m/z = 656.3 (M - CB₁₁H₁₂⁻)⁺; 383.2 (free crown + Na⁺)⁺; 361.2 (free crown + H⁺)⁺; 288.6 (M - 2 CB₁₁H₁₂⁻ + 2MeOH)²⁺; 272.6 (M - 2 CB₁₁H₁₂⁻ + MeOH)²⁺.

### Synthesis of europium(II) 1,4,7,10,13,16-hexaoxacyclooctadecan carbadodecaborate (M1+A1)

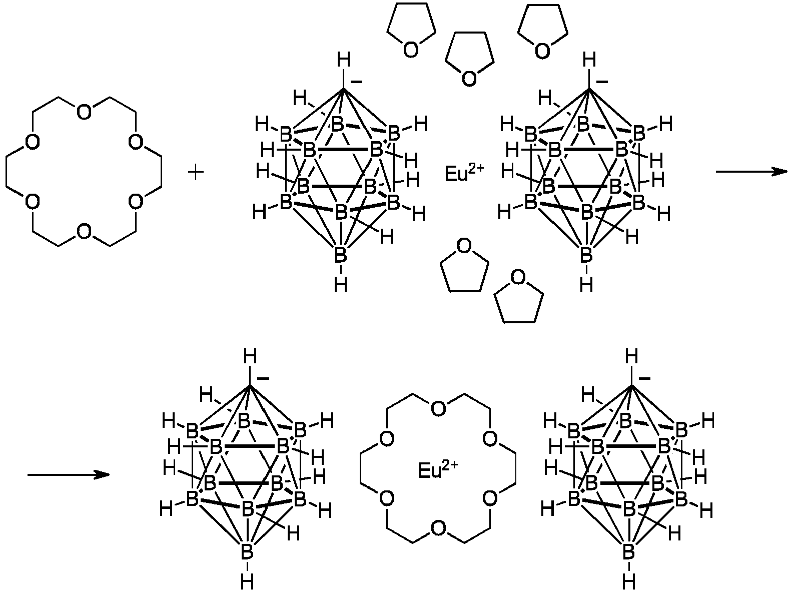

A solution of 0.53 mmol (1.0 eq., 140 mg) 1,4,7,10,13,16-hexaoxacyclooctadecan in 4.3 ml toluene was added dropwise to a solution of 0.53 mmol (0.99 eq., 420 mg) europium(II) (tetrahydrofuran)carbadodecaborate in 60 ml toluene. The resulting suspension was stirred for 1 h at room temperature. The solvent was removed in vacuum. The resulting solid powder was washed with toluene and hexane. Yield: 99 %. Resulted powder was sublimated at 265°C (4.60E-6 mBar), yield 7%. **MS (ESI):** m/z = 287.4 (free crown + Na+)+; 240.8 (M - 2 CB11H12- + 2MeOH)2+; 224.7 (M - 2 CB11H12- + MeOH)2+; 208.7 (M - 2 CB11H12-)2+. Elemental Analysis. Calc.: C, 23.94; H, 6.89. Found.: C, 23.59; H, 6.57.

### Synthesis of 7,16-Bis(3-(1-carbadodecaboranuidyl)propyl)-1,4,10,13-tetraoxa-7,16-diazacyclooctadecane europium(II) (M43)

A mixture of Trimethylammonium 7,16-Bis(3-(1-carbadodecaboranuidyl)propyl)-1,4,10,13-tetraoxa-7,16-diazacyclooctadecane (1 eq. 128 mg, 0.17 mmol) and THF (1.75 ml) was treated with a solution of Eu(HMDS)2 (1 eq, 105 mg, 0.17 mmol) in THF (1.75 ml) at rt. The mixture was stirred for 1 h at rt. The precipitate was filtered off, washed with THF (3x 2 ml) and dried in vacuum. The title compound was yielded as colorless powder.
Yield: 130 mg (97 %)

### General discussion of Syntheses results

As evident from the elemental analyses, ESI-MS, and sublimation data given above, the complexes according to invention, for example M3+A1 and M12+A1, consist of a single Eu(II), a single macrocyclic ligand, and two anions, and can be sublimed and resublimed with yields. In contrast, M3+CE-1, which possesses the same general composition, but a much higher calculated dipole moment, cannot be sublimed.

### III Photoluminescence spectra of synthesized compounds

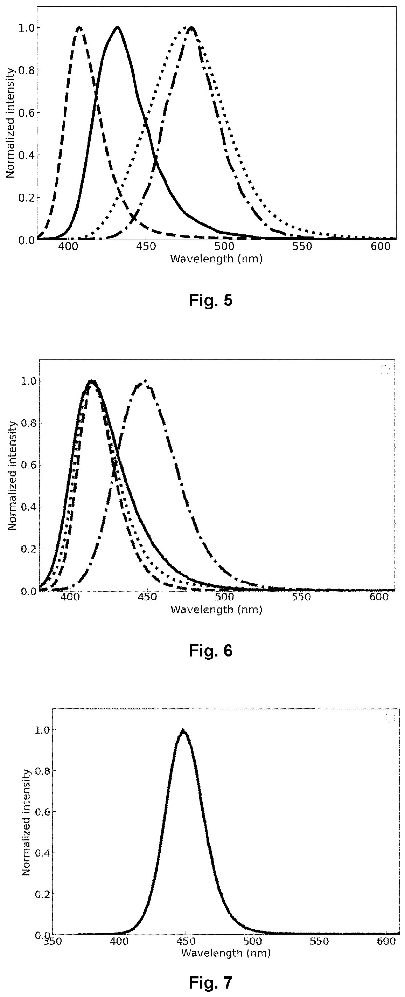

Photo-emission spectra of M12+A1 (solid line), M3+CE1 (dash-dot line), M40+A1 (dashed line), M3+A1 (dotted line) as solid powders are depicted in Fig. 5 testifying to the deep blue emission, very much inline with the corresponding calculated emission energies (EE) depicted in table 1 above, of those coordination compounds. Similarly, photoemission spectra of M39+A1 (solid line), M3+A4 (dash-dot line), M39+A4 (dashed line), and M13+A1 (dotted line) as solid powders are provided Fig. 6. Finally, Fig. 7 depicts the powder emission spectra of M43 with its A1 type anion being covalently attached to the macrocyclic organic ligand.

## Claims

1. A metal-organic coordination compound, wherein the coordination compound comprises one divalent Europium ion and a macrocyclic organic ligand in which at least 9 macrocyclic atoms are part of a single cyclic ring, the macrocycle, wherein only those carbon or heteroatoms are counted as macrocyclic atoms that do form the cyclic closed ring system around the Eu(II), comprising 12 to 48, preferably 15 to 24, and most preferably 18 to 20 macrocyclic atoms, which are present in the macrocycle, further comprising in the coordination compound at least one borane cluster anion selected from (3-1) to (3-3), or at least one borate cluster anion formed from two borate cluster fragments selected from (3-4) to (3-8), which are covalently linked with each other at the dashed line, preferably via a linear C₁₋₁₅⁻ alkylene spacer, 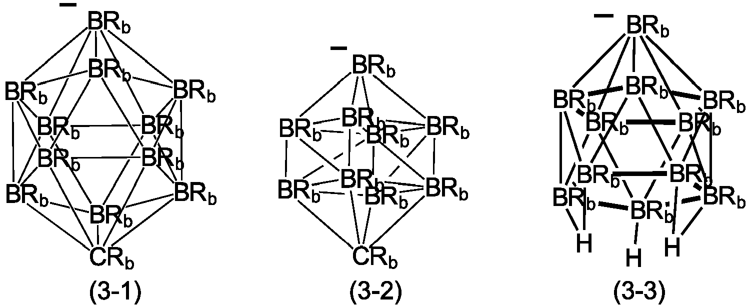 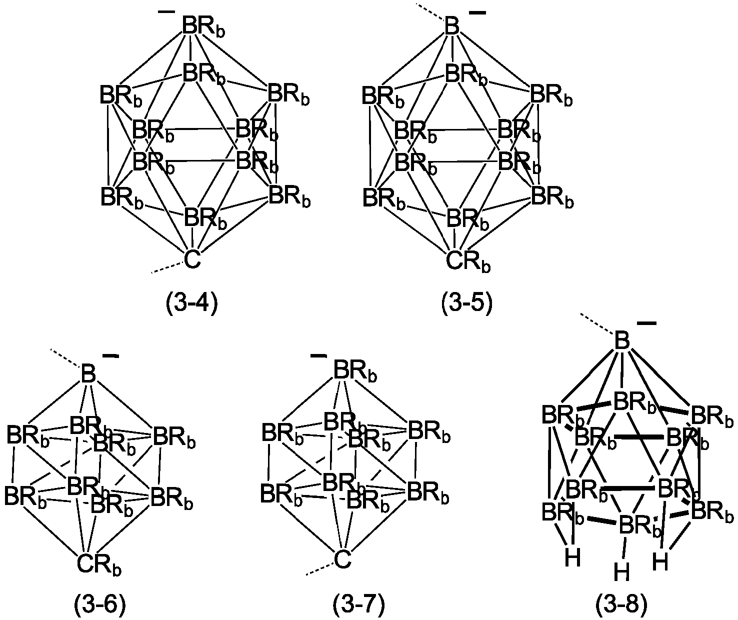
or with the macrocyclic organic ligand being singly or twofold negatively charged by means of covalent attachment of one to two borate cluster fragments selected from (3-4) or (3-8) with
• the dashed lines indicating the covalent linkage with each other or to the remainder of the macrocyclic ligand, and
• R_{b} present in (3-1) to (3-8) independently in each occurrence representing either hydrogen, deuterium, fluorene, chlorine, bromine, or alkyl, preferably C₁₋₆⁻ alkyl, most preferably methyl,
and wherein the macrocyclic organic ligand can be covalently attached to the polymer backbone of a polymer with a molecular weight Mₙ above 1000 g/mol.

2. The metal organic coordination compound according to claim 1 with the shortest sequence of atoms forming the macrocycle in the macrocyclic organic ligand being made of 12 to 48, preferably 15 to 24, most preferably 18 to 20 covalently linked atoms, wherein preferably the macrocycle contains 2 to 14, more preferably 3 to 10, most preferably 5 to 8 non-neighboring heteroatoms.

3. The metal organic coordination compound according to claim 1 or 2 wherein the metal organic coordination compound is charge neutral and contains two, preferably identical, borane cluster anions selected from (3-1) to (3-3) in the coordination compound, or contains two, preferably identical, covalently attached borate cluster fragments selected from (3-4) to (3-8), covalently attached to each other or to the metal organic coordination compound.

4. The metal organic compound according to one of claims 1 to 3 with the macrocyclic organic ligand having the formula (1-1): 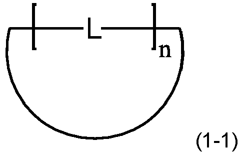 with
• n being 4-8, preferably 4-6, most preferably 6; and
• L being independently in each occurrence a divalent organic group formed by removing two hydrogen atoms from an organic molecule containing at least two hydrogen atoms, with
∘ the shortest sequence of atoms linking each L with the remainder of the macrocycle in the macrocyclic ligand being 2 to 4, preferably 3 atoms, and with
∘ one of those 2 to 4, preferably 3 atoms independently for each L being selected from the heteroatoms B, N, P, S, Se, Si or O, preferably N, S, or O, the other atoms of those atoms being carbon,
wherein preferably the heteroatoms in the macrocycle are non-neighboring.

5. The metal organic coordination compound according to claim 4 with n = 6 and L being independently in each occurrence selected from (2-1) to (2-9), preferably from (2-2), (2-4), or (2-5): 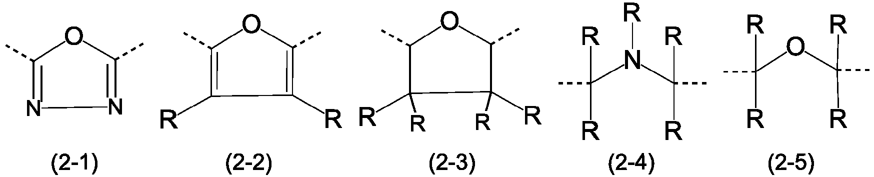 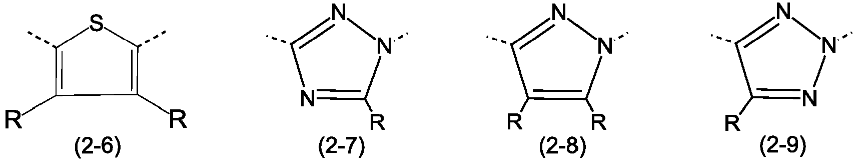 with
• R being H, D, or any covalently linked substituent being identical or different in each occurrence, and
• the dashed lines indicating the covalent linkage to the remainder of the macrocyclic ligand according to formula (1-1).

6. The metal organic coordination compound according to one of claims 2 to 5, wherein any further ring structures, forming part of the macrocyclic organic ligand in addition to the macrocycle are covalently linked with one macrocycle atom, or form part of the macrocycle by 1,2-, 1,3- or 1,4-linkage of the further ring structure with the remainder of the macrocycle, and are not additionally covalently linked with other atoms of the macrocycle.

7. The metal organic coordination compound according to one of claims 1 to 6, wherein the macrocyclic organic ligand contains 0, 1, 2 or 3 S atoms and 0, 1, or 2 atoms, selected from P, Si and B.

8. The metal organic coordination compound according to any of claims 1 to 7, with the macrocyclic organic ligand being selected from G1 to G9: 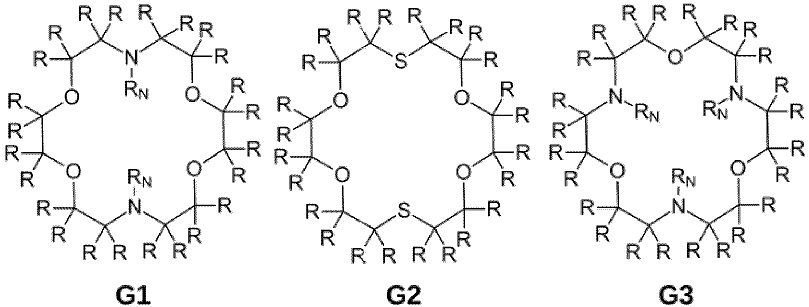 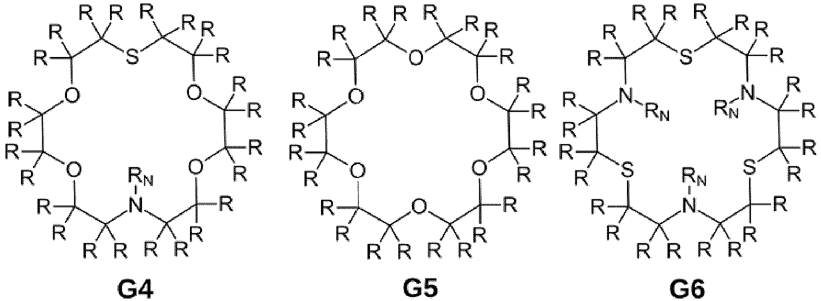 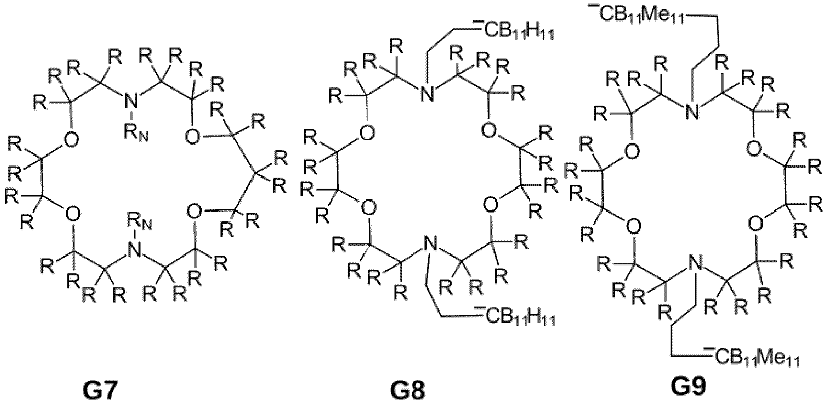 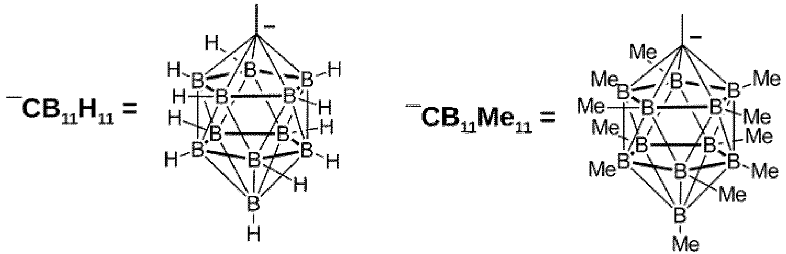 with
• R being identical or different in each occurrence and being H, D, or any covalently linked monovalent organic group formed by removing a hydrogen atom from an organic molecule containing at least one hydrogen atom whereby two, three, or four instances of R being present on the same or neighboring carbon atoms can be fused, and
• **R_{N}** representing hydrogen, deuterium, or any covalently linked monovalent organic group formed by removing a hydrogen atom from an organic molecule containing at least one hydrogen atom.

9. The metal organic coordination compound according to one of claims 1 to 8, being selected from E1 to E17:

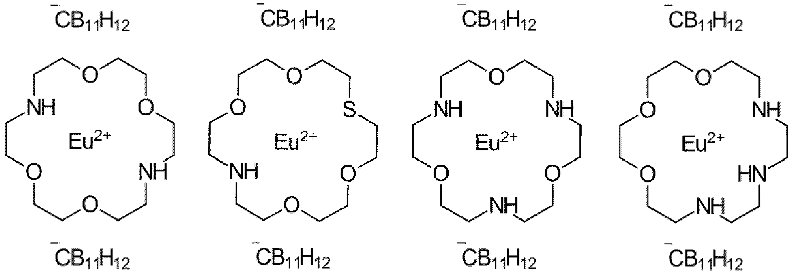

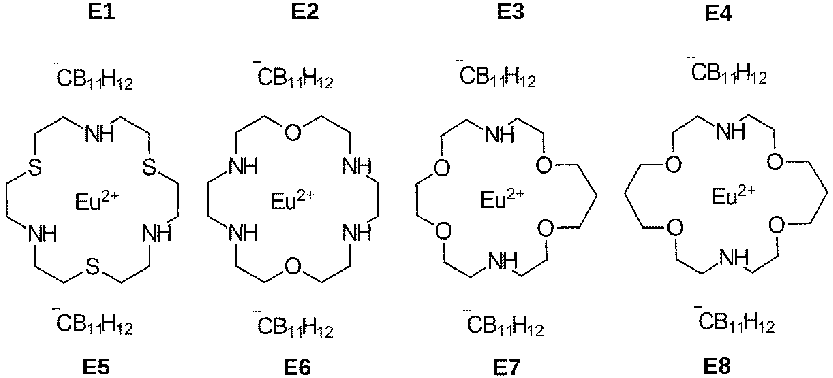

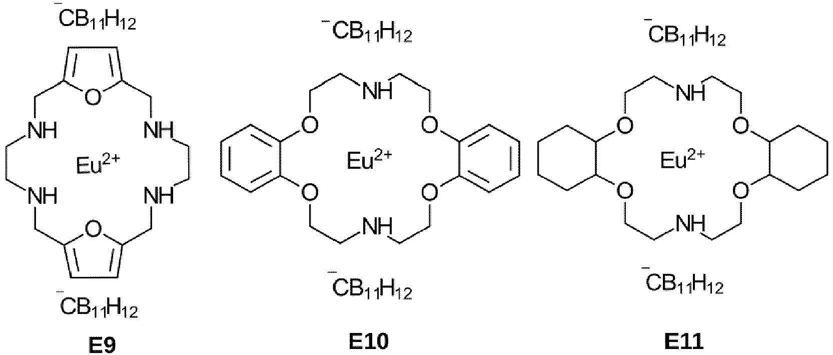

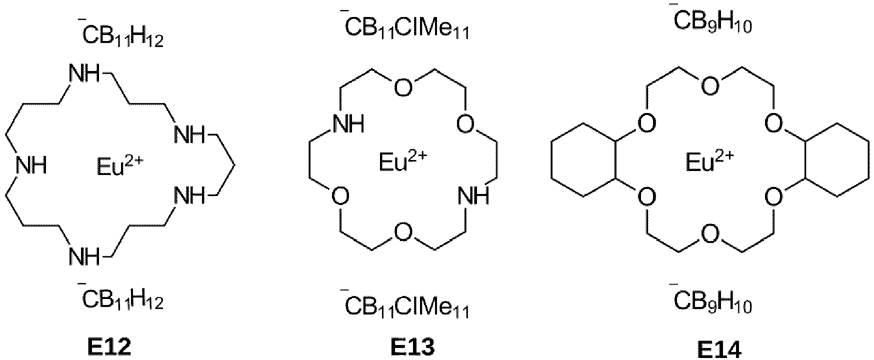

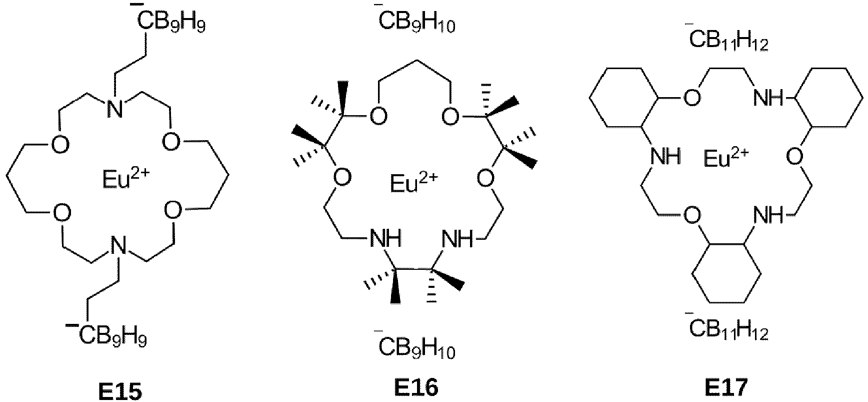

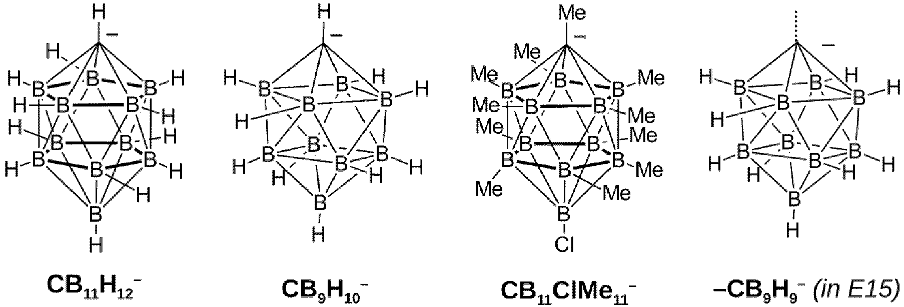

10. A mixture comprising
(a) the coordination compound according to one of claims 1 to 9, and
(b) at least one second electrically neutral organic compound different therefrom,
wherein the coordination compound (a) is imbedded into the at least one second electrically neutral organic compound (b), the mixture having preferably one or more of the following features:
- The second compound (b) is a charge-neutral host material;
- The second organic compound (b) has a triplet energy higher than 2.5 eV, preferably higher than 2.6 eV and more preferably higher than 2.7 eV;
- Compounds (a) and (b) are physically mixed or the second charge-neutral compound (b) has a molecular weight Mₙ above 1000 g/mol and the coordination compound (a) and the second charge-neutral compound (b) are chemically linked with each other, for example by a covalent or coordinative chemical bond;
- The coordination compound (a) is electrically neutral or singly positively charged and is imbedded into the at least one second electrically neutral organic compound (b) in a ratio of 0.1 to 99 vol%, based on the mixture which is 100 vol%;
- The second organic compound (b) has a lower hole affinity compared to the electrically neutral coordination compound (a);
- The second organic compound (b) has a lower electron affinity compared to the singly positively charged coordination compound (a);
- The second material is a charge transport organic material capable of transporting positive or negative charges.

11. An organic electronic device, comprising:
a first electrode;
a second electrode; and
an organic layer arranged such that it is electrically interposed between the first and second electrodes, wherein the organic layer comprises the coordination compound according to one of claims 1 to 9,
wherein preferably the organic electronic device is an optoelectronic device, the optoelectronic device preferably being at least one of an organic light emitting diode, an organic photodetector, or a photovoltaic cell.

12. A method of forming an organic device according to claim 11, the method comprising:
forming a layer of the coordination compound according to any one of claims 1 to 9, or the mixture of claim 10,
wherein the layer is deposited from a gas phase, in particular using an evaporation and/or sublimation and/or carrier gas process, or wherein the layer is deposited from solution,
whereby preferably the solution is comprised substantially or organic solvents with a polarity index of less than 5.0, preferably of less than 4.0, more preferably of less than 3.0.

13. The use of a coordination compound according to one of claims 1 to 9 or of a mixture according to claim 10 as active emitting material in electroluminescent electronics applications or as a dye or contrast enhancement medium for magnetic resonance tomography.

14. The use of borane clusters in which the borane cluster anion is selected from (3-1) to (3-3), for forming coordination compounds according to any one of claims 1 to 9 comprising one divalent Europium ion, a macrocyclic organic ligand, and further comprising at least one borane cluster anion selected from (3-1) to (3-3), or at least one borate cluster anion formed from two borate cluster fragments selected from (3-4) to (3-8), which are covalently linked with each other at the dashed line, preferably via a linear C₁₋₁₅⁻ alkylene spacer, or with the macrocyclic organic ligand being singly or twofold negatively charged by means of covalent attachment of one or two borate cluster fragments selected from (3-4) to (3-8) 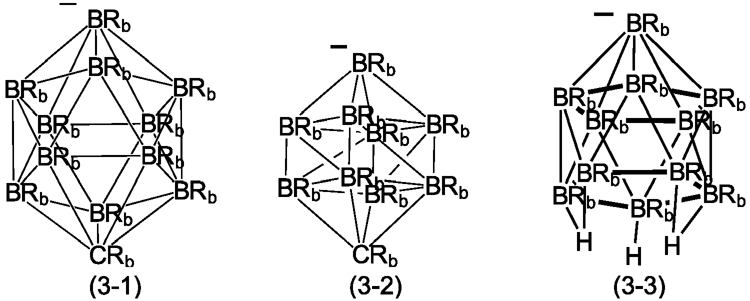 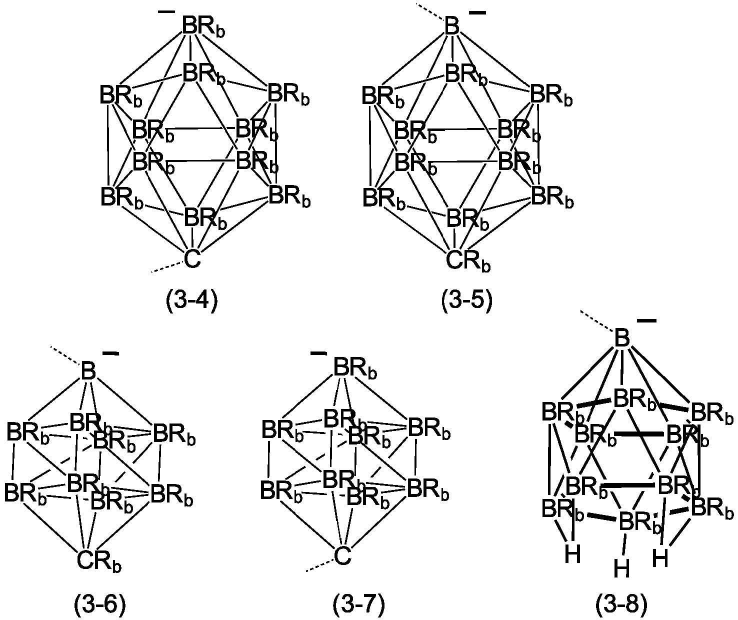 with
• the dashed lines indicating the covalent linkage to the remainder of the macrocyclic ligand, and
• **R_{b}** present in (3-1) - (3-8) independently in each occurrence representing either hydrogen, deuterium, fluorene, chlorine, bromine, or alkyl, preferably C₁₋₆⁻ alkyl, most preferably methyl,
and wherein the macrocyclic organic ligand can be covalently attached to the polymer backbone of a polymer with a molecular weight Mₙ above 1000 g/mol.

## Patentansprüche

1. Metallorganische Koordinationsverbindung, wobei die Koordinationsverbindung ein zweiwertiges Europiumion und einen makrocyclischen organischen Liganden umfasst, in welchem mindestens 9 makrocyclische Atome Teil eines einzigen cyclischen Rings, des Makrocyclus, sind, wobei nur diejenigen Kohlenstoff- oder Heteroatome als makrocyclische Atome gezählt werden, die das cyclische geschlossene Ringsystem um das Eu(II) bilden, umfassend 12 bis 48, vorzugsweise 15 bis 24 und ganz besonders bevorzugt 18 bis 20 makrocyclische Atome, die in dem Makrocyclus vorliegen, ferner umfassend in der Koordinationsverbindung mindestens ein Borancluster-Anion, ausgewählt aus (3-1) bis (3-3), oder mindestens ein Boratcluster-Anion, gebildet aus zwei Boratcluster-Fragmenten, die aus (3-4) bis (3-8) ausgewählt sind, die an der gestrichelten Linie kovalent miteinander verknüpft sind, vorzugsweise über einen linearen C₁₋₁₅-Alkylen-Spacer, 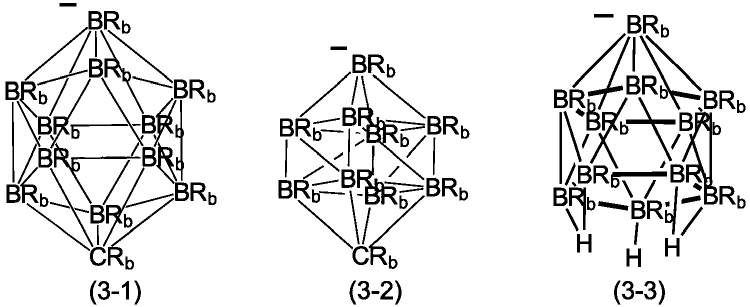 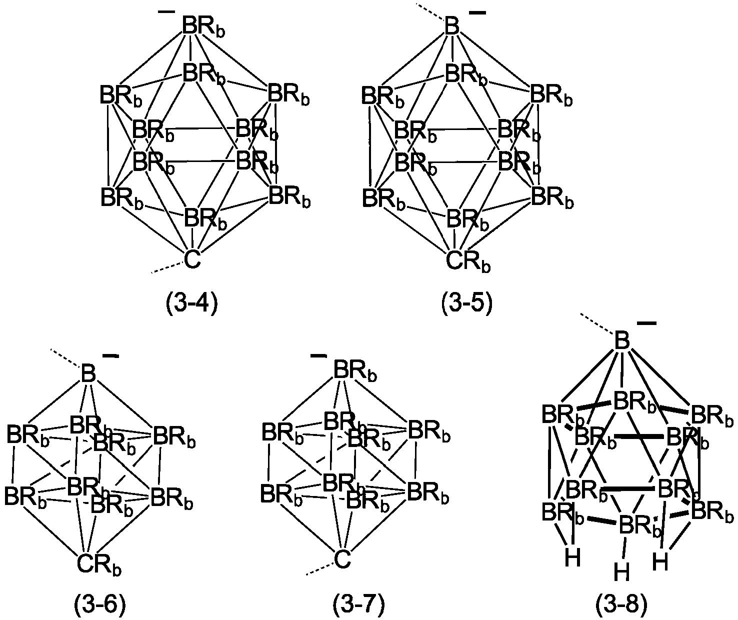
oder wobei der makrocyclische organische Ligand durch kovalente Anbindung von einem bis zwei Boratcluster-Fragmenten, die aus (3-4) oder (3-8) ausgewählt sind, ein- oder zweifach negativ geladen ist, wobei
• die gestrichelten Linien die kovalente Verknüpfung miteinander oder mit dem Rest des makrocyclischen Liganden anzeigen und
• R_{b}, das in (3-1) bis (3-8) vorliegt, unabhängig bei jedem Auftreten für Wasserstoff, Deuterium, Fluor, Chlor, Brom oder Alkyl, vorzugsweise C₁₋₆-Alkyl, ganz besonders bevorzugt Methyl, steht,
und wobei der makrocyclische organische Ligand kovalent an die Polymerhauptkette eines Polymers mit einem Molekulargewicht Mₙ über 1000 g/mol gebunden sein kann.

2. Metallorganische Koordinationsverbindung nach Anspruch 1, wobei die kürzeste Abfolge von den Makrocyclus bildenden Atomen in dem makrocyclischen organischen Liganden aus 12 bis 48, vorzugsweise 15 bis 24, ganz besonders bevorzugt 18 bis 20, kovalent verknüpften Atomen besteht, wobei der Makrocyclus vorzugsweise 2 bis 14, weiter bevorzugt 3 bis 10, ganz besonders bevorzugt 5 bis 8, nicht benachbarte Heteroatome enthält.

3. Metallorganische Koordinationsverbindung nach Anspruch 1 oder 2, wobei die metallorganische Koordinationsverbindung ladungsneutral ist und zwei, vorzugsweise identische, Borancluster-Anionen, die aus (3-1) bis (3-3) ausgewählt sind, in der Koordinationsverbindung enthält oder zwei, vorzugsweise identische, kovalent verknüpfte Borancluster-Fragmente, die aus (3-4) bis (3-8) ausgewählt sind, die kovalent miteinander oder mit der metallorganischen Koordinationsverbindung verknüpft sind, enthält.

4. Metallorganische Verbindung nach einem der Ansprüche 1 bis 3, wobei der makrocyclische organische Ligand die Formel (1-1) aufweist: 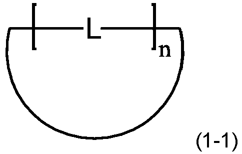 wobei
• n für 4-8, vorzugsweise 4-6, ganz besonders bevorzugt 6, steht und
• L unabhängig bei jedem Auftreten für eine zweiwertige organische Gruppe, die durch Entfernen von zwei Wasserstoffatomen von einem organischen Molekül, das mindestens zwei Wasserstoffatome enthält, gebildet wird, steht, wobei
∘ die kürzeste Abfolge von Atomen, die jedes L mit dem Rest des Makrocyclus in dem makrocyclischen Liganden verknüpft, 2 bis 4, vorzugsweise 3, Atome, beträgt und wobei
∘ eines dieser 2 bis 4, vorzugsweise 3, Atome unabhängig für jedes L aus den Heteroatomen B, N, P, S, Se, Si oder O, vorzugsweise N, S oder O, ausgewählt ist, wobei es sich bei den anderen Atomen dieser Atome um Kohlenstoff handelt,
wobei vorzugsweise die Heteroatome in dem Makrocyclus nicht benachbart sind.

5. Metallorganische Koordinationsverbindung nach Anspruch 4 mit n = 6 und wobei L unabhängig bei jedem Auftreten aus (2-1) bis (2-9), vorzugsweise aus (2-2), (2-4) oder (2-5), ausgewählt ist: 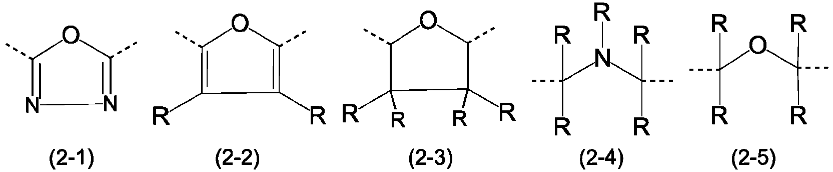 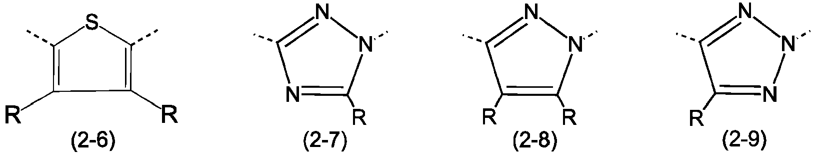 wobei
• R für H, D oder einen beliebigen kovalent verknüpften Substituenten, der bei jedem Auftreten gleich oder verschieden ist, steht und
• die gestrichelten Linien die kovalente Verknüpfung mit dem Rest des makrocyclischen Liganden gemäß Formel (1-1) anzeigen.

6. Metallorganische Koordinationsverbindung nach einem der Ansprüche 2 bis 5, wobei beliebige weitere Ringstrukturen, die zusätzlich zu dem Makrocyclus einen Teil des makrocyclischen organischen Liganden bilden, kovalent mit einem Makrocyclusatom verknüpft sind oder durch 1,2-, 1,3- oder 1,4-Verknüpfung der weiteren Ringstruktur mit dem Rest des Makrocyclus einen Teil des Makrocyclus bilden und nicht zusätzlich kovalent mit anderen Atomen des Makrocyclus verknüpft sind.

7. Metallorganische Koordinationsverbindung nach einem der Ansprüche 1 bis 6, wobei der makrocyclische organische Ligand 0, 1, 2 oder 3 S-Atome und 0, 1 oder 2 Atome, die aus P, Si und B ausgewählt sind, enthält.

8. Metallorganische Koordinationsverbindung nach einem der Ansprüche 1 bis 7, wobei der makrocyclische organische Ligand aus G1 bis G9 ausgewählt ist: 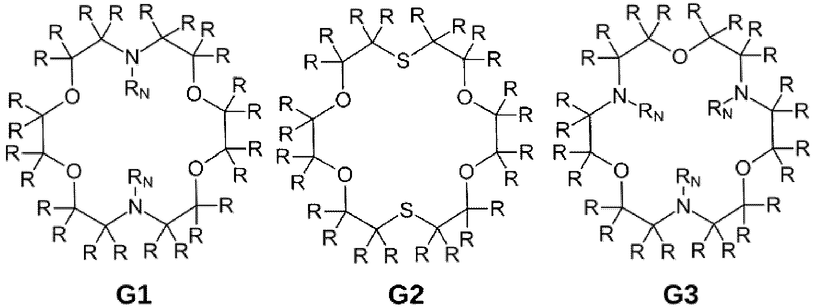 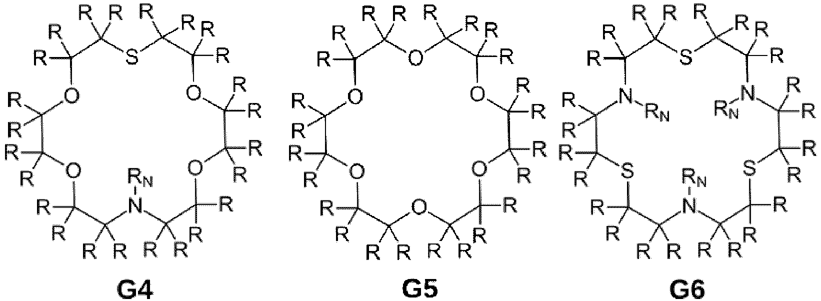 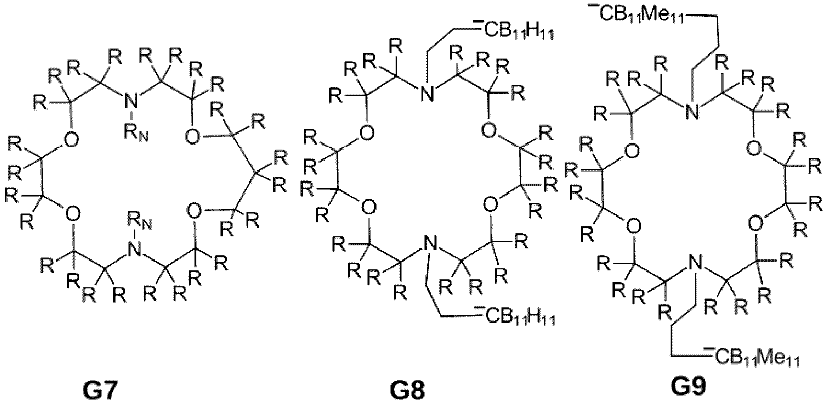 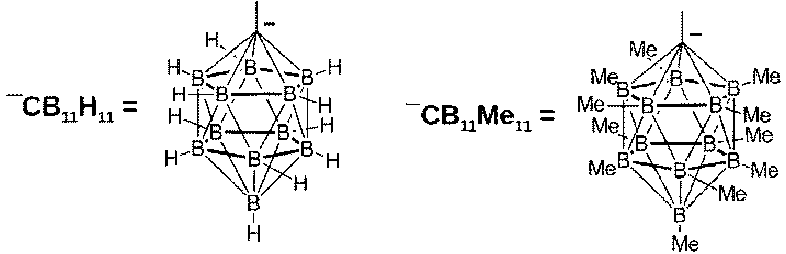 wobei
• R bei jedem Auftreten gleich oder verschieden ist und für H, D oder eine kovalent verknüpfte einwertige organische Gruppe, die durch Entfernen eines Wasserstoffatoms von einem organischen Molekül, das mindestens ein Wasserstoffatom enthält, gebildet wird, steht, wobei zwei, drei oder vier Vorkommen von R, die an demselben Kohlenstoffatom oder benachbarten Kohlenstoffatomen vorliegen, kondensiert sein können, und
• R_{N} für Wasserstoff, Deuterium oder eine kovalent verknüpfte einwertige organische Gruppe, die durch Entfernen eines Wasserstoffatoms von einem organischen Molekül, das mindestens ein Wasserstoffatom enthält, gebildet wird, steht.

9. Metallorganische Koordinationsverbindung nach einem der Ansprüche 1 bis 8, die aus E1 bis E17 ausgewählt ist:

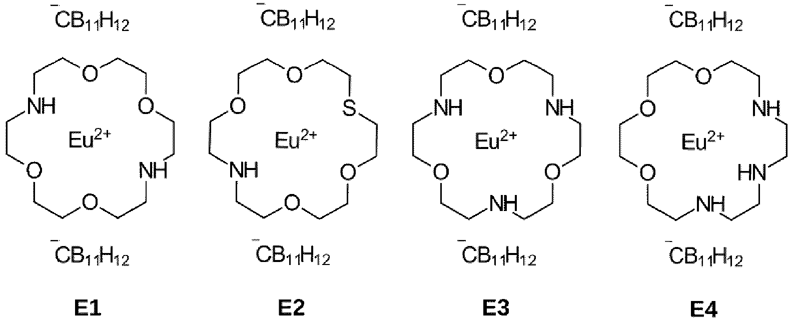

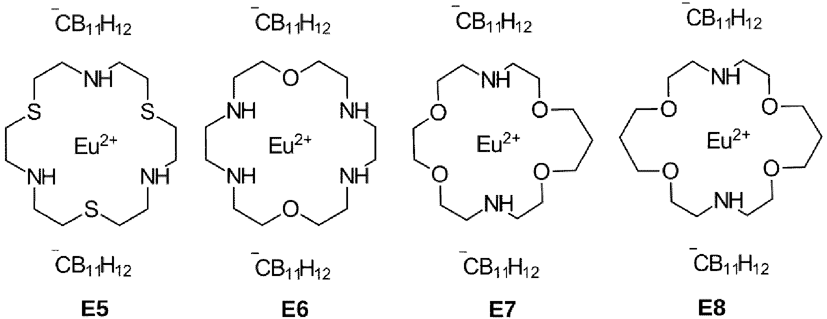

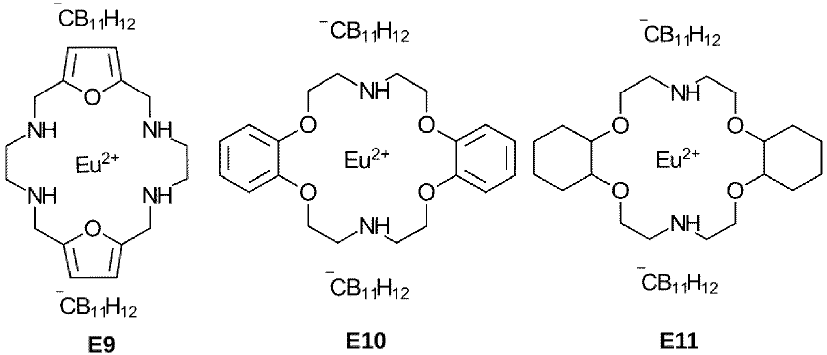

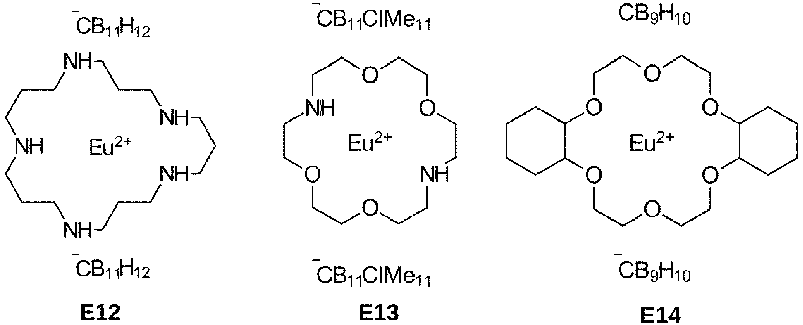

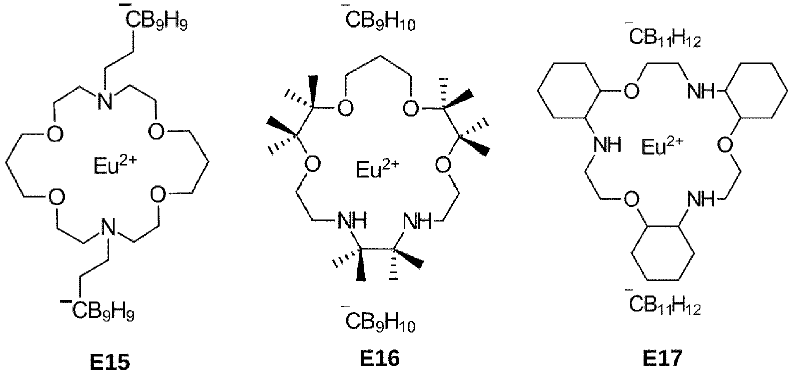

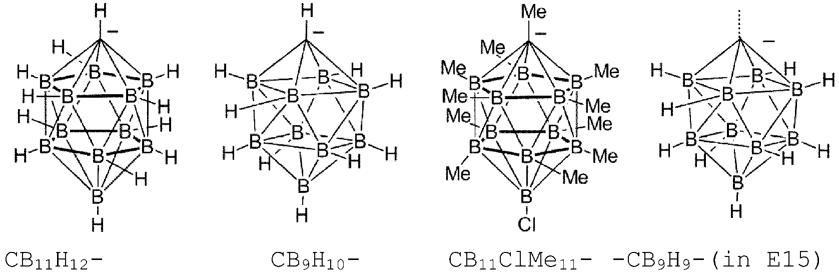

10. Mischung, umfassend
(a) die Koordinationsverbindung nach einem der Ansprüche 1 bis 9 und
(b) mindestens eine davon verschiedene zweite neutrale organische Verbindung,
wobei die Koordinationsverbindung (a) in die mindestens eine zweite elektrisch neutrale organische Verbindung (b) eingebettet ist, wobei die Mischung vorzugsweise eines oder mehrere der folgenden Merkmale aufweist:
- die zweite Verbindung (b) ist ein ladungsneutrales Wirtsmaterial;
- die zweite organische Verbindung (b) weist eine Triplettenergie von mehr als 2,5 eV, vorzugsweise mehr als 2,6 eV und ganz besonders bevorzugt mehr als 2,7 eV auf;
- die Verbindungen (a) und (b) sind physikalisch gemischt oder die zweite ladungsneutrale Verbindung (b) weist ein Molekulargewicht Mₙ über 1000 g/mol auf und die Koordinationsverbindung (a) und die zweite ladungsneutrale Verbindung (b) sind chemisch miteinander verknüpft, zum Beispiel über eine kovalente oder koordinative chemische Bindung;
- die Koordinationsverbindung (a) ist elektrisch neutral oder einfach positiv geladen und ist in einem Anteil von 0,1 bis 99 Vol.-%, bezogen auf die Mischung, die 100 Vol.-% beträgt, in die mindestens eine zweite elektrisch neutrale organische Verbindung (b) eingebettet;
- die zweite organische Verbindung (b) weist eine geringere Lochaffinität als die elektrisch neutrale Koordinationsverbindung (a) auf;
- die zweite organische Verbindung (b) weist eine geringere Elektronenaffinität als die einfach positiv geladene Koordinationsverbindung (a) auf;
- das zweite Material ist ein organisches Ladungstransportmaterial, das positive oder negative Ladungen transportieren kann.

11. Organische elektronische Vorrichtung, umfassend:
eine erste Elektrode;
eine zweite Elektrode und
eine organische Schicht, die so angeordnet ist, dass sie sich elektrisch zwischen der ersten und der zweiten Elektrode befindet, wobei die organische Schicht die Koordinationsverbindung nach einem der Ansprüche 1 bis 9 umfasst,
wobei vorzugsweise die organische elektronische Vorrichtung eine optoelektronische Vorrichtung ist, wobei es sich bei der optoelektronischen Vorrichtung vorzugsweise um eine organische Leuchtdiode, einen organischen Photodetektor und/oder eine Photovoltaikzelle handelt.

12. Verfahren zur Bildung einer organischen Vorrichtung nach Anspruch 11, wobei das Verfahren Folgendes umfasst:
Bilden einer Schicht aus der Koordinationsverbindung nach einem der Ansprüche 1 bis 9 oder der Mischung nach Anspruch 10,
wobei die Schicht aus einer Gasphase, insbesondere unter Verwendung eines Verdampfungs- und/oder Sublimations- und/oder Trägergasverfahrens, abgeschieden wird oder
wobei die Schicht aus Lösung abgeschieden wird, wobei die Lösung vorzugsweise weitgehend aus organischen Lösungsmitteln mit einem Polaritätsindex von weniger als 5,0, vorzugsweise von weniger als 4,0, weiter bevorzugt von weniger als 3,0, besteht.

13. Verwendung einer Koordinationsverbindung nach einem der Ansprüche 1 bis 9 oder einer Mischung nach Anspruch 10 als aktives emittierendes Material in Elektrolumineszenz-Elektronikanwendungen oder als Farbstoff oder Kontrastverstärkungsmedium für die Magnetresonanztomographie.

14. Verwendung von Boranclustern, in denen das Borancluster-Anion aus (3-1) bis (3-3) ausgewählt ist, zur Bildung von Koordinationsverbindung nach einem der Ansprüche 1 bis 9, umfassend ein zweiwertiges Europiumion, einen Makro cyclischen organischen Liganden und ferner mindestens ein aus (3-1) bis (3-3) ausgewähltes Borancluster-Anion oder mindestens ein Boratcluster-Anion, gebildet aus zwei Boratcluster-Fragmenten, die aus (3-4) bis (3-8) ausgewählt sind, die an der gestrichelten Linie kovalent miteinander verknüpft sind, vorzugsweise über einen linearen C₁₋₁₅-Alkylen-Spacer, oder wobei der makrocyclische organische Ligand durch kovalente Anbindung von einem bis zwei Boratcluster-Fragmenten, die aus (3-4) oder (3-8) ausgewählt sind, ein- oder zweifach negativ geladen ist, 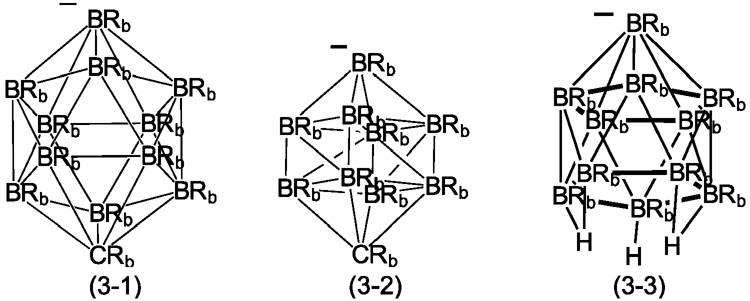 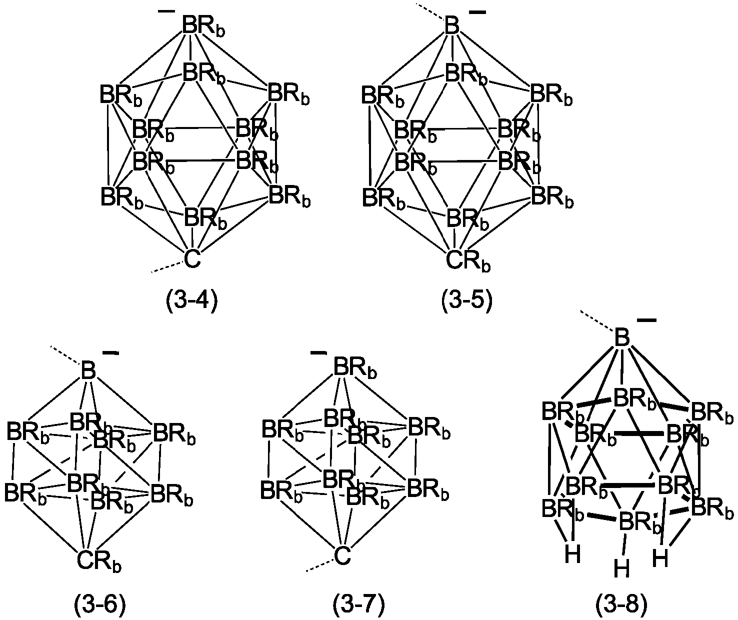 wobei
• die gestrichelten Linien die kovalente Verknüpfung mit dem Rest des makrocyclischen Liganden anzeigen und
• R_{b}, das in (3-1) - (3-8) vorliegt, unabhängig bei jedem Auftreten für Wasserstoff, Deuterium, Fluor, Chlor, Brom oder Alkyl, vorzugsweise C₁₋₆-Alkyl, ganz besonders bevorzugt Methyl, steht,
und wobei der makrocyclische organische Ligand kovalent an die Polymerhauptkette eines Polymers mit einem Molekulargewicht Mₙ über 1000 g/mol gebunden sein kann.

## Revendications

1. Composé de coordination organométallique, dans lequel le composé de coordination comprend un ion Europium divalent et un ligand organique macrocyclique dans lequel au moins 9 atomes macrocycliques font partie d'un seul cycle cyclique, le macrocycle, dans lequel seuls les atomes de carbone ou hétéroatomes sont comptés comme atomes macrocycliques qui forment le système cyclique à cycles fermés autour du Eu(II), comprenant 12 à 48, de préférence 15 à 24, et le plus préférablement 18 à 20 atomes macrocycliques, qui sont présents dans le macrocycle, comprenant en outre dans le composé de coordination au moins un anion de cluster de borane choisi parmi (3-1) à (3-3), ou au moins un anion de cluster de borate formé à partir de deux fragments de cluster de borate choisis parmi (3-4) à (3-8), qui sont liés de manière covalente l'un à l'autre au niveau de la ligne pointillée, de préférence par l'intermédiaire d'un espaceur alkylène linéaire en C₁₋₁₃, 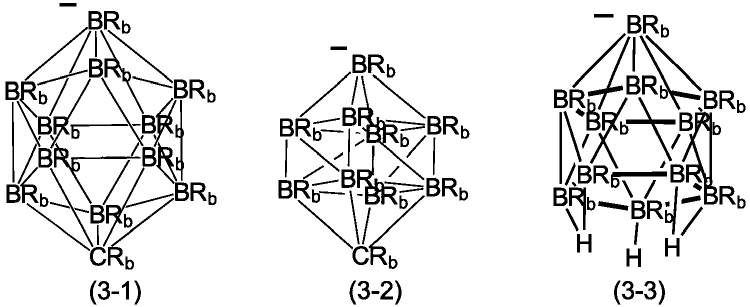 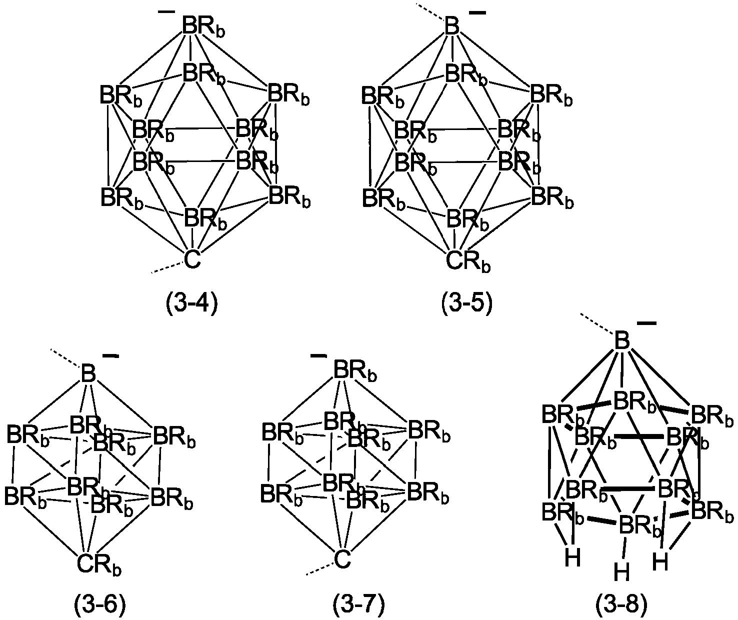
ou avec le ligand organique macrocyclique qui est chargé négativement une ou deux fois au moyen d'une fixation covalente d'un à deux fragments de cluster de borate choisis parmi (3-4) ou (3-8) avec
• les lignes pointillées indiquant la liaison covalente de l'un à l'autre ou avec le reste du ligand macrocyclique, et
• R_{b} présent dans (3-1) à (3-8) indépendamment en chaque occurrence représentant soit hydrogène, deutérium, fluor, chlore, brome ou alkyle, de préférence alkyle en C₁₋₆, le plus préférablement méthyle,
et dans lequel le ligand organique macrocyclique peut être fixé de manière covalente au squelette de polymère d'un polymère ayant un poids moléculaire Mₙ supérieur à 1 000 g/mole.

2. Composé de coordination organométallique selon la revendication 1, dans lequel la séquence d'atomes la plus courte formant le macrocycle dans le ligand organique macrocyclique est composée de 12 à 48, de préférence 15 à 24, le plus préférablement 18 à 20 atomes liés de manière covalente, dans lequel de préférence le macrocycle contient 2 à 14, plus préférablement 3 à 10, le plus préférablement 5 à 8 hétéroatomes non voisins.

3. Composé de coordination organométallique selon la revendication 1 ou 2, dans lequel le composé de coordination organométallique est neutre en charge et contient deux anions de cluster de borane, de préférence identiques, choisis parmi (3-1) à (3-3) dans le composé de coordination, ou contient deux fragments de cluster de borate, de préférence identiques, fixés de manière covalente, choisis parmi (3-4) à (3-8), fixés de manière covalente l'un à l'autre ou au composé de coordination organométallique.

4. Composé organométallique selon l'une des revendications 1 à 3, le ligand organique macrocyclique ayant la formule (1-1) : 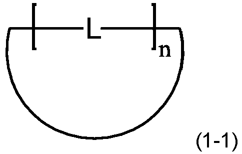
• n étant 4-8, de préférence 4-6, le plus préférablement 6 ; et
• L étant indépendamment en chaque occurrence un groupe organique divalent formé par élimination de deux atomes d'hydrogène d'une molécule organique contenant au moins deux atomes d'hydrogène,
∘la séquence d'atomes la plus courte liant chaque L au reste du macrocycle dans le ligand macrocyclique étant de 2 à 4, de préférence 3 atomes, et
∘ l'un de ces 2 à 4, de préférence 3 atomes indépendamment pour chaque L étant choisi parmi les hétéroatomes B, N, P, S, Se, Si ou O, de préférence N, S ou O, les autres atomes parmi ces atomes étant carbone, de préférence, les hétéroatomes dans le macrocycle étant non voisins.

5. Composé de coordination organométallique selon la revendication 4, n = 6 et L étant indépendamment en chaque occurrence choisi parmi (2-1) à (2-9), de préférence parmi (2-2), (2-4), ou (2-5) : 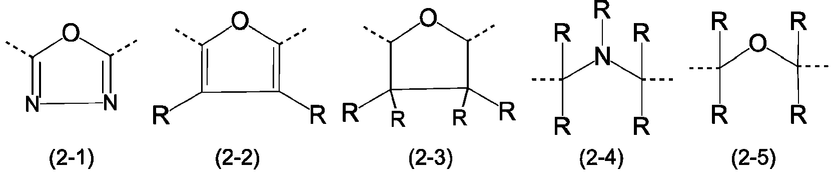 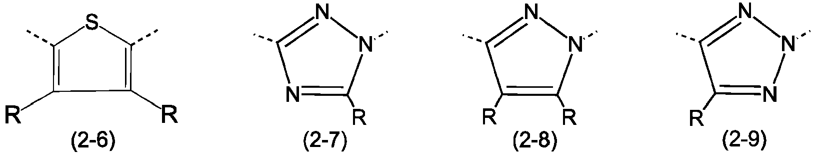
• R étant H, D, ou tout substituant lié de manière covalente qui est identique ou différent en chaque occurrence, et
• les lignes pointillées indiquant la liaison covalente au reste du ligand macrocyclique selon la formule (1-1).

6. Composé de coordination organométallique selon l'une des revendications 2 à 5, dans lequel toutes structures cycliques supplémentaires, faisant partie du ligand organique macrocyclique en plus du macrocycle, sont liées de manière covalente à un atome du macrocycle, ou font partie du macrocycle par une liaison 1,2-, 1,3- ou 1,4- de la structure cyclique supplémentaire avec le reste du macrocycle, et ne sont pas de plus liées de manière covalente à d'autres atomes du macrocycle,

7. Composé de coordination organométallique selon l'une des revendications 1 à 6, dans lequel le ligand organique macrocyclique contient 0, 1, 2 ou 3 atomes de S et 0, 1 ou 2 atomes, choisis parmi P, Si et B.

8. Composé de coordination organométallique selon l'une quelconque des revendications 1 à 7, le ligand organique macrocyclique étant choisi parmi G1 à G9 : 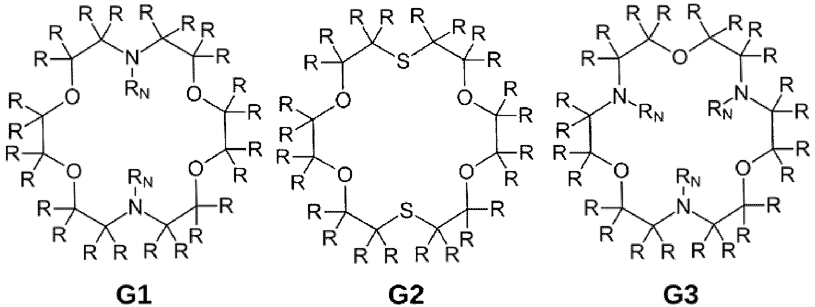 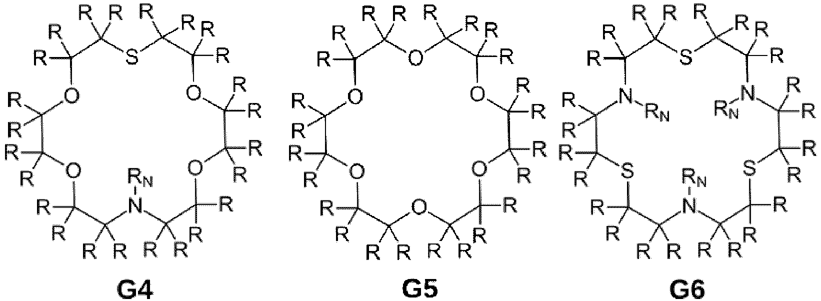 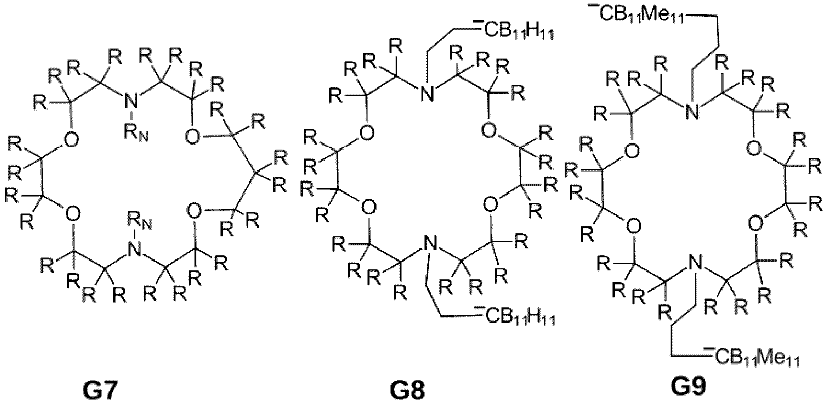 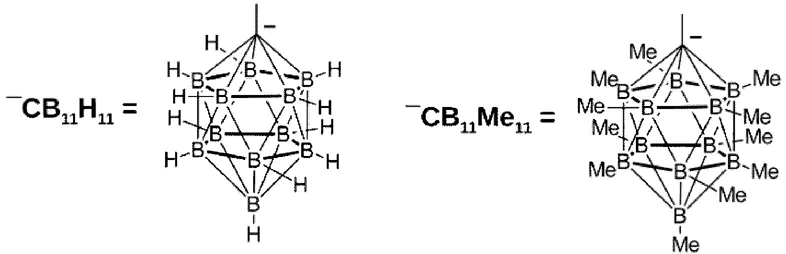
• R étant identique ou différent en chaque occurrence et représentant H, D, ou tout groupe organique monovalent lié de manière covalente formé par élimination d'un atome d'hydrogène d'une molécule organique contenant au moins un atome d'hydrogène, deux, trois ou quatre occurrences de R étant présentes sur les mêmes atomes de carbone ou des atomes de carbone voisins pouvant être fusionnées, et
• R_{N} représentant hydrogène, deutérium ou tout groupe organique monovalent lié de manière covalente formé par élimination d'un atome d'hydrogène d'une molécule organique contenant au moins un atome d'hydrogène.

9. Composé de coordination organométallique selon l'une des revendications 1 à 8, qui est choisi parmi E1 à E17 :

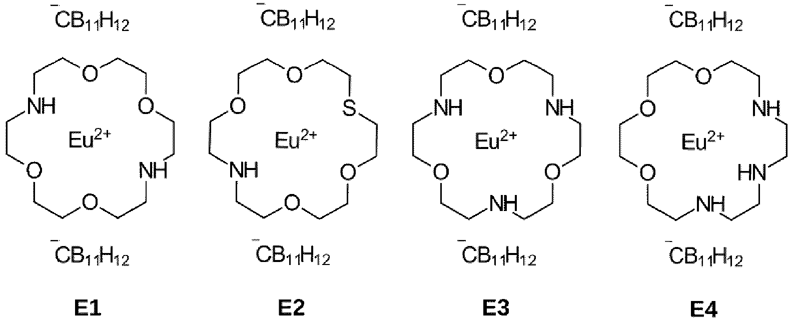

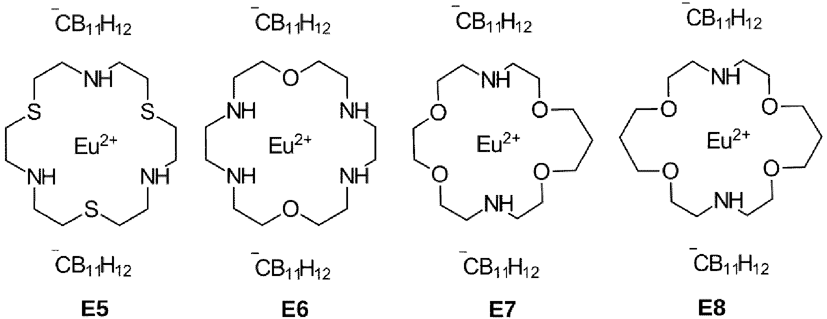

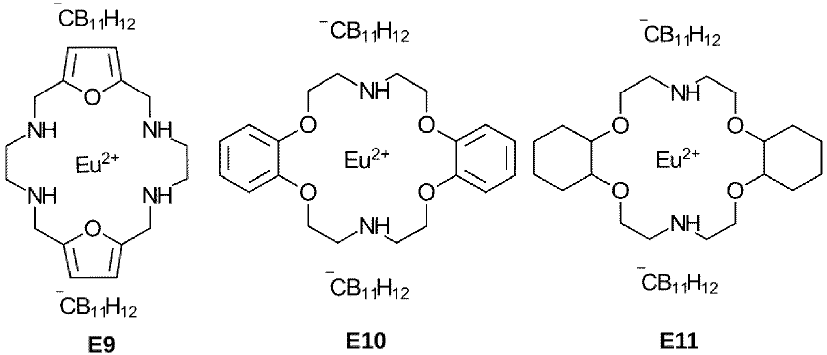

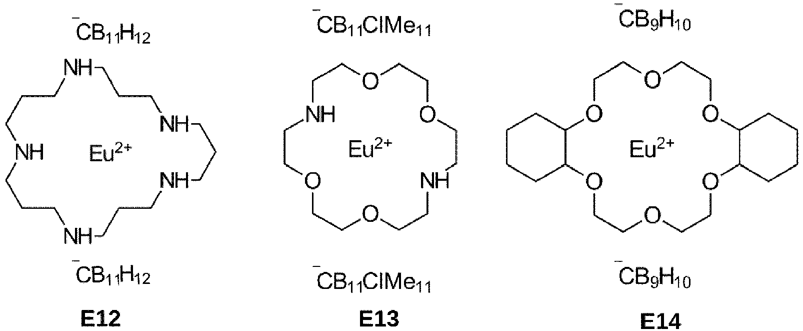

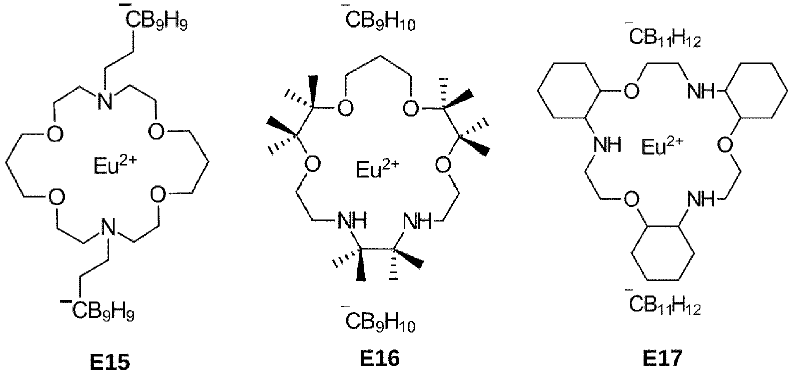

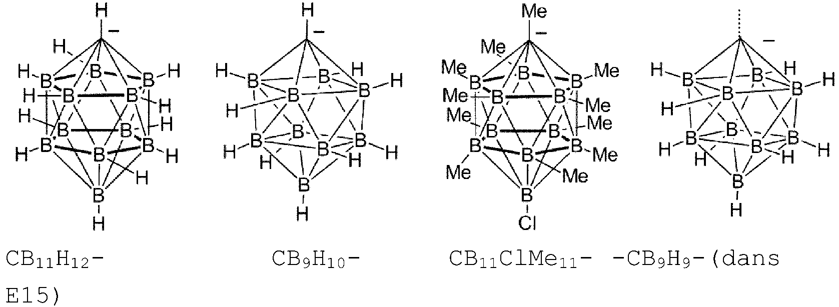

10. Mélange comprenant
(a) le composé de coordination selon l'une des revendications 1 à 9, et
(b) au moins un deuxième composé organique électriquement neutre différent de celui-ci,
dans lequel le composé de coordination (a) est incorporé dans l'au moins un deuxième composé organique électriquement neutre (b), le mélange ayant de préférence une ou plusieurs des caractéristiques suivantes :
- le deuxième composé (b) est un matériau hôte à charge neutre ;
- le deuxième composé organique (b) a une énergie de triplet supérieure à 2,5 eV, de préférence supérieure à 2,6 eV et plus préférablement supérieure à 2,7 eV;
- les composés (a) et (b) sont physiquement mélangés ou le deuxième composé à charge neutre (b) a une masse moléculaire Mₙ supérieure à 1 000 g/mol et le composé de coordination (a) et le deuxième composé à charge neutre (b) sont chimiquement liés l'un à l'autre, par exemple par une liaison chimique covalente ou de coordination ;
- le composé de coordination (a) est électriquement neutre ou chargé positivement une seule fois et est incorporé dans l'au moins un deuxième composé organique électriquement neutre (b) en une proportion de 0,1 à 99 % en volume, par rapport au mélange qui est de 100 % en volume ;
- le deuxième composé organique (b) a une plus faible affinité pour les trous par comparaison au composé de coordination électriquement neutre (a) ;
- le deuxième composé organique (b) a une plus faible affinité pour les électrons par comparaison au composé de coordination chargé positivement une seule fois (a) ;
- le deuxième matériau est un matériau organique de transport de charge capable de transporter des charges positives ou négatives.

11. Dispositif électronique organique, comprenant :
une première électrode ;
une deuxième électrode ; et
une couche organique agencée de manière à être électriquement interposée entre la première et la deuxième électrode, la couche organique comprenant le composé de coordination selon l'une des revendications 1 à 9,
dans lequel le dispositif électronique organique est un dispositif optoélectronique, le dispositif optoélectronique étant de préférence au moins l'un d'une diode électroluminescente organique, d'un photodétecteur organique ou d'une cellule photovoltaïque.

12. Procédé de formation d'un dispositif organique selon la revendication 11, le procédé comprenant :
la formation d'une couche du composé de coordination selon l'une quelconque des revendications 1 à 9, ou du mélange selon la revendication 10,
dans lequel la couche est déposée à partir d'une phase gazeuse, en particulier à l'aide d'un processus d'évaporation et/ou de sublimation et/ou avec gaz vecteur, ou dans lequel la couche est déposée à partir d'une solution, la solution étant alors de préférence constituée essentiellement de solvants organiques présentant un indice de polarité inférieur à 5,0, de préférence inférieur à 4,0, plus préférablement inférieur à 3,0.

13. Utilisation d'un composé de coordination selon l'une des revendications 1 à 9 ou d'un mélange selon la revendication 10 en tant que matériau émetteur actif dans des applications électroniques électroluminescentes ou en tant que colorant ou agent améliorant le contraste pour la tomographie par résonance magnétique.

14. Utilisation de clusters de borane dans lesquels l'anion de cluster de borane est choisi parmi (3-1) à (3-3), pour former des composés de coordination selon l'une quelconque des revendications 1 à 9 comprenant un ion Europium divalent, un ligand organique macrocyclique, et comprenant en outre au moins un anion de cluster de borane choisi parmi (3-1) à (3-3), ou au moins un anion de cluster de borate formé à partir de deux fragments de cluster de borate choisis parmi (3-4) à (3-8), qui sont liés de manière covalente l'un à l'autre au niveau de la ligne pointillée, de préférence par l'intermédiaire d'un espaceur alkylène linéaire en C₁₋₁₅, ou avec le ligand organique macrocyclique chargé négativement une ou deux fois au moyen d'une fixation covalente d'un ou deux fragments de cluster de borate choisis parmi (3-4) à (3-8) 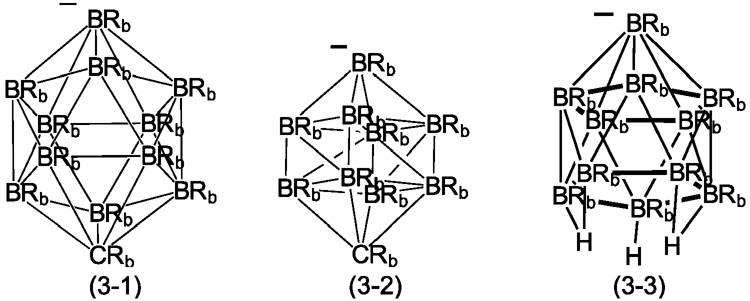 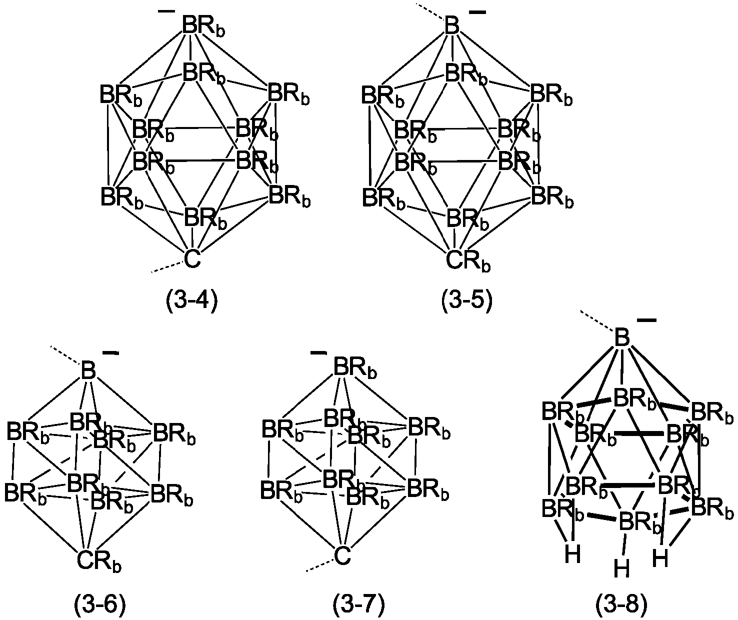
• les lignes pointillées indiquant la liaison covalente avec le reste du ligand macrocyclique, et
• R_{b} présent dans (3-1) - (3-8) indépendamment en chaque occurrence représentant soit hydrogène, deutérium, fluor, chlore, brome ou alkyle, de préférence alkyle en C₁₋₆, le plus préférablement méthyle,
et dans lequel le ligand organique macrocyclique peut être fixé de manière covalente au squelette de polymère d'un polymère ayant un poids moléculaire Mₙ supérieur à 1 000 g/mole.
